# FASCICULE DE BREVET EUROPEEN

(11) **EP 3 215 494 B1**
(45) Date de publication et mention de la délivrance du brevet: **12.12.2018**
(21) Numéro de dépôt: 15808708.0
(22) Date de dépôt: 04.11.2015
(51) Int. Cl.: C07D 233/64, H01G 9/20, H01L 51/42

(54) **UTILISATION DE DÉRIVES HALOGÈNES DE L'HISTIDINE A TITRE DE SEL D'ÉLECTROLYTE DANS UNE CELLULE PHOTOVOLTAÏQUE A COLORANT**
VERWENDUNG VON HALOGENDERIVATEN VON HISTIDIN ALS ELEKTROLYTSALZ IN EINER PHOTOVOLTAISCHEN FARBSTOFFZELLE
USE OF HALOGEN DERIVATIVES OF HISTIDINE AS ELECTROLYTIC SALT IN A PHOTOVOLTAIC DYE CELL

(30) Priorité: 05.11.2014 FR 1460661
(43) Date de publication de la demande: 13.09.2017
(73) Titulaire: Centre National de la Recherche Scientifique, 75016 Paris (FR); Université de Picardie Jules Verne, 80025 Amiens Cedex 1 (FR)
(72) Inventeur: SAGAIDAK, Iryna, 80080 Amiens (FR); FLASQUE, Miguel, 80000 Amiens (FR); SAUVAGE, Frédéric, 80290 Digeon (FR); NGUYEN VAN NHIEN, Albert, 80090 Amiens (FR)
(74) Mandataire: Lavoix
(86) Numéro de dépôt international: PCT/FR2015/052984
(87) Numéro de publication internationale: WO 2016/071637

(56) Documents cités:
- WO-A2-2004/038745
- XIN XU ET AL: "Performance Enhancement of Dye-Sensitized Solar Cells Using an Ester-Functionalized Imidazolium Iodide as the Solid State Electrolyte", ACS APPLIED MATERIALS & INTERFACES, vol. 5, no. 8, 24 avril 2013 (2013-04-24), pages 3219-3223, XP055169001, ISSN: 1944-8244, DOI: 10.1021/am4002293

## Description

La présente invention est relative à l'utilisation de dérivés halogénés de l'histidine à titre de sels d'électrolyte pour la préparation d'une composition d'électrolyte dans une cellule photoélectrochimique basée sur la sensibilisation à la lumière de molécules photo-actives, ainsi qu'à une cellule photoélectrochimique basée sur la sensibilisation à la lumière de molécules photo-actives comprenant une composition d'électrolyte comprenant au moins un dérivé halogéné d'histidine à titre de sel d'électrolyte.

Le domaine de l'invention peut être défini comme celui des cellules photoélectrochimique basées sur la sensibilisation à la lumière de molécules photo-actives, et plus particulièrement des cellules de conversion photovoltaïques à colorant (ou cellules solaires à colorant).

Les cellules photovoltaïques les plus répandues (dites cellules solaires de première génération) sont constituées de semi-conducteurs massifs, principalement à base de silicium (Si) amorphe ou monocristallin. Elles se présentent généralement sous la forme de fines plaques d'une dizaine de centimètres de côté avec des zones dopées p et des zones dopées n, afin de créer une jonction p-n. Le semi-conducteur constitue le milieu dit photoactif à l'intérieur duquel la lumière est absorbée, créant ainsi des paires électrons-trous. Ces plaques sont prises en sandwich entre deux contacts métalliques, pour une épaisseur de l'ordre du millimètre. Les cellules à base de silicium les plus performantes comprennent une couche active de silicium monocristallin dont le rendement de conversion peut atteindre 40 % en laboratoire.

Cependant, bien que très performantes, les cellules photovoltaïques à base de silicium, et en particulier de silicium monocristallin, présentent l'inconvénient majeur d'être onéreuses en raison du coût élevé lié à cette matière première et sa transformation. C'est pourquoi une partie de la recherche s'est tournée vers les cellules à base de semi-conducteurs en couches minces.

En effet, la technologie couches minces permet de diminuer la quantité de semi-conducteurs utilisée et rend, de plus, possible l'utilisation de substrats de faible coût, pouvant être flexibles et de grande surface. Dans les cellules photovoltaïques en couches minces (dites cellules solaires de deuxième génération), utilisant à titre de matériau semi-cristallin du silicium (silicium amorphe ou silicium cristallin, en général polycristallin), du disélénium de cuivre indium (CIS) ou du tellure de cadmium (CdTe). Les couches minces de matériau (de l'ordre du micron) sont déposées sur un substrat, par exemple en verre. Cependant, bien que d'un coût de revient moins élevé que les cellules solaires de première génération, les cellules de conversion photovoltaïques à base de couches minces en silicium amorphe sont sujettes à des problèmes de stabilité lorsqu'elles sont exposées au soleil. De plus, du fait de sa structure désordonnée, les propriétés de transport de charge du silicium amorphe sont médiocres, d'où un rendement médiocre. Ainsi une chute de rendement de 10 à 50 % de ces cellules se produit au cours des premières centaines d'heures d'exposition à la lumière des cellules à base de silicium amorphe.

Des cellules à base de semi-conducteurs organiques, et en particulier de composés organométalliques dont le coût de revient est moindre que celui du silicium ont également été proposées (cellules solaires de troisième génération). Leur utilisation dans le domaine photovoltaïque est basée sur la capacité de certains polymères et oligomères π-conjugués, ou encore de certaines petites molécules π-conjuguées, à convertir l'énergie lumineuse en énergie électrique. Lorsqu'on constitue une jonction p-n composée de deux semi-conducteurs de nature différente parmi lesquels au moins un est un composé organique, on définit ainsi une hétéroj onction.

Un autre type de cellule solaire est constitué par les cellules photovoltaïques basées sur la sensibilisation à la lumière de molécules photo-actives (ou cellules solaires à colorant, dites « Cellules de Graetzel »). Ces cellules différencient les fonctions d'absorption de la lumière et de séparation des charges électriques et sont inspirées de la photosynthèse. Les cellules solaires à colorant sont constituées d'une photoanode comprenant une couche de nanoparticules d'un oxyde semiconducteur tel que par exemple le dioxyde de titane sur laquelle est adsorbé un colorant (ou « sensibilisateur »), une contre-électrode comprenant un catalyseur au platine et un électrolyte séparant la photoanode de la contre-électrode, ledit électrolyte incluant une paire ionique red/ox. Sous l'effet du rayonnement solaire, le colorant est excité par les photons incidents absorbés et gagne suffisamment d'énergie pour pouvoir injecter un électron dans la bande de conduction de l'oxyde semi-conducteur. Ces cellules présentent l'avantage que les matériaux qu'elles utilisent sont peu couteux.

Parmi les constituants des cellules solaires à colorants, l'électrolyte est le constituant qui détermine la durée de vie de la cellule, ainsi que ses performances, notamment le facteur de forme (FF) et l'efficacité de la conversion lumineuse (Eff). Les électrolytes des cellules solaires à colorant actuelles sont des liquides, en particulier des liquides ioniques halogénés (I⁻, Br⁻), ou des gels à base du couple redox I₃⁻/I⁻.

Il a en particulier déjà été proposé, notamment dans la demande internationale WO 2010/117871 d'utiliser des halogénures formés d'un cation comprenant un ou plusieurs groupes contenant au moins un atome d'azote quaternaire, un atome de soufre tertiaire ou un atome de phosphore quaternaire et d'un anion comprenant un ion halogénure (Br⁻ ou I⁻). Selon cette demande internationale le cation contenant au moins un atome d'azote quaternaire peut en particulier être un groupe de type imidazolium ou triazolium, et notamment un groupe 1,3-dialkylimidazolium. Le document WO 2004/038745 décrit des sels d'imidazolium et leur utilisation comme électrolytes dans les cellules photovoltaïques.

Cependant, l'utilisation de ces liquides ioniques ne donne pas entièrement satisfaction dans la mesure où les halogénures de dialkylimidazolium en particulier sont des composés très hygroscopiques (environ 30 % de perte en masse sensible due à la présence d'eau) et dont l'utilisation dans les électrolytes des cellules solaires pose un certain nombre d'inconvénients. En effet, dans la mesure où la présence d'eau est néfaste pour la stabilité des cellules solaires, les halogénures de dialkylimidazolium, et tout particulièrement les iodures de dialkylimidazolium, doivent être stockés en condition anhydre et impérativement séchés avant d'être utilisés pour la fabrication de cellules solaires, sous peine de voir la stabilité de celles-ci fortement altérée. Les composés dérivés d'imidazole sont par ailleurs généralement synthétisés à partir de ressources pétrolifères, ce qui n'est pas satisfaisant d'un point de vue de la protection de l'environnement et des énergies renouvelables.

Il existe donc un besoin pour des composés pouvant être utilisés à titre de sels d'électrolyte dans une composition d'électrolyte d'une cellule photoélectrochimique basée sur la sensibilisation à la lumière de molécules photo-actives et en particulier dans une cellule photovoltaïque à colorant, lesdits composés ne présentant pas les inconvénients des composés utilisés dans l'art antérieur, notamment pour des composés non hygroscopiques et pouvant être synthétisés de façon simple et peu couteuse à partir de matières premières ne provenant pas de ressources pétrolifères.

Les inventeurs ont maintenant découvert que certains halogénures dérivés de la *L*-histidine et dont la formule va être définie ci-après, présentent d'excellentes propriétés électrolytiques, ce qui leur permet d'être avantageusement utilisés pour à titre de sel d'électrolyte d'une composition d'électrolyte dans une cellule photoélectrochimique basée sur la sensibilisation à la lumière de molécules photo-actives et en particulier dans une cellule photovoltaïque à colorant.

La présente invention a donc pour premier objet l'utilisation d'au moins un halogénure de formule (I) suivante : 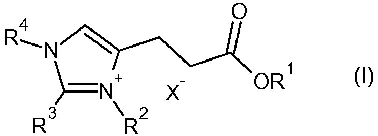 dans laquelle :
- R¹ représente un atome d'hydrogène ou un radical alkyle linéaire ayant de 1 à 10 atomes de carbone ;
- R² et R⁴, identiques ou différents, représentent chacun un radical alkyle linéaire ou ramifié ayant de 1 à 15 atomes de carbone, de préférence de 1 à 6 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote ;
- R³ représente un atome d'hydrogène ou un radical alkyle linéaire ou ramifié ayant de 1 à 15 atomes de carbone, de préférence de 1 à 6 atomes de carbone,
- X⁻ représente un anion iodure ou bromure,
à titre de sel d'électrolyte dans une composition d'électrolyte d'une cellule photoélectrochimique basée sur la sensibilisation à la lumière de molécules photo-actives et en particulier dans une cellule photovoltaïque à colorant.

Les composés de formule (I) définis ci-dessus peuvent être facilement obtenus à partir de la *L*-histidine dans des conditions douces, la *L*-histidine étant un acide aminé ne dépendant pas des ressources pétrolifères. Par ailleurs, ces composés ne sont pas hygroscopiques et ne nécessitent aucune précaution particulière de stockage ou d'utilisation pour la fabrication de cellules photoélectrochimiques basées sur la sensibilisation à la lumière de molécules photo-actives et en particulier de cellules photovoltaïques à colorant. De plus, les cellules photovoltaïques à colorant intégrant de tels composés à titre d'électrolyte présentent des performances améliorées par rapport à des cellules photovoltaïques à colorants équivalentes mettant en oeuvre un halogénure d'imidazolium à titre d'électrolyte, notamment en termes de stabilité thermique au regard du protocole standard de vieillissement défini par la norme IEC 61646 (pour «*International Electrotechnical Commission* » qui est une organisation internationale de normalisation chargée des domaines de l'électricité, de l'électronique et des techniques connexes). La norme IEC 61646 est dédiée aux cellules photovoltaïques de type couche mince (pour application terrestre) et certifie une garantie de qualité en matière de stabilité mécanique et de respect des paramètres électriques de la cellule, le but étant de montrer que la cellule est apte à supporter une exposition prolongée aux climats définis dans le domaine d'application. Enfin, les cellules photovoltaïques à colorant intégrant de tels composés de formule (I) à titre d'électrolyte génèrent plus de photocourant (environ + 2 mA/cm²) qu'une cellule équivalente mettant en oeuvre un halogénure d'imidazolium à titre d'électrolyte.

Parmi les radicaux alkyle en C₁-C₆ mentionnés pour les radicaux R¹ à R⁴, on peut plus particulièrement citer les radicaux méthyle, éthyle, n-propyle, n-butyle. Parmi ces radicaux, on préfère les radicaux méthyle, éthyle et n-propyle.

Parmi les halogénures de formule (I) ci-dessus, les iodures sont préférés.

Parmi les composés de formule (I) ci-dessus, on peut en particulier citer :
- le iodure de 4-(3-méthoxy-3-oxopropyl)-1,3-diméthyl-1*H*-imidazol-3-ium,
- le iodure de 4-(3-éthoxy-3-oxopropyl)-1,3-diméthyl-1*H*-imidazol-3-ium,
- le iodure de 4-(3-méthoxy-3-oxopropyl)-1,3-diéthyl-1*H*-imidazol-3-ium,
- le iodure de 4-(3-méthoxy-3-oxopropyl)-1,3-dipropyl-1*H*-imidazol-3-ium,
- le iodure de 4-(3-méthoxy-3-oxopropyl)-1,3-dibutyl-1*H*-imidazol-3-ium,
- le iodure de 4-(3-méthoxy-3-oxopropyl)-1,3-dipentyl-1*H*-imidazol-3-ium,
- le iodure de 4-(3-méthoxy-3-oxopropyl)-1,3-dihexyl-1*H*-imidazol-3-ium,
- le iodure de 4-(-3-méthoxy-3-oxopropyl)-1-méthyl-3-propyl-1*H*-imidazol-3-ium,
- le iodure de 3-éthyl-4-(3-méthoxy-3-oxopropyl)-1-méthyl-1*H*-imidazol-3-ium,
- le iodure de 4-(3-méthoxy-3-oxopropyl)-1-méthyl-3-propyl-1*H*-imidazol-3-ium,
- le iodure de 3-butyl-4-(3-méthoxy-3-oxopropyl)-1-méthyl-1*H*-imidazol-3-ium,
- le iodure de 4-(3-méthoxy-3-oxopropyl)-1-méthyl-3-pentyl-1*H*-imidazol-3-ium,
- le iodure de 3-hexyl-4-(3-méthoxy-3-oxopropyl)-1-méthyl-1*H*-imidazol-3-ium,
- le iodure de 1-éthyl-4-(3-méthoxy-3-oxopropyl)-3-méthyl-1*H*-imidazol-3-ium,
- le iodure de 1,3-diéthyl-4-(-3-méthoxy-3-oxopropyl)-1*H*-imidazol-3-ium,
- le iodure de 1-éthyl-4-(3-méthoxy-3-oxopropyl)-3-propyl-1*H*-imidazol-3-ium,
- le iodure de 3-butyl-1-éthyl-4-(3-méthoxy-3-oxopropyl)-1*H*-imidazol-3-ium,
- le iodure de 1-éthyl-4-(3-méthoxy-3-oxopropyl)-3-pentyl-1*H*-imidazol-3-ium,
- le iodure de 1-éthyl-3-hexyl-4-(3-méthoxy-3-oxopropyl)-1*H*-imidazol-3-ium,
- le iodure de 4-(3-méthoxy-3-oxopropyl)-3-méthyl-1-propyl-1*H*-imidazol-3-ium,
- le iodure de 3-éthyl-4-(3-méthoxy-3-oxopropyl)-1-propyl-1*H*-imidazol-3-ium,
- le iodure de 4-(3-méthoxy-3-oxopropyl)-1,3-dipropyl-1*H*-imidazol-3-ium,
- le iodure de 3-butyl-4-(-3-méthoxy-3-oxopropyl)-1-propyl-1*H*-imidazol-3-ium,
- le iodure de 4-(3-méthoxy-3-oxopropyl)-3-pentyl-1-propyl-1*H*-imidazol-3-ium,
- le iodure de 3-hexyl-4-(3-méthoxy-3-oxopropyl)-1-propyl-1*H*-imidazol-3-ium,
- le iodure de 1-butyl-4-(3-méthoxy-3-oxopropyl)-3-méthyl-1*H*-imidazol-3-ium,
- le iodure de 1-butyl-3-éthyl-4-(3-méthoxy-3-oxopropyl)-1*H*-imidazol-3-ium,
- le iodure de 1-butyl-4-(3-méthoxy-3-oxopropyl)-3-propyl-1*H*-imidazol-3-ium,
- le iodure de 1,3-dibutyl-4-(3-méthoxy-3-oxopropyl)-1*H*-imidazol-3-ium,
- le iodure de 1-butyl-4-(3-méthoxy-3-oxopropyl)-3-pentyl-1*H*-imidazol-3-ium,
- le iodure de 1-butyl-3-hexyl-4-(3-méthoxy-3-oxopropyl)-1*H*-imidazol-3-ium,
- le iodure de 4-(3-méthoxy-3-oxopropyl)-3-méthyl-1-pentyl-1*H*-imidazol-3-ium,
- le iodure de 3-éthyl-4-(3-méthoxy-3-oxopropyl)-1-pentyl-1*H*-imidazol-3-ium,
- le iodure de 4-(3-méthoxy-3-oxopropyl)-1-pentyl-3-propyl-1*H*-imidazol-3-ium,
- le iodure de 3-butyl-4-(3-méthoxy-3-oxopropyl)-1-pentyl-1*H*-imidazol-3-ium,
- le iodure de 4-(3-méthoxy-3-oxopropyl)-1,3-dipentyl-1*H*-imidazol-3-ium,
- le iodure de 3-hexyl-4-(3-méthoxy-3-oxopropyl)-1-pentyl-1*H*-imidazol-3-ium,
- le iodure de 1-hexyl-4-(3-méthoxy-3-oxopropyl)-3-méthyl-1*H*-imidazol-3-ium,
- le iodure de 3-éthyl-1-hexyl-4-(3-méthoxy-3-oxopropyl)-1*H*-imidazol-3-ium,
- le iodure de 1-hexyl-4-(3-méthoxy-3-oxopropyl)-3-propyl-1*H*-imidazol-3-ium,
- le iodure de 3-butyl-1-hexyl-4-(3-méthoxy-3-oxopropyl)-1*H*-imidazol-3-ium,
- le iodure de 1-hexyl-4-(3-méthoxy-3-oxopropyl)-3-pentyl-1*H*-imidazol-3-ium,
- le iodure de 1,3-dihexyl-4-(3-méthoxy-3-oxopropyl)-1*H*-imidazol-3-ium,
- le bromure de 3-éthyl-4-(3-méthoxy-3-oxopropyl)-1-méthyl-1*H*-imidazol-3-ium,
- le bromure de 4-(3-méthoxy-3-oxopropyl)-1-méthyl-3-propyl-1*H*-imidazol-3-ium,
- le bromure de 3-butyl-4-(3-méthoxy-3-oxopropyl)-1-méthyl-1*H*-imidazol-3-ium,
- le bromure de 4-(3-méthoxy-3-oxopropyl)-1-méthyl-3-pentyl-1*H*-imidazol-3-ium,
- le bromure de 3-hexyl-4-(3-méthoxy-3-oxopropyl)-1-méthyl-1*H*-imidazol-3-ium,
- le bromure de 1-éthyl-4-(3-méthoxy-3-oxopropyl)-3-méthyl-1*H* imidazol-3-ium,
- le bromure de 1,3-diéthyl-4-(-3-méthoxy-3-oxopropyl)-1*H*-imidazol-3-ium,
- le bromure de 1-éthyl-4-(3-méthoxy-3-oxopropyl)-3-propyl-1*H*-imidazol-3-ium,
- le bromure de 3-butyl-1-éthyl-4-(3-méthoxy-3-oxopropyl)-1*H*-imidazol-3-ium,
- le bromure de 1-éthyl-4-(3-méthoxy-3-oxopropyl)-3-pentyl-1*H*-imidazol-3-ium,
- le bromure de 1-éthyl-3-hexyl-4-(3-méthoxy-3-oxopropyl)-1*H*-imidazol-3-ium,
- le bromure de 4-(3-méthoxy-3-oxopropyl)-3-méthyl-1-propyl-1*H*-imidazol-3-ium,
- le bromure de 3-éthyl-4-(3-méthoxy-3-oxopropyl)-1-propyl-1*H*-imidazol-3-ium,
- le bromure de 4-(3-méthoxy-3-oxopropyl)-1,3-dipropyl-1*H*-imidazol-3-ium,
- le bromure de 3-butyl-4-(-3-méthoxy-3-oxopropyl)-1-propyl-1*H*-imidazol-3-ium,
- le bromure de 4-(3-méthoxy-3-oxopropyl)-3-pentyl-1-propyl-1*H*-imidazol-3-ium,
- le bromure de 3-hexyl-4-(3-méthoxy-3-oxopropyl)-1-propyl-1*H*-imidazol-3-ium,
- le bromure de 1-butyl-4-(3-méthoxy-3-oxopropyl)-3-méthyl-1*H*-imidazol-3-ium,
- le bromure de 1-butyl-3-éthyl-4-(3-méthoxy-3-oxopropyl)-1*H*-imidazol-3-ium,
- le bromure de 1-butyl-4-(3-méthoxy-3-oxopropyl)-3-propyl-1*H*-imidazol-3-ium,
- le bromure de 1,3-dibutyl-4-(3-méthoxy-3-oxopropyl)-1*H*-imidazol-3-ium,
- le bromure de 1-butyl-4-(3-méthoxy-3-oxopropyl)-3-pentyl-1*H*-imidazol-3-ium,
- le bromure de 1-butyl-3-hexyl-4-(3-méthoxy-3-oxopropyl)-1*H*-imidazol-3-ium,
- le bromure de 4-(3-méthoxy-3-oxopropyl)-3-méthyl-1-pentyl-1*H*-imidazol-3-ium,
- le bromure de 3-éthyl-4-(3-méthoxy-3-oxopropyl)-1-pentyl-1*H*-imidazol-3-ium,
- le bromure de 4-(3-méthoxy-3-oxopropyl)-1-pentyl-3-propyl-1*H*-imidazol-3-ium,
- le bromure de 3-butyl-4-(3-méthoxy-3-oxopropyl)-1-pentyl-1*H*-imidazol-3-ium,
- le bromure de 4-(3-méthoxy-3-oxopropyl)-1,3-dipentyl-1*H*-imidazol-3-ium,
- le bromure de 3-hexyl-4-(3-méthoxy-3-oxopropyl)-1-pentyl-1*H*-imidazol-3-ium,
- le bromure de 1-hexyl-4-(3-méthoxy-3-oxopropyl)-3-méthyl-1*H*-imidazol-3-ium,
- le bromure de 3-éthyl-1-hexyl-4-(3-méthoxy-3-oxopropyl)-1*H*-imidazol-3-ium,
- le bromure de 1-hexyl-4-(3-méthoxy-3-oxopropyl)-3-propyl-1*H*-imidazol-3-ium,
- le bromure de 3-butyl-1-hexyl-4-(3-méthoxy-3-oxopropyl)-1*H*-imidazol-3-ium,
- le bromure de 1-hexyl-4-(3-méthoxy-3-oxopropyl)-3-pentyl-1*H*-imidazol-3-ium,
- le bromure de 1,3-dihexyl-4-(3-méthoxy-3-oxopropyl)-1*H*-imidazol-3-ium.
- le iodure de 4-(3-méthoxy-3-oxopropyl)-1,2,3-triméthyl-1*H*-imidazol-3-ium,
- le iodure de 3-éthyl-4-(3-méthoxy-3-oxopropyl)-1,2-diméthyl-1*H*-imidazol-3-ium,
- le iodure de 4-(3-méthoxy-3-oxopropyl)-1,2-diméthyl-3-propyl-1*H*-imidazol-3-ium,
- le iodure de 3-butyl-4-(3-méthoxy-3-oxopropyl)-1,2-diméthyl-1*H*-imidazol-3-ium,
- le iodure de 4-(3-méthoxy-3-oxopropyl)-1,2-diméthyl-3-pentyl-1*H*-imidazol-3-ium,
- le iodure de 3-hexyl-4-(3-méthoxy-3-oxopropyl)-1,2-diméthyl-1*H*-imidazol-3-ium,
- le iodure de 1,3-diéthyl-4-(3-méthoxy-3-oxopropyl)-2-méthyl-1*H*-imidazol-3-ium,
- le iodure de 4-(3-méthoxy-3-oxopropyl)-2-méthyl-1,3-dipropyl-1*H*-imidazol-3-ium,
- le iodure de 1,3-dibutyl-4-(3-méthoxy-3-oxopropyl)-2-méthyl-1*H*-imidazol-3-ium,
- le iodure de 4-(3-méthoxy-3-oxopropyl)-2-méthyl-1,3-dipentyl-1*H*-imidazol-3-ium,
- le iodure de 1,3-dihexyl-4-(3-méthoxy-3-oxopropyl)-2-méthyl-1*H*-imidazol-3-ium,
- le iodure de 1-éthyl-3-hexyl-4-(3-méthoxy-3-oxopropyl)-2-méthyl-1*H*-imidazol-3-ium,
- le bromure de 4-(3-méthoxy-3-oxopropyl)-1-méthyl-3-propyl-1*H*-imidazol-3-ium,
- le bromure de 4-(3-méthoxy-3-oxopropyl)-1,2,3-triméthyl-1*H*-imidazol-3-ium,
- le bromure de 3-éthyl-4-(3-méthoxy-3-oxopropyl)-1,2-diméthyl-1*H*-imidazol-3-ium,
- le bromure de 4-(3-méthoxy-3-oxopropyl)-1,2-diméthyl-3-propyl-1*H*-imidazol-3-ium,
- le bromure de 3-butyl-4-(3-méthoxy-3-oxopropyl)-1,2-diméthyl-1*H*-imidazol-3-ium,
- le bromure de 4-(3-méthoxy-3-oxopropyl)-1,2-diméthyl-3-pentyl-1*H*-imidazol-3-ium,
- le bromure de 3-hexyl-4-(3-méthoxy-3-oxopropyl)-1,2-diméthyl-1*H*-imidazol-3-ium,
- le bromure de 1,3-diéthyl-4-(3-méthoxy-3-oxopropyl)-2-méthyl-1*H*-imidazol-3-ium,
- le bromure de 4-(3-méthoxy-3-oxopropyl)-2-méthyl-1,3-dipropyl-1*H*-imidazol-3-ium,
- le bromure de 1,3-dibutyl-4-(3-méthoxy-3-oxopropyl)-2-méthyl-1*H*-imidazol-3-ium,
- le bromure de 4-(3-méthoxy-3-oxopropyl)-2-méthyl-1,3-dipentyl-1*H*-imidazol-3-ium, et
- le iodure de 1-éthyl-3-hexyl-4-(3-méthoxy-3-oxopropyl)-2-méthyl-1*H*-imidazol-3-ium.

Le iodure de 4-(3-méthoxy-3-oxopropyl)-1,3-diméthyl-1*H*-imidazol-3-ium est un sel solide dont la température de fusion est d'environ 134,7°C tout comme le iodure de 4-(3-méthoxy-3-oxopropyl)-1,3-diméthyl-1*H*-imidazol-3-ium (température de fusion d'environ 111°C) et le iodure de 4-(3-éthoxy-3-oxopropyl)-1,3-diméthyl-1*H*-imidazol-3-ium. Les autres composés de formule (I), et en particulier le iodure de 4-(3-méthoxy-3-oxopropyl)-1-méthyl-3-propyl-1*H-*imidazol-3-ium, sont des liquides ioniques visqueux à température ambiante.

Des analyses thermogravimétriques sous air réalisées par les inventeurs ont montrés que les composés de formule (I) sont stables jusqu'à une température de 300°C environ, ce qui représente une stabilité nettement supérieure aux conditions de vieillissement accéléré d'une cellule solaire selon la norme IEC 61646 (1000 heures à 60°C/100 mW.cm⁻² d'illumination et 1000 heures à 85°C dans le noir).

Les composés de formule (I) tels que définis ci-dessus, peuvent être préparés selon un procédé simple et peu couteux à partir de l'acide urocanique. Si on le souhaite, l'acide urocanique peut être obtenue par désamination de la *L*-histidine de formule suivante : 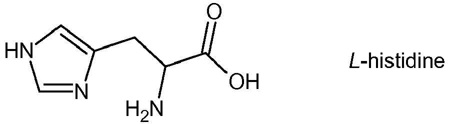 par voie enzymatique en présence d'une histidinase pour conduire à l'acide urocanique de formule suivante :

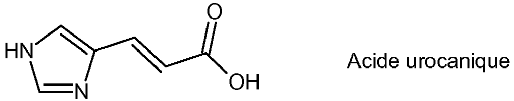

Il est également possible de se procurer l'acide urocanique directement dans la mesure où il s'agit d'un produit commercial (CAS N° 104-98-3).

Le procédé de synthèse des composés de formule (I) varie ensuite en fonction de la nature des radicaux définis pour R¹ à R⁴.

Lorsque R¹ est différent d'un atome d'hydrogène, c'est-à-dire lorsque R¹ représente un radical alkyle linéaire ayant de 1 à 10 atomes de carbone, la première étape du procédé consiste à réaliser l'estérification de la fonction acide carboxylique de l'acide urocanique en présence d'un alcool R'¹OH pour obtenir un composé (**1**), selon la réaction (i) suivante :

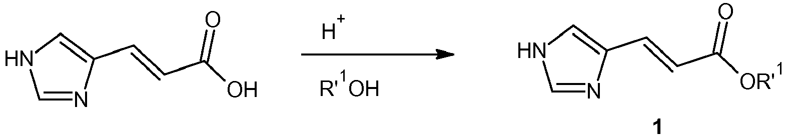

R'¹ étant un radical alkyle linéaire ayant de 1 à 10 atomes de carbone.

La réaction (i) est de préférence réalisée dans un solvant organique pouvant par exemple être choisi parmi les alcools inférieurs tels que l'éthanol, le diméthylformamide (DMF), le diméthylsulfoxyde (DMSO), le toluène, le tetrahydrofurane (THF), le dichlorométhane et le dioxane, à une température variant de la température ambiante à la température de reflux du solvant utilisé et à un pH acide (compris entre 2et 5 environ).

Le composé (**1**) est alors hydrogéné en présence d'un catalyseur tel que le palladium sur charbon, pour conduire à un composé (**2**) dans lequel R'¹ représente un radical alkyle linéaire ayant de 1 à 10 atomes de carbone, selon la réaction (ii) suivante :

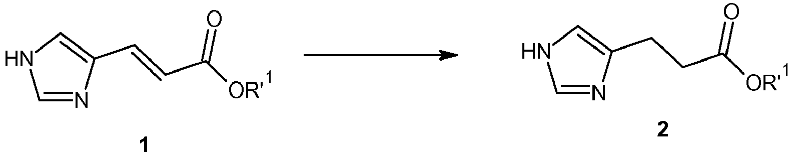

La réaction (ii) est de préférence réalisée dans un solvant organique tel que le méthanol.

Lorsque l'on souhaite obtenir un composé de formule (I) dans lequel R³ est un atome d'hydrogène et R⁴ est un radical alkyle identique au radical alkyle choisi pour R² (composé (**I-1**)) le composé (**2**), est ensuite engagé dans la réaction (iii-a) suivante : 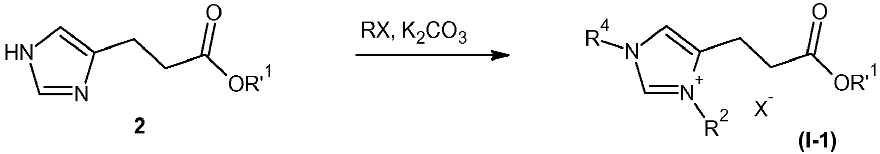 ladite réaction (iii-a) consistant à faire réagir ledit composé (**2**) avec un halogénure d'alkyle ou d'aryle RX dans lequel X représente l'iode ou le brome, R est un radical alkyle linéaire ou ramifié ayant de 1 à 15 atomes de carbone, de préférence de 1 à 6 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote, ledit radical R étant identique aux radicaux R² et R⁴ que l'on souhaite avoir sur le composé (**I-1**), dans un solvant organique tel que l'acétone, en présence de carbonate de potassium et à une température variant de la température ambiante à 56°C environ.

Les composés de formule (I) dans lesquels R³ est un atome d'hydrogène et R² est un radical alkyle ou aryle différent du radical alkyle ou aryle choisi pour R⁴ (composés (**I-2**)), peuvent être obtenus à partir d'un composé (**2**), selon la réaction (iii-b) suivante : 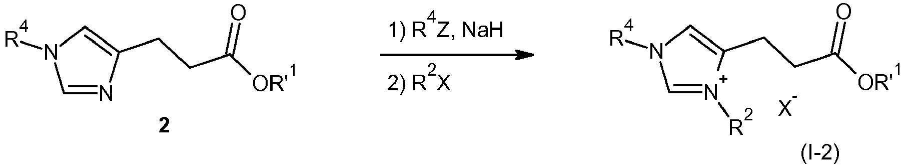 selon laquelle, dans une sous-étape 1), on réalise l'alkylation de l'atome d'azote en position 1 du cycle imidazole par un halogénure d'alkyle ou d'aryle. de formule R⁴Z dans lequel R⁴ est un radical alkyle linéaire ou ramifié ayant de 1 à 15 atomes de carbone, de préférence de 1 à 6 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote et Z représente un atome d'halogène (Cl, Br, I), puis dans une sous-étape 2), on fait réagir le composé intermédiaire ainsi obtenu avec un halogénure d'alkyle ou d'aryle RX dans lequel X est l'iode ou le brome et R est un radical alkyle linéaire ou ramifié ayant de 1 à 15 atomes de carbone, de préférence de 1 à 6 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote, R étant par ailleurs identique ou différent de R⁴.

La sous-étape 1) de la réaction (iii-a) peut être réalisée dans un solvant organique tel que par exemple le diméthylformamide, en présence d'une base forte telle que par exemple l'hydrure de sodium. La sous-étape 2) de la réaction iii-b) peut être réalisée dans les mêmes conditions que l'étape (iii-a) ci-dessus.

Les composés de formule (I) dans lesquels R² et R⁴, identiques ou différents, représentent un radical alkyle linéaire ou ramifié ayant de 1 à 15 atomes de carbone, de préférence de 1 à 6 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote, et R³ représente un radical alkyle linéaire ayant de 1 à 15 atomes de carbone, de préférence de 1 à 6 atomes de carbone, (composé (**I-3**)) peuvent être obtenus à partir d'un composé (**2**) selon la réaction suivante (iii-c) :

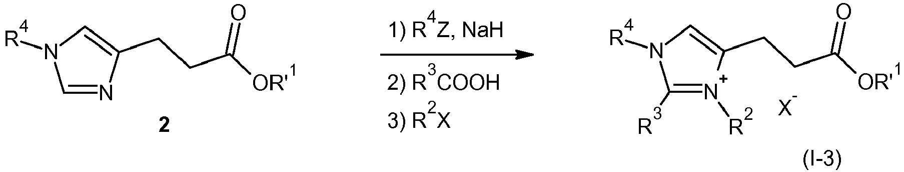

La sous-étape 1) peut être réalisée dans les mêmes conditions que la sous-étape 1) de la réaction (iii-b). La sous-étape 2) est réalisée en présence d'un acide carboxylique de formule R³COOH dans lequel R³ représente un atome d'hydrogène ou un radical alkyle linéaire ayant de 1 à 15 atomes de carbone, de préférence de 1 à 6 atomes de carbone. La sous-étape 2) est de préférence réalisée à pH acide dans un solvant organique tel que par exemple le méthanol, l'eau, le toluène ou le dioxane en présence de nitrate d'argent et d'un agent oxydant tel que le persulfate d'ammonium, à une température comprise entre la température ambiante et la température de reflux du solvant utilisé. La sous-étape 3) peut être réalisée dans un solvant organique tel que par exemple l'acétone ou sans solvant à une température comprise entre la température ambiante et la température de reflux du solvant du solvant utilisé le cas échéant, en utilisant un composé de formule (R²X dans lequel R² est un radical alkyle linéaire ou ramifié ayant de 1 à 15 atomes de carbone, de préférence de 1 à 6 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote et X représente l'atome d'iode.

Les composés de formule (I) dans lesquels R¹ est un atome d'hydrogène peuvent être obtenus par déprotection de la fonction acide carboxylique d'un composé de formules (I-1), (I-2) ou (I-3) telles que décrites précédemment, suivant le composé de formule (I) que l'on souhaite obtenir. L'étape de déprotection de la fonction acide carboxylique est de préférence réalisée à pH basique dans l'eau à une température comprise entre température ambiante et la température de reflux de l'eau.

Ce procédé est simple et peu couteux à mettre en oeuvre, et il permet d'accéder aux composés de formule (I) avec un très bon rendement.

Comme on l'a vu précédemment les composés de formule (I) sont des composés non hygroscopiques qui peuvent ainsi être avantageusement utilisés à titre de sel d'électrolyte d'un couple redox réversible dans une composition d'électrolyte d'une cellule photoélectrochimique basée sur la sensibilisation à la lumière de molécules photo-actives et en particulier d'une cellule photovoltaïque à colorant.

L'invention a donc également pour objet une cellule photoélectrochimique basée sur la sensibilisation à la lumière de molécules photo-actives comprenant :
- un ensemble formant une photoélectrode comprenant :
   i) un substrat transparent revêtu d'une couche d'un matériau d'électrode transparent et conducteur,
   ii) une couche photoactive formée sur ledit matériau d'électrode, ladite couche photoactive comprenant un matériau porteur transparent et au moins une molécule photoactive ;
- un ensemble formant une contre-électrode ; et
- une composition d'électrolyte interposée entre ladite couche photoactive et ladite contre-électrode, ladite cellule étant caractérisée en ce que ladite composition d'électrolyte comprend, dans un solvant d'électrolyte, un couple redox réversible (I₃⁻/I⁻) à base de diiode et d'au moins un composé de formule (I) tel que défini précédemment.

Selon une forme de réalisation préférée de l'invention, la cellule photoélectrochimique basée sur la sensibilisation à la lumière de molécules photo-actives est une cellule photovoltaïque à colorants. Egalement de façon préférée, la photoélectrode est une photoanode.
Le composé de formule (I) est de préférence choisi parmi le iodure de 4-(3-méthoxy-3-oxopropyl)-1,3-diméthyl-1*H*-imidazol-3-ium, le iodure de 4-(-3-méthoxy-3-oxopropyl)-1-méthyl-3-propyl-1*H*-imidazol-3-ium, le bromure de 4-(3-méthoxy-3-oxopropyl)-1,3-diméthyl-1*H*-imidazol-3-ium et le bromure de 4-(-3-méthoxy-3-oxopropyl)-1-méthyl-3-propyl-1*H*-imidazol-3-ium le iodure de 4-(3-éthoxy-3-oxopropyl)-1,3-diméthyl-1*H*-imidazol-3-ium, le iodure de 4-(3-méthoxy-3-oxopropyl)-1,3-diéthyl-1*H*-imidazol-3-ium, le iodure de 4-(3-méthoxy-3-oxopropyl)-1,3-dipropyl-1*H*-imidazol-3-ium, le iodure de 4-(3-méthoxy-3-oxopropyl)-1,3-dibutyl-1*H*-imidazol-3-ium, le iodure de 4-(3-méthoxy-3-oxopropyl)-1,3-dipentyl-1*H*-imidazol-3-ium, et le iodure de 4-(3-méthoxy-3-oxopropyl)-1,3-dihexyl-1*H*-imidazol-3-ium.

Parmi ces composés de formule (I), on préfère tout particulièrement le iodure de 4-(3-méthoxy-3-oxopropyl)-1,3-dimétliyl-1*H*-imidazol-3-ium, le iodure de 4-(-3-méthoxy-3-oxopropyl)-1-méthyl-3-propyl-1*H*-imidazol-3-ium, le bromure de 4-(3-méthoxy-3-oxopropyl)-1,3-diméthyl-1*H*-imidazol-3-ium et le bromure de 4-(-3-méthoxy-3-oxopropyl)-1-méthyl-3-propyl-1*H*-imidazol-3-ium.

Selon une forme de réalisation préférée de l'invention, la concentration du ou des composés de formule (I) au sein de la composition d'électrolyte varie de 0,1 à 5 mol/L environ, et encore plus préférentiellement de 0,5 à 1,5 mol/L environ.

La concentration en diiode au sein de la composition d'électrolyte varie de préférence de 0,001 à 0,5 mol/L environ, et encore plus préférentiellement de 0,01 à 0,2 mol/L environ.

Le ou les solvants d'électrolyte peuvent être choisis parmi les nitriles tels que par exemple l'acétonitrile, le propionitrile, le méthoxypropionitrile, le méthoxyacétonitrile, le glutaronitrile, le succinonitrile, le 3-hydroxypropionitrile, le benzonitrile, le phénylacétonitrile, le butyronitrile ou le valéronitrile ; le sulfolane ; le diméthylsulfoxyde ; le dioxane ; le tétrahydrofurane ; le nitrométhane ; les amides tels que par exemple le N,N-diméthylformamide ou le N,N-diméthylacétamide ; la N-méthyl-2-pyrrolidinone ; les carbonates tels que par exemple le carbonate de propylène, le carbonate d'éthylène, le carbonate de diméthylène ou le carbonate de butylène ; les éthylène glycols tels que par exemple le tétraglyme ; les alcools tels que par exemple l'éthanol, le méthanol, l'éthoxyéthanol, le méthoxyéthanol, le propanol, le butanol ou l'hexanol ; les cétones telles que par exemple la cyclopentanone, la benzylacétone ; les lactones comme la gamma-butyrolactone ou l'acétylbutyrolactone ; les anhydrides tels que par exemple l'anhydride acétique ; les éthers tels que par exemple le 2-méthoxyéthyléther ; l'eau ; les phtalates ; les adipates ; les citrates ; les sébaçates ; les maléates ; les benzoates et les succinates.

Le ou les solvants de l'électrolyte peuvent également être choisis parmi les liquides ioniques. Les liquides ioniques sont des sels comprenant l'association d'un anion et d'un cation dans des proportions stoechiométriques assurant la neutralité électrique du sel. Les cations les plus utilisés ont une structure du type ammonium, imidazolium, pyridinium, pyrrolidinium, phosphonium, thiazolium, quinolinium, tétraminium. Les anions sont de préférence choisis parmi les halogénures différents du iodure (F, Cl, Br) ; les anions tétrafluoroborate (BF₄), hexafluorophosphate (PF₆), sulfate (SO₄), hydrogénosulfate (HSO₄) ; les anions carboxylate, par exemple, les formiates (HCOO), acétate (CH₃COO), trifluoroacétate (CF₃COO), propanoate (CH₃-CH2-COO) ; les anions sulfonylimide, par exemple, les anions bis((trifluorométhyl)sulfonyl)imide ((CF₃-SO₂)₂N), bis(méthylsulfonyl)imide ((CH₃-SO₂)₂N) ; l'anion dicyanamide (N(CN)₂) ; les anions sulfonate tels que par exemple les méthylsulfonate (CH3SO₃), trifluorométhylsulfonate (CF₃SO₃), benzènesulfonate (C₆H₅-SO₃), p-toluènesulfonate (CH₃-C₆H4-SO₃), et perfluorobutylsulfonate (C₄F₉SO₃) ; les anions sulfinate tels que, par exemple, les trifluorométhanesulfinate (CF₃SO₂), perfluorobutylsulfinate (C₄F₉SO₂) ; les anions phosphate tels que par exemple les diméthyl phosphate ((CH₃O)₂PO₂), diéthyl phosphate ((C₂H₅O)₂PO₂), dihydrogénophosphate (H₂PO₄), hydrogénophosphate (HPO₄) et phosphate (PO₄) ; les anions phosphonate tels que, par exemple, les méthylphosphonate (CH₃PO₃H), éthylphosphonate (C₂H₅PO₃H) ; et les autres anions tels que, par exemple, l'hexafluoroarsénate (AsF₆), l'hexafluoroniobiate (NbF₆), et l'hexafluoroantimonate (SbF₆).

Lorsqu'un liquide ionique est utilisé à titre de solvant de la composition d'électrolyte, l'anion dudit liquide ionique doit en effet être différent de l'anion iodure de façon à ne pas interférer avec le couple redox réversible (I₃/I⁻) à base de diiode et d'au moins un composé de formule (I).

Parmi les liquides ioniques mentionnés ci-dessus, on peut plus particulièrement citer le bis-(trifluorométhanesulfonyl)imide de 1-éthyl-3-méthylimidazolium (EMI-TFSI), le bis-(trifluorométhanesulfonyl)imide de 1-butyl-3-méthylimidazolium (BMI-TFSI), l'hexafluorophosphate de 1-butyl-3-méthylimidazolium (BMI-PF₆), le bis(trifluorométhylsulfonyl)imide de 1-butyl-1-méthylpyrrolidinium et leurs mélanges. Parmi ces liquides ioniques, le BMI-TFSI est particulièrement préféré en raison de sa large fenêtre électrochimique.

La composition d'électrolyte peut en outre renfermer un ou plusieurs additifs destinés à améliorer les performances de la cellule photoélectrochimique. De tels additifs peuvent notamment être choisis parmi les dérivés de benzimidazole, tels que par exemple le N-méthylbenzimidazole et le N-butylbenzimidazole ; les dérivés de la pyridine, tels que par exemple la tert-butylpyridine (TBP) ; les dérivés de triazole, tels que par exemple le N,N-dipropyl-4H-1,2,4-triazol-4-amine ; les dérivés de pyrazole, tels que par exemple la 5-tert-butyl-1H-pyrazol-3-amine ; les dérivés d'imidazole, tels que par exemple le 1,2-diméthyl-1H-imidazole ; le carbonate d'éthylène ; le thiocyanate de guanidine ; l'acide chénodésoxycholique ; le iodure de magnésium ; et les sels alcalins de formules LiY ou NaY dans lesquels Y peut notamment être un anion I⁻, Br⁻, trifluorosulfonate, ClO₄⁻, NO₃⁻ ou TFSI⁻.

La concentration de ces additifs au sein de la composition électrolytique peut alors de préférence varier de 0,01 à 1 mol/L environ, et encore plus préférentiellement de 0,05 à 0,5 mol/L.

La composition d'électrolyte peut également renfermer en outre au moins un agent gélifiant de manière à former un gel. L'agent gélifiant peut être notamment choisi parmi les copolymères d'éthylène, d'acétate de vinyle, d'éthylène-acétate de vinyl (EVA), les polyuréthanes (PU), le polyvinyl butyral (PVB), les polyimides (PI), les polyamides (PA), le polystyrène (PS), le poly(fluorure de vinylidène) (PVDF), les polyéther-cétones (PEK), les polyéther-éther-cétones (PEEK), les copolymères d'épichlorohydrine, les polyoléfines, le poly(oxyde d'éthylène) (POE), les polyacrylates, le poly(méthacrylate de méthyl) (PMMA), les silicones ou bien leurs dérivés ou leurs monomères ou encore leurs pré-polymères.

La concentration de ces agents gélifiants au sein de la composition électrolytique peut alors de préférence varier de 0,01 à 1 mol/L environ, et encore plus préférentiellement de 0,05 à 0,5 mol/L.

Le substrat transparent de la photoélectrode est en un matériau qui peut être souple ou rigide, par exemple du verre ou une feuille en un matériau plastique telle que par exemple en un matériau à base de polyéthylène téréphtalate (PET), de polyéthylène naphtalène (PEN), de polycarbonate (PC), de polyimide (PI) ou de substrats fluorés.

Le matériau d'électrode de la photoélectrode est appliqué sur le substrat transparent. Il se présente généralement sous la forme d'une couche d'un oxyde conducteur transparent (TCO, selon l'expression anglophone « *Transparent Conducting Oxide* »), telles que des couches d'oxyde d'étain dopé au fluor (SnO₂:F ou FTO), d'oxyde d'indium et d'étain (In₂O₃:SnO₂ ou ITO), d'oxyde d'indium dopé à l'antimoine (In₂O₃:Sb), et d'oxyde de zinc dopé à l'aluminium (ZnO:Al), au gallium (ZnO:Ga) ou à l'indium (ZnO:In).

Le matériau porteur transparent de la couche photoactive de la photoanode est de préférence une couche mésoporeuse d'un oxyde métallique, par exemple à base d'oxyde de titane, d'oxyde de zinc, d'oxyde d'étain, d'oxyde de niobium, d'oxyde de tungstène, d'oxyde de strontium, d'oxyde de zirconium ou de l'un de leurs mélanges. Selon une forme de réalisation particulièrement préférée de l'invention, le matériau porteur transparent de la couche photoactive de la photoanode est une couche nanoporeuse d'oxyde de titane ou d'un oxyde ternaire à base de titane, d'étain ou de zinc tel que par exemple BaTiO₃, BaSnO₃, Li₄Ti₅O₁₂, et ZnSn₂O₄.

La molécule photoactive de la couche photoactive peut être choisie parmi tous les colorants habituellement utilisés dans les cellules photovoltaïques à colorant et bien connus de l'homme du métier. On peut en particulier mentionner les colorants organiques de la série des cyanines, des mérocyanines, des oxonols, des xanthènes, des squaryliums, des polyméthines, des coumarines, des riboflavines, et des périlènes, des complexes colorants tels que des complexes de ruthénium ou d'osmium ou des dérivés de phtalocyanines métalliques, des dérivés de porphyrines métalliques ou des dérivés de la chlorophylle.

Parmi les complexes de ruthénium, on peut plus particulièrement citer les complexes au ruthénium contenant des ligands pyridyle tels que par exemple le complexe hétéroleptique hydrophobe de formule RuLL'(NCS)₂ avec L = acide 2,2'-bipyridyle-4,4'-dicarboxylique et L' = 4,4'-bis-(5-(hexylthio)thiophène-2-yl)-2,2'-bipyridine.

La molécule photoactive est adsorbée sur le matériau porteur, par exemple par immersion du matériau porteur dans une solution contenant cette molécule photoactive.

La contre-électrode est de préférence constituée d'un contre-substrat transparent revêtu d'une couche d'un matériau formant la contre-électrode transparente, telle qu'une couche de SnO₂:F ou de ZnO dopé ou d'un empilement de couches conductrices transparentes telles que par exemple un ensemble verre + TCO ou métal. Le contre-substrat peut être choisi parmi les mêmes substrats que ceux mentionnés précédemment pour le substrat transparent de la photoanode.

Une couche de catalyseur est par ailleurs formée sur le matériau formant la contre-électrode, la couche de catalyseur pouvant par exemple être une couche de platine ou de carbone ou de chalcogénure inorganique.

La cellule photovoltaïque conforme à l'invention peut être préparée selon les techniques connues de l'homme du métier, et telles que par exemple par enduction au rouleau (« *roll coating* » ou « *Roll to Roll* »), flexographie, sérigraphie, pulvérisation cathodique, trempage-retrait (« dip coating »), enduction à la tournette (« *spin coating* »), application au couteau à pelliculer (« *doctor blade* »), etc... ou un mélange de ces techniques. Les systèmes assemblés peuvent être monolithiques, laminés en structure ouverte ou fermées.

Enfin, certains des composés de formule (I) ci-dessus sont nouveaux en soi et constituent à ce titre un autre objet de l'invention. Ces composés répondent à la formule (I') suivante : 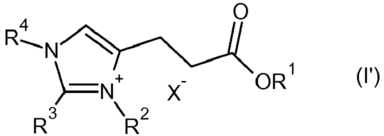 dans laquelle :
- R¹ représente un atome d'hydrogène ou un radical alkyle linéaire ayant de 1 à 10 atomes de carbone,
- R² et R⁴ identiques ou différents représentent chacun un radical alkyle linéaire ou ramifié ayant de 1 à 15 atomes de carbone, de préférence de 1 à 6 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote,
- R³ représente un atome d'hydrogène, un radical alkyle linéaire ou ramifié ayant de 1 à 15 atomes de carbone,
- X⁻ représente un anion iodure ou bromure,
étant entendu que :
- lorsque X⁻ représente un anion iodure, que R¹ représente un radical méthyle, et que R³ est un atome d'hydrogène, alors les radicaux R² et R⁴ ne peuvent représenter simultanément un radical méthyle ou un radical éthyle,
- lorsque X⁻ représente un anion bromure, que R¹ représente un radical méthyle, et que R³ est un atome d'hydrogène, alors les radicaux R² et R⁴ ne peuvent représenter simultanément un radical benzyle,
- lorsque X⁻ représente un anion bromure, que R¹ représente un radical méthyle, que R² est un radical butyle et que R³ est un atome d'hydrogène, alors le radical R⁴ est différent d'un radical méthyle, éthyle ou benzyle,
- lorsque X⁻ représente un anion iodure, que R¹ et R² représentent un radical méthyle, et que R³ est un atome d'hydrogène, alors le radical R⁴ est différent d'un radical trityle, et
- lorsque X⁻ représente un anion bromure, que R¹ représente un radical méthyle, que R² est un radical butyle et que R³ est un radical isopropyle, alors le radical R⁴ est différent d'un radical méthyle.

Les composés exclus de la formule (I') ci-dessus sont des composés ayant déjà été décrits à titre de liquide ionique dans l'article de R. K. Dubey et al., Synthetic Communications, 2012, 42, 2207-2216 ou dans la demande internationale WO 2010/052253 qui décrit en particulier l'utilisation du iodure de 5-(2-méthoxycarbonyléthyl)-1-méthyl-3-trityl-3H-imidazolium à titre de composé intermédiaire pour la synthèse de dérivés d'oxoazétidine utiles en médecine humaine ou en cosmétique. Leur utilisation à titre de sel d'électrolyte dans une composition d'électrolyte d'une cellule photoélectrochimique n'a cependant jamais été décrite.

Les composés de formule (I') peuvent être préparés selon le même procédé que celui utilisé pour préparer les composés de formule (I) détaillé ci-dessus.

Parmi les composés de formule (I') ci-dessus, on peut en particulier citer :
- le iodure de 4-(3-éthoxy-3-oxopropyl)-1,3-dimétliyl-1*H*-imidazol-3-ium,
- le iodure de 4-(3-méthoxy-3-oxopropyl)-1,3-dipropyl-1*H*-imidazol-3-ium,
- le iodure de 4-(3-méthoxy-3-oxopropyl)-1,3-dibutyl-1*H*-imidazol-3-ium,
- le iodure de 4-(3-méthoxy-3-oxopropyl)-1,3-dipentyl-1*H*-imidazol-3-ium,
- le iodure de 4-(3-méthoxy-3-oxopropyl)-1,3-dihexyl-1*H*-imidazol-3-ium,
- le iodure de 3-éthyl-4-(3-méthoxy-3-oxopropyl)-1-méthyl-1*H*-imidazol-3-ium,
- le iodure de 4-(3-méthoxy-3-oxopropyl)-1-méthyl-3-propyl-1*H*-imidazol-3-ium,
- le iodure de 3-butyl-4-(3-méthoxy-3-oxopropyl)-1-méthyl-1*H*-imidazol-3-ium,
- le iodure de 4-(3-méthoxy-3-oxopropyl)-1-méthyl-3-pentyl-1*H*-imidazol-3-ium,
- le iodure de 3-hexyl-4-(3-méthoxy-3-oxopropyl)-1-méthyl-1*H*-imidazol-3-ium,
- le iodure de 1-éthyl-4-(3-méthoxy-3-oxopropyl)-3-méthyl-1*H*-imidazol-3-ium,
- le iodure de 1-éthyl-4-(3-méthoxy-3-oxopropyl)-3-propyl-1*H*-imidazol-3-ium,
- le iodure de 3-butyl-1-éthyl-4-(3-méthoxy-3-oxopropyl)-1*H*-imidazol-3-ium,
- le iodure de 1-éthyl-4-(3-méthoxy-3-oxopropyl)-3-pentyl-1*H*-imidazol-3-ium,
- le iodure de 1-éthyl-3-hexyl-4-(3-méthoxy-3-oxopropyl)-1*H*-imidazol-3-ium,
- le iodure de 4-(3-méthoxy-3-oxopropyl)-3-méthyl-1-propyl-1*H*-imidazol-3-ium,
- le iodure de 3-éthyl-4-(3-méthoxy-3-oxopropyl)-1-propyl-1*H*-imidazol-3-ium,
- le iodure de 4-(3-méthoxy-3-oxopropyl)-1,3-dipropyl-1*H*-imidazol-3-ium,
- le iodure de 3-butyl-4-(-3-méthoxy-3-oxopropyl)-1-propyl-1*H*-imidazol-3-ium,
- le iodure de 4-(3-méthoxy-3-oxopropyl)-3-pentyl-1-propyl-1*H*-imidazol-3-ium,
- le iodure de 3-hexyl-4-(3-méthoxy-3-oxopropyl)-1-propyl-1*H*-imidazol-3-ium,
- le iodure de 1-butyl-4-(3-méthoxy-3-oxopropyl)-3-méthyl-1*H*-imidazol-3-ium,
- le iodure de 1-butyl-3-éthyl-4-(3-méthoxy-3-oxopropyl)-1*H*-imidazol-3-ium,
- le iodure de 1-butyl-4-(3-méthoxy-3-oxopropyl)-3-propyl-1*H*-imidazol-3-ium,
- le iodure de 1,3-dibutyl-4-(3-méthoxy-3-oxopropyl)-1*H*-imidazol-3-ium,
- le iodure de 1-butyl-4-(3-méthoxy-3-oxopropyl)-3-pentyl-1*H*-imidazol-3-ium,
- le iodure de 1-butyl-3-hexyl-4-(3-méthoxy-3-oxopropyl)-1*H*-imidazol-3-ium,
- le iodure de 4-(3-méthoxy-3-oxopropyl)-3-méthyl-1-pentyl-1*H*-imidazol-3-ium,
- le iodure de 3-éthyl-4-(3-méthoxy-3-oxopropyl)-1-pentyl-1*H*-imidazol-3-ium,
- le iodure de 4-(3-méthoxy-3-oxopropyl)-1-pentyl-3-propyl-1*H*-imidazol-3-ium,
- le iodure de 3-butyl-4-(3-méthoxy-3-oxopropyl)-1-pentyl-1*H*-imidazol-3-ium,
- le iodure de 4-(3-méthoxy-3-oxopropyl)-1,3-dipentyl-1*H*-imidazol-3-ium,
- le iodure de 3-hexyl-4-(3-méthoxy-3-oxopropyl)-1-pentyl-1*H*-imidazol-3-ium,
- le iodure de 1-hexyl-4-(3-méthoxy-3-oxopropyl)-3-méthyl-1*H*-imidazol-3-ium,
- le iodure de 3-éthyl-1-hexyl-4-(3-méthoxy-3-oxopropyl)-1*H*-imidazol-3-ium,
- le iodure de 1-hexyl-4-(3-méthoxy-3-oxopropyl)-3-propyl-1*H*-imidazol-3-ium,
- le iodure de 3-butyl-1-hexyl-4-(3-méthoxy-3-oxopropyl)-1*H*-imidazol-3-ium,
- le iodure de 1-hexyl-4-(3-méthoxy-3-oxopropyl)-3-pentyl-1*H*-imidazol-3-ium,
- le iodure de 1,3-dihexyl-4-(3-méthoxy-3-oxopropyl)-1*H*-imidazol-3-ium,
- le bromure de 3-éthyl-4-(3-méthoxy-3-oxopropyl)-1-méthyl-1*H*-imidazol-3-ium,
- le bromure de 4-(3-méthoxy-3-oxopropyl)-1-méthyl-3-propyl-1*H*-imidazol-3-ium,
- le bromure de 4-(3-méthoxy-3-oxopropyl)-1-méthyl-3-pentyl-1*H*-imidazol-3-ium,
- le bromure de 3-hexyl-4-(3-méthoxy-3-oxopropyl)-1-méthyl-1*H*-imidazol-3-ium,
- le bromure de 1-éthyl-4-(3-méthoxy-3-oxopropyl)-3-méthyl-1*H*-imidazol-3-ium,
- le bromure de 1,3-diéthyl-4-(-3-méthoxy-3-oxopropyl)-1*H*-imidazol-3-ium,
- le bromure de 1-éthyl-4-(3-méthoxy-3-oxopropyl)-3-propyl-1*H*-imidazol-3-ium,
- le bromure de 1-éthyl-4-(3-méthoxy-3-oxopropyl)-3-pentyl-1*H*-imidazol-3-ium,
- le bromure de 1-éthyl-3-hexyl-4-(3-méthoxy-3-oxopropyl)-1*H*-imidazol-3-ium,
- le bromure de 4-(3-méthoxy-3-oxopropyl)-3-méthyl-1-propyl-1*H*-imidazol-3-ium,
- le bromure de 3-éthyl-4-(3-méthoxy-3-oxopropyl)-1-propyl-1*H*-imidazol-3-ium,
- le bromure de 4-(3-méthoxy-3-oxopropyl)-1,3-dipropyl-1*H*-imidazol-3-ium,
- le bromure de 3-butyl-4-(-3-méthoxy-3-oxopropyl)-1-propyl-1*H*-imidazol-3-ium,
- le bromure de 4-(3-méthoxy-3-oxopropyl)-3-pentyl-1-propyl-1*H*-imidazol-3-ium,
- le bromure de 3-hexyl-4-(3-méthoxy-3-oxopropyl)-1-propyl-1*H*-imidazol-3-ium,
- le bromure de 1-butyl-4-(3-méthoxy-3-oxopropyl)-3-propyl-1*H*-imidazol-3-ium,
- le bromure de 1,3-dibutyl-4-(3-méthoxy-3-oxopropyl)-1*H*-imidazol-3-ium,
- le bromure de 1-butyl-4-(3-méthoxy-3-oxopropyl)-3-pentyl-1*H*-imidazol-3-ium,
- le bromure de 1-butyl-3-hexyl-4-(3-méthoxy-3-oxopropyl)-1*H*-imidazol-3-ium,
- le bromure de 4-(3-méthoxy-3-oxopropyl)-3-méthyl-1-pentyl-1*H*-imidazol-3-ium,
- le bromure de 3-éthyl-4-(3-méthoxy-3-oxopropyl)-1-pentyl-1*H*-imidazol-3-ium,
- le bromure de 4-(3-méthoxy-3-oxopropyl)-1-pentyl-3-propyl-1*H*-imidazol-3-ium,
- le bromure de 3-butyl-4-(3-méthoxy-3-oxopropyl)-1-pentyl-1*H*-imidazol-3-ium,
- le bromure de 4-(3-méthoxy-3-oxopropyl)-1,3-dipentyl-1*H*-imidazol-3-ium,
- le bromure de 3-hexyl-4-(3-méthoxy-3-oxopropyl)-1-pentyl-1*H*-imidazol-3-ium,
- le bromure de 1-hexyl-4-(3-méthoxy-3-oxopropyl)-3-méthyl-1*H*-imidazol-3-ium,
- le bromure de 3-éthyl-1-hexyl-4-(3-méthoxy-3-oxopropyl)-1*H*-imidazol-3-ium,
- le bromure de 1-hexyl-4-(3-méthoxy-3-oxopropyl)-3-propyl-1*H*-imidazol-3-ium,
- le bromure de 3-butyl-1-hexyl-4-(3-méthoxy-3-oxopropyl)-1*H*-imidazol-3-ium,
- le bromure de 1-hexyl-4-(3-méthoxy-3-oxopropyl)-3-pentyl-1*H*-imidazol-3-ium,
- le bromure de 1,3-dihexyl-4-(3-méthoxy-3-oxopropyl)-1*H*-imidazol-3-ium,
- le iodure de 4-(3-méthoxy-3-oxopropyl)-1,2,3-triméthyl-1*H*-imidazol-3-ium,
- le iodure de 3-éthyl-4-(3-méthoxy-3-oxopropyl)-1,2-diméthyl-1*H* imidazol-3-ium,
- le iodure de 4-(3-méthoxy-3-oxopropyl)-1,2-diméthyl-3-propyl-1*H* imidazol-3-ium,
- le iodure de 3-butyl-4-(3-méthoxy-3-oxopropyl)-1,2-diméthyl-1*H*-imidazol-3-ium,
- le iodure de 4-(3-méthoxy-3-oxopropyl)-1,2-diméthyl-3-pentyl-1*H*-imidazol-3-ium,
- le iodure de 3-hexyl-4-(3-méthoxy-3-oxopropyl)-1,2-diméthyl-1*H*-imidazol-3-ium,
- le iodure de 1,3-diéthyl-4-(3-méthoxy-3-oxopropyl)-2-méthyl-IH-imidazol-3-ium,
- le iodure de 4-(3-méthoxy-3-oxopropyl)-2-méthyl-1,3-dipropyl-1*H*-imidazol-3-ium,
- le iodure de 1,3-dibutyl-4-(3-méthoxy-3-oxopropyl)-2-méthyl-1*H*-imidazol-3-ium,
- le iodure de 4-(3-méthoxy-3-oxopropyl)-2-méthyl-1,3-dipentyl-1*H*-imidazol-3-ium,
- le iodure de 1,3-dihexyl-4-(3-méthoxy-3-oxopropyl)-2-méthyl-1*H*-imidazol-3-ium,
- le iodure de 1-éthyl-3-hexyl-4-(3-méthoxy-3-oxopropyl)-2-méthyl-1*H*-imidazol-3-ium,
- le bromure de 4-(3-méthoxy-3-oxopropyl)-1-méthyl-3-propyl-1*H*-imidazol-3-ium,
- le bromure de 4-(3-méthoxy-3-oxopropyl)-1,2,3-triméthyl-1*H*-imidazol-3-ium,
- le bromure de 3-éthyl-4-(3-méthoxy-3-oxopropyl)-1,2-diméthyl-1*H*-imidazol-3-ium,
- le bromure de 4-(3-méthoxy-3-oxopropyl)-1,2-diméthyl-3-propyl-1*H*-imidazol-3-ium,
- le bromure de 3-butyl-4-(3-méthoxy-3-oxopropyl)-1,2-diméthyl-1*H*-imidazol-3-ium,
- le bromure de 4-(3-méthoxy-3-oxopropyl)-1,2-diméthyl-3-pentyl-1*H*-imidazol-3-ium,
- le bromure de 3-hexyl-4-(3-méthoxy-3-oxopropyl)-1,2-diméthyl-1*H*-imidazol-3-ium,
- le bromure de 1,3-diéthyl-4-(3-méthoxy-3-oxopropyl)-2-méthyl-1*H*-imidazol-3-ium,
- le bromure de 4-(3-méthoxy-3-oxopropyl)-2-méthyl-1,3-dipropyl-1*H*-imidazol-3-ium,
- le bromure de 1,3-dibutyl-4-(3-méthoxy-3-oxopropyl)-2-méthyl-1*H*-imidazol-3-ium,
- le bromure de 4-(3-méthoxy-3-oxopropyl)-2-méthyl-1,3-dipentyl-1*H*-imidazol-3-ium, et
- le iodure de 1-éthyl-3-hexyl-4-(3-méthoxy-3-oxopropyl)-2-méthyl-1*H*-imidazol-3-ium.

La présente invention est illustrée par les exemples de réalisation suivants, auxquels elle n'est cependant pas limitée.

### EXEMPLES

Les matières premières suivantes ont été utilisées dans les exemples :
- N-butylbenzimidazole (Merck) ;
- Acide urocanique ; méthanol anhydre ; sulfate de sodium ; acide sulfurique concentré ; carbonate de sodium; cyclohexane ; palladium sur charbon à 10 % (Pd/C à 10 %) ; carbonate de potassium ; iodure de méthyle ; iodure d'éthyle ; iodure de potassium; 1-bromopropane ; 1-iodopropane ; 1-iodobutane ; 1-iodopentane ; 1-iodohexane ; Diiode ; thiocyanate de guanidinium (Sigma-Aldrich) ;
- Acétate d'éthyle ; acétone ; dichlorométhane (VWR) ;
- 3-méthoxypropionitrile (Alfa Aesar) ;
- 1,3-diméthylimidazolium (DMII) (Solvionic)

### EXEMPLE 1

### Synthèse du iodure de 4-(3-méthoxy-3-oxopropyl)-1,3-diméthyl-1H-imidazol-3-ium (composé 3a)

Dans cet exemple, a été réalisé la synthèse d'un composé de formule (I) dans lequel les radicaux R¹, R² et R⁴ sont identiques et représentent un radical méthyle, R³ représentant un atome d'hydrogène :

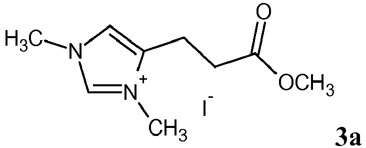

### 1) Première étape : Synthèse de l'ester méthylique de l'acide urocanique (composé 1)

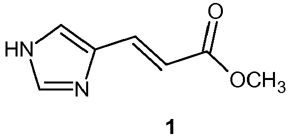

On a ajouté 11,5 mL d'acide sulfurique concentré à une solution d'acide urocanique (20 g, 145 mmol) et de sulfate de sodium (2,8 g) dans le méthanol anhydre (215 mL). Après agitation pendant une nuit à 70°C, le mélange réactionnel a été refroidi à température ambiante et filtré. Le solvant a ensuite été éliminé sous pression réduite et de l'eau a été ajoutée (1 mL). La solution ainsi obtenue a été neutralisée jusqu'à pH neutre par ajout d'une solution saturée de carbonate de sodium. La solution a ensuite été extraite 4 fois à l'acétate d'éthyle, puis les phases organiques ont été réunies et séchées sur sulfate de sodium. Le solvant a ensuite été éliminé sous pression réduite. Après solubilisation du précipité avec une quantité minimale d'acétate d'éthyle, 300 mL de cyclohexane ont été ajoutés. Après filtration, le composé **1** attendu a été obtenu sous la forme d'un solide blanc (20 g, rendement 92 %).

Température de fusion : 74-76°C
RMN ¹H (DMSOd₆, 300 MHz) : δ (ppm) 7,80 (s, 1 H, NCHN) ; 7,55 (d, 1 H, *J* = 15,6 Hz, C*H*=CH) ; 7,51 (s, 1 H, NCH) ; 6,40 (d, 1 H, *J* = 15,6 Hz, CH=C*H*) ; 3,65 (s, 3 H, OCH₃)
RMN ¹³C (DMSOd₆, 75 MHz) : δ (ppm) 167,7 (CO) ; 138,2 (NCHN) ; 136,3 (CH=) ; 134,7 (Cq) ; 123,9 (CH=) ; 111,1 (NCH) ; 51,5 (OCH₃).

Masse molaire calculée pour C₇H₈N₂O₂Na⁺ : 175 g·mol⁻¹ ; Trouvée 175 g·mo⁻¹.

### 2) Deuxième étape : Synthèse du méthyl 3-(1H-imidazol-4-yl)propanoate (composé 2)

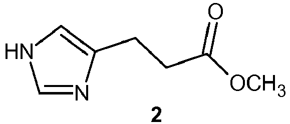

A une solution de 20 g (133 mmol) de l'ester méthylique de l'acide urocanique (composé la obtenu à l'étape 1) précédente) dans 150 mL de méthanol, on a ajouté 1,5 g de Pd/C à 10 % sous pression de dihydrogène. Après hydrogénation pendant 14 heures à température ambiante, le milieu réactionnel a été filtré sur célite. Le filtrat a ensuite été évaporé sous pression réduite pour conduire à 19,6 g du composé **2** attendu (rendement 95 %) sous la forme d'un sirop légèrement orange.

RMN ¹H (CDCl₃, 300 MHz) : δ (ppm) 9,65 (s, 1 H, NCHN) ; 7,55 (s, 1 H, NCH) ; 6,77 (s, 1 H, NH) ; 3,61 (s, 3 H, OCH₃) ; 2,90 (t, 2 H, *J* = 7.5 Hz, CH₂) ; 2,63 (t, 2 H, *J* = 7.5 Hz, CH₂)
RMN ¹³C (CDCl₃, 75 MHz) : δ (ppm) 173,6 (CO) ; 135,6 (Cq) ; 134,7 (NCHN) ; 116,7 (NCH) ; 51,6 (OCH₃) ; 33,8 (CH₂) ; 22,1 (CH₂).

Masse molaire calculée pour C₇H₁₀N₂O₂Na⁺: 177 g/mol, trouvée 177 g/mol.

### 3) Troisième étape : Synthèse de l'iodure de 4-(3-méthoxy-3-oxopropyl)-1,3-diiméthyl-1H-imidazol-3-ium (composé 3a)

On a ajouté 5 g du composé **2** obtenu ci-dessus à l'étape précédente (32,4 mmol), 9 g de carbonate de potassium (65 mmol) et 8,1 mL d'iodure de méthyle dans 100 mL d'acétone. Le mélange réactionnel a été maintenu à 70°C sous agitation pendant 1 heure. Après refroidissement jusqu'à la température ambiante, le mélange réactionnel a été filtré et le solvant a été éliminé sous pression réduite. Le précipité formé a été solubilisé dans le dichlorométhane, puis le mélange a été filtré et le solvant éliminé sous pression réduite. Le mélange réactionnel a ensuite été trituré dans le cyclohexane et le solide a été filtré pour conduire à 8,6 g du compose **3a** attendu sous la forme d'un solide blanc (rendement 86 %).

Température de fusion : 134,7°C
RMN ¹H (CDCl₃, 300 MHz) : δ (ppm) 9,76 (s, 1 H, NCHN) ; 7,35 (s, 1 H, NCH) ; 3,99 ; 3,94 (s, 6 H, 2 x NCH₃) ; 3,64 (s, 3 H, OCH₃) ; 2,95 (dd, 2 H, *J* = 6,5 Hz, *J* = 7.5 Hz, CH₂) ; 2,73 (t, 2 H, *J* = 7,0 Hz, CH₂)
RMN ¹³C (CDCl₃, 75 MHz): δ (ppm) 171,7 (CO) ; 136,7 (NCHN) ; 134,5 (Cq) ; 120,7 (NCH) ; 52,2 (OCH₃) ; 36,9 ; 34,4 (2 x NCH₃) ; 31,8 (CH₂) ; 18,9 (CH₂).

Masse molaire calculée pour C₉H₁₅N₂O₂⁺ : 183 g/mol, trouvée 183 g/mol.

Une analyse calorimétrique sous air du composé **3a** a été réalisée à l'aide d'un calorimètre vendu sous la dénomination commerciale DSC204 F1 par la société Netzsch.

Une analyse thermogravimétrique sous air du composé **3a** a également été réalisée après stockage à l'air pendant 3 semaines.

A titre comparatif, une analyse thermogravimétrique d'un iodure d'imidazolium ne faisant pas partie de l'invention, à savoir l'iodure de 1,3-diméthylimidazolium (DMII) a également été réalisée.

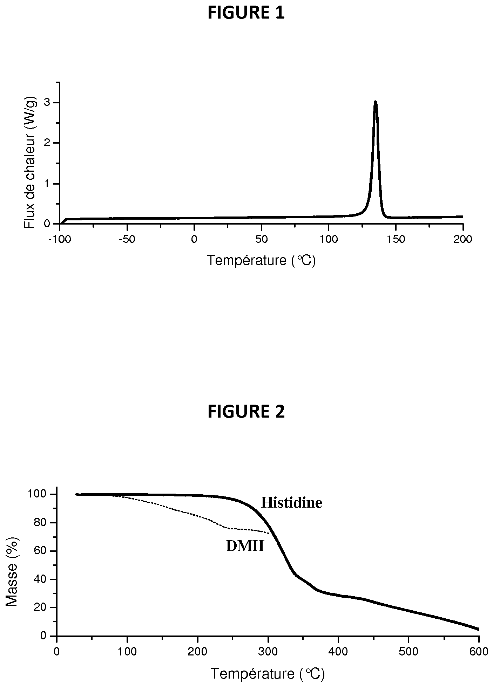

Les résultats obtenus sont reportés sur les figures 1 et 2 annexées. La figure 1 donne les résultats de l'analyse calorimétrique du composé **3a.** Sur cette figure, le flux de chaleur (en W/g) est fonction de la température (en °C). La figure 2 donne les résultats de l'analyse thermogravimétrique du composé **3a** (trait continu) comparativement au DMII (trait discontinu).

Les résultats de l'analyse thermogravimétrique sous air montrent que le composé **3a** est particulièrement stable jusqu'à une température de 300°C, donc bien au-delà des conditions de vieillissement accéléré des cellules photovoltaïques en couche mince établie par la norme IEC 61646 qui est de 1000 heures à 60°C/100mW.cm⁻² sous illumination et de 1000 heures à 85°C dans le noir. En comparaison, le DMII admet une perte de masse de l'ordre de 30 % provenant de l'eau captée du fait de son hygroscopicité. Au contraire, même après stockage du composé **3a** à l'air pendant 3 semaines, celui ne présente aucun caractère d'hygroscopicité, ce qui ouvre à son utilisation et son stockage en dehors de boites à gants ou de salles sèches.

### EXEMPLE 2

### Synthèse du iodure de 4-(3-méthoxy-3-oxopropyl)-1,3-diéthyl-1H-imidazol-3-ium (composé 3b)

Dans cet exemple, a été réalisé la synthèse d'un composé de formule (I) dans lequel R¹ est un radical méthyle, les radicaux R² et R⁴ sont identiques et représentent un radical éthyle, R³ représentant un atome d'hydrogène :

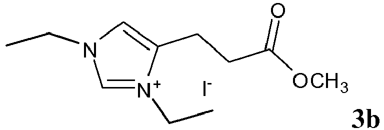

On a ajouté 5 g du composé **2** obtenu ci-dessus à l'étape 2) de l'exemple 1 (32,4 mmol), 9 g de carbonate de potassium (65 mmol) et 7,8 mL d'iodure d'éthyle dans 100 mL d'acétone. Le mélange réactionnel a été maintenu à 70°C sous agitation pendant 1 nuit. Après refroidissement jusqu'à la température ambiante, le mélange réactionnel a été filtré et le solvant a été éliminé sous pression réduite. Le précipité formé a été solubilisé dans le dichlorométhane, puis le mélange a été filtré et le solvant éliminé sous pression réduite. Le mélange réactionnel a ensuite été trituré dans le cyclohexane et le solide a été filtré pour conduire à 9,5 g du composé **3b** attendu sous la forme d'un solide jaune clair (rendement 86 %).

Température de fusion : 111°C
RMN ¹H (CDCl₃, 300 MHz) : δ (ppm) 9,86 (s, 1 H, NCHN) ; 7,41 (s, 1 H, NCH) ; 4,30 (dd, 2 H, *J* = 7,3 Hz, *J* = 14,7 Hz, NCH₂) ; 4,24 (dd, 2 H, *J* = 7,3 Hz, *J* = 14,7 Hz, NCH₂) ; 3,61 (s, 3 H, OCH₃) ; 2,92 (m, 2 H, CH₂) ; 2,72 (t, 2 H, *J* = 6.7 Hz, CH₂) ; 1,52 (m, 6 H, 2 x CH₃)
RMN ¹³C (CDCl₃, 75 MHz) : δ (ppm) 171,8 (CO) ; 135,2 (NCHN) ; 133,8 (Cq) ; 119,2 (NCH) ; 52,1 (OCH₃) ; 45,2 ; 42.6 (2 x NCH₂) ; 31,8 (CH₂) ; 18,9 (CH₂) ; 15,6 ; 15,5 (2 x CH₃)
Masse molaire calculée pour C₁₁H₁₉N₂O₂⁺ : 211,1447 g/mol ; trouvée 211,1446 g/mol.

### EXEMPLE 3

### Synthèse du iodure de 4-(3-méthoxy-3-oxopropyl)-1,3-dipropyl-1H-imidazol-3-ium (composé 3c)

Dans cet exemple, a été réalisé la synthèse d'un composé de formule (I) dans lequel R¹ est un radical méthyle, les radicaux R² et R⁴ sont identiques et représentent un radical propyle, R³ représentant un atome d'hydrogène :

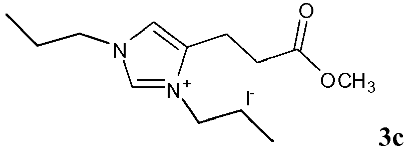

On a ajouté 4,6 g du composé **2** obtenu ci-dessus à l'étape 2) de l'exemple 1 (29,7 mmol), 8,2 g de carbonate de potassium (59,4 mmol) et 8,7 mL de 1-iodopropane dans 100 mL d'acétone. Le mélange réactionnel a été maintenu à 70°C sous agitation pendant 3 jours. Après refroidissement jusqu'à la température ambiante, le mélange réactionnel a été filtré et le solvant a été éliminé sous pression réduite. Après purification sur gel de silice (méthanol/acétate d'éthyle 10/90), 3,8 g du composé **3c** est obtenu sous la forme d'un liquide visqueux orange (rendement 25 %).

RMN ¹H (DMSO-d₆, 300 MHz) : δ (ppm) 9,25 (s, 1 H, NCHN) ; 7,66 (s, 1 H, NCH) ; 4,13 (m, 4 H, 2 x NCH₂) ; 3,63 (s, 3 H, OCH₃) ; 2,93 (m, 2 H, CH₂) ; 2,75 (m, 2 H, CH₂) ; 1,81 (m, 4 H, 2 x CH₂) ; 0,88 (m, 6 H, 2 x CH₃)
RMN ¹³C (DMSO-d₆, 75 MHz) : δ (ppm) 172,3 (CO) ; 136,0 (NCHN) ; 134,2 (Cq) ; 119,6 (NCH) ; 52,1 (OCH₃) ; 50,8 ; 48,2 (2 x NCH₂) ; 31,5 ; 23,1 ; 22,8 ; 18,8 (4 x CH₂) ; 10,9 ; 10,8 (2 x CH₃)
Masse molaire calculée pour C₁₃H₂₃N₂O₂⁺ : 239,1760 g/mol, trouvée 239,1760 g/mol.

### EXEMPLE 4

### Synthèse du iodure de 4-(3-méthoxy-3-oxopropyl)-1,3-dibutyl-1H-imidazol-3-ium (composé 3d)

Dans cet exemple, a été réalisé la synthèse d'un composé de formule (I) dans lequel R¹ est un radical méthyle, les radicaux R² et R⁴ sont identiques et représentent un radical butyle, R³ représentant un atome d'hydrogène :

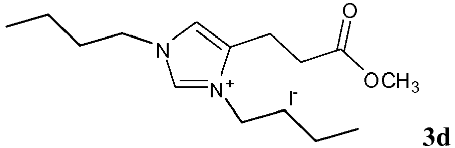

On a ajouté 5 g du composé **2** obtenu ci-dessus à l'étape 2) de l'exemple 1 (32,4 mmol), 8,9 g de carbonate de potassium (65 mmol) et 11,1 mL de 1-iodobutane dans 100 mL d'acétone. Le mélange réactionnel a été maintenu à 70°C sous agitation pendant 3 jours. Après refroidissement jusqu'à la température ambiante, le mélange réactionnel a été filtré et le solvant a été éliminé sous pression réduite. Après purification sur gel de silice (méthanol/acétate d'éthyle 10/90), 5,8 g du composé **3d** est obtenu sous la forme d'un liquide visqueux orange (rendement 45 %).

RMN ¹H (DMSO-d₆, 300 MHz) : δ (ppm) 9,35 (s, 1 H, NCHN) ; 7,71 (s, 1 H, NCH) ; 4,17 (m, 4 H, 2 x NCH2) ; 3,61 (s, 3 H, OCH₃) ; 2,93 (t, 2 H, *J* = 7,2 Hz, CH₂) ; 2,74 (t, 2 H, *J* = 7,2 Hz, CH₂) ; 1,76 (m, 4 H, 2 x CH₂) ; 1,26 (m, 4 H, 2 x CH₂) ; 0,88 (m, 6 H, 2 x CH₃)
RMN ¹³C (DMSO-d₆, 75 MHz) : δ (ppm) 172,3 (CO) ; 135,9 (NCHN) ; 134,0 (Cq) ; 119,7 (NCH) ; 52,2 (OCH₃) ; 49,0 ; 46,6 (2 x NCH₂) ; 31,7 ; 31,6 ; 31,3 ; 19,4 ; 19,2 ; 18,9 (6 x CH₂) ; 13,8, 13.7 (2 x CH₃)
Masse molaire calculée pour C₁₅H₂₇N₂O₂⁺: 267.2073, trouvée 267.2081.

### EXEMPLE 5

### Synthèse du iodure de 4-(3-méthoxy-3-oxopropyl)-1,3-dipentyl-1H-imidazol-3-ium (composé 3e)

Dans cet exemple, a été réalisé la synthèse d'un composé de formule (I) dans lequel R¹ est un radical méthyle, les radicaux R² et R⁴ sont identiques et représentent un radical pentyle, R³ représentant un atome d'hydrogène :

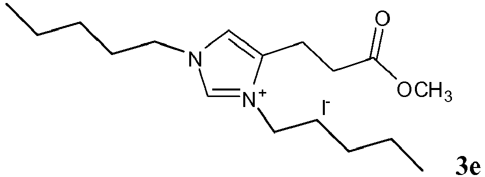

On a ajouté 5 g du composé **2** obtenu ci-dessus à l'étape 2 de l'exemple 1 (32,4 mmol), 8,9 g de carbonate de potassium (65 mmol) et 11,1 mL de 1-iodopentane dans 100 mL d'acétone. Le mélange réactionnel a été maintenu à 70°C sous agitation pendant 3 jours. Après refroidissement jusqu'à la température ambiante, le mélange réactionnel a été filtré et le solvant a été éliminé sous pression réduite. Après purification sur gel de silice (méthanol/acétate d'éthyle 10/90), 10,6 g du composé **3e** est obtenu sous la forme d'un liquide visqueux orange (rendement 79 %).

RMN ¹H (DMSO-d₆, 300 MHz) : δ (ppm) 9,25 (s, 1 H, NCHN) ; 7,66 (s, 1 H, NCH) ; 4,15 (t, 4 H, *J* = 7,4 Hz, 2 x NCH₂) ; 3,63 (s, 3 H, OCH₃) ; 2,93 (t, 2 H, *J* = 7,2 Hz, CH₂) ; 2,76 (t, 2 H, *J* = 7,2 Hz, CH₂) ; 1,79 (m, 4 H, 2 x CH₂) ; 1,29 (m, 8 H, 4 x CH₂) ; 0,88 (m, 6 H, 2 x CH₃)
RMN ¹³C (DMSO-d₆, 75 MHz) : δ (ppm) 172,3 (CO) ; 136,0 (NCHN) ; 134,1 (Cq) ; 119,6 (NCH) ; 52,1 (OCH₃) ; 49,3 ; 46,7 (2 x NCH₂) ; 31,5 ; 29,3 ; 29,0 ; 28,2 ; 28,0 ; 22,0 ; 21,9, 18,8 (8 x CH₂) ; 14, (2 x CH₃)
Masse molaire calculée pour C₁₇H₃₁N₂O₂⁺ : 295,2386 g/mol, trouvée 295,2382 g/mol.

### EXEMPLE 6

### Synthèse du iodure de 4-(3-méthoxy-3-oxopropyl)-1,3-dihexyl-1H-imidazol-3-ium (composé 3f)

Dans cet exemple, a été réalisé la synthèse d'un composé de formule (I) dans lequel R¹ est un radical méthyle, les radicaux R² et R⁴ sont identiques et représentent un radical hexyle, R³ représentant un atome d'hydrogène :

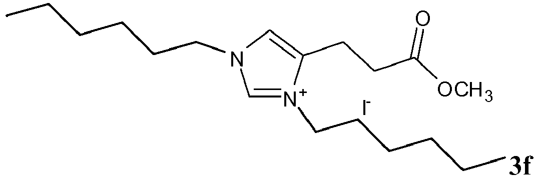

On a ajouté 5 g du composé **2** obtenu ci-dessus à l'étape 2) de l'exemple 1 (32,4 mmol), 8,9 g de carbonate de potassium (65 mmol) et 14,3 mL de 1-iodohexane dans 100 mL d'acétone. Le mélange réactionnel a été maintenu à 70°C sous agitation pendant 3 jours. Après refroidissement jusqu'à la température ambiante, le mélange réactionnel a été filtré et le solvant a été éliminé sous pression réduite. Après purification sur gel de silice (méthanol/acétate d'éthyle 10/90), 11,6 g du composé **3f** est obtenu sous la forme d'un liquide visqueux orange (rendement 79 %).

RMN ¹H (DMSO-d₆, 300 MHz) : δ (ppm) 9,31 (s, 1 H, NCHN) ; 7,69 (s, 1 H, NCH) ; 4,16 (t, 4 H, *J* = 7,2 Hz, 2 x NCH₂) ; 3,62 (s, 3 H, OCH₃) ; 2,93 (t, 2 H, *J* = 7,2 Hz, CH₂) ; 2,76 (t, 2 H, *J* = 7,2 Hz, CH₂) ; 1,78 (m, 4 H, 2 x CH₂) ; 1,29 (m, 12 H, 6 x CH₂) ; 0,85 (m, 6 H, 2 x CH₃)
RMN ¹³C (DMSO-d₆, 75 MHz) : δ (ppm) 172,3 (CO) ; 136,0 (NCHN), 134,1 (Cq) ; 119,6 (NCH) ; 52,1 (OCH₃) ; 49,3 ; 46,8 (2 x NCH₂) ; 31,5 ; 31,0 ; 30,9 ; 29,6 ; 29,3 ; 25,7 ; 25,5 ; 22,3 ; 18,9 (10 x CH₂) ; 14,2 (2 x CH₃)
Masse molaire calculée pour C₁₉H₃₅N₂O₂⁺ : 323,2699 g/mol, trouvée 323,2691 g/mol.

### EXEMPLE 7

### Synthèse du iodure de 4-(3-éthoxy-3-oxopropyl)-1,3-diméthyl-1H-imidazol-3-ium (composé 6)

Dans cet exemple, a été réalisé la synthèse d'un composé de formule (I) dans lequel les radicaux R² et R⁴ sont identiques et représentent un radical méthyle, R¹ représente un radical éthyle, R³ représentant un atome d'hydrogène :

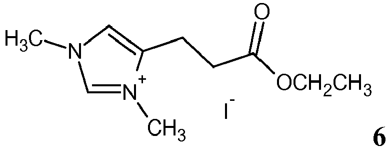

### 1) Première étape : Synthèse de l'ester éthylique de l'acide urocanique (composé 4)

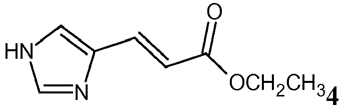

On a ajouté 14,3 mL d'acide sulfurique concentré à une solution d'acide urocanique (25 g, 181 mmol) et de sulfate de sodium (3,5 g) dans l'éthanol anhydre (165 mL). Après agitation pendant une nuit à 70°C, le mélange réactionnel a été refroidi à température ambiante et filtré. Le solvant a ensuite été éliminé sous pression réduite et de l'eau a été ajoutée (1 mL). La solution ainsi obtenue a été neutralisée jusqu'à pH neutre par ajout d'une solution saturée de carbonate de sodium. La solution a ensuite été extraite 4 fois à l'acétate d'éthyle, puis les phases organiques ont été réunies et séchées sur sulfate de sodium. Le solvant a ensuite été éliminé sous pression réduite. Après solubilisation du précipité avec une quantité minimale d'acétate d'éthyle, 300 mL de cyclohexane ont été ajoutés. Après filtration, le composé **4** attendu a été obtenu sous la forme d'un solide blanc (29 g, rendement 96 %).

Température de fusion : 81-82°C
RMN ¹H (DMSO-d₆, 400 MHz) : *δ* (ppm) 7,78 (s, 1 H, NCHN) ; 7,54 (s, 1 H, N=CH) ; 7,53 (d, 1 H, J_{a,b} = 15,6 Hz, Hₐ CH=) ; 6,33 (d, 1 H, J_{a,b} = 15,6 Hz, H_{b} CH=); 4,15 (q, 2 H, J_{CH2,CH3} = 7,1 Hz, CH₂) ; 1,23 (t, 3 H, J_{CH2,CH3} = 7,1 Hz, CH₃)
RMN ¹³C (DMSO-d₆, 100 MHz) : *δ* (ppm) 167,0 (CO) ; 138,2 (NCH=, CH=), 114,0 (CH=, NCH=) ; 60,0 (CH₂) ; 14,7 (CH₃)

Masse molaire calculée pour C₈H₁₂N₂O₂Na : 191,0796 g/mol, trouvée 191,0800 g/mol.

### 2) Deuxième étape : Synthèse du éthyl 3-(1H-imidazol-4-yl)propanoate (composé 5)

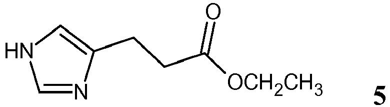

A une solution de 25 g (150 mmol) de l'ester éthylique de l'acide urocanique (composé **4** obtenu à l'étape 1) précédente) dans 150 mL de méthanol, on a ajouté 1,8 g de Pd/C à 10 % sous pression de dihydrogène. Après hydrogénation pendant 14 heures à température ambiante, le milieu réactionnel a été filtré sur célite. Le filtrat a ensuite été évaporé sous pression réduite pour conduire à 23 g du composé **5** attendu (rendement 91 %) sous la forme d'un sirop légèrement orange.

RMN ¹H (DMSO-d₆, 400 MHz) : *δ* (ppm) 7,55 (s, 1 H, NCHN) ; 6,78 (s, 1 H, N=CH) ; 4,04 (q, 2 H, J_{CH2,CH3} = 7,2 Hz, C*H*₂CH₃) ; 2,78 (t, 2 H, J_{CH2,CH2} = 7,8 Hz, *C*H₂CH₂) ; 2,59 (t, 2 H, J_{CH2,CH2} = 7,8 Hz, CH₂) ; 1,15 (t, 3 H, J_{CH2,CH3} = 7,2 Hz, CH₂C*H*₃)
RMN ¹³C (DMSO-d₆, 100 MHz) : *δ* (ppm) 172,7 (CO) ; 136,1 (Cq) ; 135,0 (CH=) ; 116,5 (CH=) ; 60,2 (*C*H₂CH₃) ; 33,9 (CH₂) ; 22,6 (CH₂) ; 14,7 (CH₂*C*H₃)
Masse molaire calculée pour C₈H₁₀N₂O₂Na : 189,0640 g/mol, trouvée 189,0638 g/mol.

### 3) Troisième étape : Synthèse de l'iodure de 4-(3-éthoxy-3-oxopropyl)-1,3-diiméthyl-1H-imidazol-3-ium (composé 6)

On a ajouté 5 g du composé **5** obtenu ci-dessus à l'étape précédente (29,7 mmol), 8,3 g de carbonate de potassium (149 mmol) et 9,2 mL d'iodure de méthyle dans 100 mL d'acétone. Le mélange réactionnel a été maintenu à 70°C sous agitation pendant 1 nuit. Après refroidissement jusqu'à la température ambiante, le mélange réactionnel a été filtré et le solvant a été éliminé sous pression réduite. Le précipité formé a été solubilisé dans le dichlorométhane, puis le mélange a été filtré et le solvant éliminé sous pression réduite. Le mélange réactionnel a ensuite été trituré dans le cyclohexane et le solide a été filtré pour conduire à 6,9 g du composé **6** attendu sous la forme d'un solide blanc (rendement 57 %).

RMN ¹H (DMSO-d₆, 400 MHz) : *δ* (ppm) 7,55 (s, 1 H, NCHN) ; 6,78 (s, 1 H, N=CH) ; 4,04 (q, 2 H, J_{CH2,CH3} = 7,2 Hz, C*H*₂CH₃) ; 2,78 (t, 2 H, J_{CH2,CH2} = 7,8 Hz, *C*H₂CH₂) ; 2,59 (t, 2 H, J_{CH2,CH2} = 7,8 Hz, CH₂) ; 1,15 (t, 3 H, J_{CH2,CH3} = 7,2 Hz, CH₂C*H*₃)
RMN ¹³C (DMSO-d₆, 100 MHz) : *δ* (ppm) 171,9 (CO) ; 136,9 (CH=) ; 134,4 (Cq) ; 120,6 (CH=) ; 60,7 (CH₂CH₃) ; 36,2 (NCH₃) ; 33,7 (NCH₃) ; 31,6 (CH₂) ; 18,6 (CH₂) ; 14,5 (CH₂*C*H₃)
Masse molaire calculée pour C₁₀H₁₇N₂O₂Na : 197,1290 g/mol, trouvée 197,1289 g/mol.

### EXEMPLE 8

### Synthèse du iodure de 4-(3-méthoxy-3-oxopropyl)-1-méthyl-3-propyl-1H-imidazol-3-ium (composé 8)

Dans cet exemple, a été réalisée la synthèse d'un composé de formule (I) dans lequel les radicaux R¹ et R² sont identiques et représentent un radical méthyle, R³ représente un atome d'hydrogène et R⁴ représente un radical propyle :

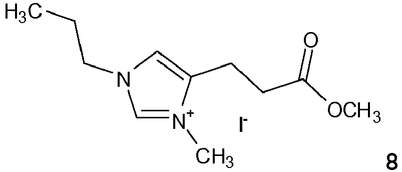

### 1) Première étape : Synthèse du méthyl 3-(-propyl-1H-imidazol-4-yl)propanoate (composé 7)

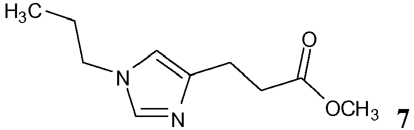

A une solution de 4,9 g (31,9 mmol) du composé **2** obtenu ci-dessus à l'étape 2) de l'exemple 1, de 5,3 g (31,9 mmol) de iodure de potassium, et de 8,8 g (63,9 mmol) de carbonate de potassium dans 100 mL d'acétone, on a ajouté 3,9 g (31,9 mmol) de 1-bromopropane. Le mélange réactionnel a été maintenu sous agitation à 70°C pendant une nuit puis filtré. Le solvant a été éliminé sous pression réduite. Après chromatographie flash sur gel de silice (éluant acétate d'éthyle 100 % puis mélange méthanol/acétate d'éthyle 5/95 v/v) on a obtenu 1,4 g du compose **7** attendu (rendement 23 %) sous la forme d'un sirop incolore.

RMN ¹H (CDCl₃, 300 MHz) : δ (ppm) 9,91 (s, 1 H, NCHN) ; 7,40 (s, 1 H, NCH) ; 4,22 (m, 2 H, NCH₂) ; 3,64 (s, 3 H, OCH₃) ; 2,94 (t, 2 H, *J* = 7,2 Hz, CH₂) ; 2,76 (t, 2 H, *J* = 6,9 Hz, CH₂) ; 1,94 (m, 2 H, C*H*₂CH₃) ; 0,95 (m, 3 H, C*H*₃CH₂)
RMN ¹³C (CDCl₃, 75 MHz) : δ (ppm) 171,7 (CO) ; 135,9 (NCHN) ; 133,9 (Cq) ; 119,4 (NCH) ; 52,2 (OCH₃) ; 48,7 (NCH₂) ; 31,8 (CH₂) ; 23,5 (C*H*₂CH₃) ; 19,0 (CH₂) ; 10,8 (C*H*₃CH₂).

Masse moléculaire calculée pour C₁₀H₁₆N₂O₂Na⁺ : 219 g/mol, trouvée 219 g/mol.

### 2) Deuxième étape : Synthèse du iodure de 4-(3-méthoxy-3-oxopropyl)-1-méthyl-3-propyl-1H-imidazol-3-ium (composé 8)

Une solution de 1,4 g (7,4 mmol) du composé **7** obtenu ci-dessus à l'étape précédente, de 2,0 g (14,9 mmol) de carbonate de potassium et de 1,38 mL (22,3 mmol) d'iodure de potassium dans 100 mL d'acétone a été maintenue sous agitation à 70°C pendant 2 heures. Après refroidissement à température ambiante, le mélange réactionnel a été filtré et le solvant a été éliminé sous pression réduite.

Après chromatographie flash sur gel de silice (éluant acétate d'éthyle 100 % puis mélange méthanol/acétate d'éthyle 5/95 v/v), on a obtenu 931 mg du composé **8** attendu sous la forme d'un sirop légèrement orangé (rendement 37 %).

RMN ¹H (CD₃OD, 300 MHz) : δ (ppm) 8,95 (s, 1 H, NCHN) ; 7,50 (s, 1 H, NCH) ; 4,18 (t, 2 H, *J* = 7,2 Hz, NCH₂) ; 3,90 (s, 3 H, NCH₃) ; 3,73 (s, 3 H, OCH₃) ; 3,04 (t, 2 H, *J* = 7,0 Hz, CH₂) ; 2,83 (t, 2 H, *J* = 7,0 Hz, CH₂) ; 1,95 (q, 2 H, *J =* 3,9 Hz ; CH₂CH₃) ; 1.00 (m, 3 H, *J* = 7,5 Hz, CH₃CH₂)
RMN ¹³C (CD₃OD, 75 MHz) : δ (ppm) 172,4 (CO) ; 135,9 (NCHN) ; 135,1 (Cq) ; 119,2 (NCH) ; 51,1 (OCH₃) ; 50,9 (NCH₂) ; 32,9 (NCH₃) ; 31,0 (CH₂) ; 23.0 (C*H*₂CH₃) ; 18,4 (CH₂) ; 9,6 (C*H*₃CH₂).

Masse molaire calculée pour C₁₁H₁₉N₂O₂⁺ : 211 g/mol, Trouvée 211 g/mol.

### EXEMPLE 9

### Préparation de cellules de conversion photovoltaïque à colorant et études de leurs propriétés

Le composé **3a** tel que préparé ci-dessus a ensuite été utilisé pour la préparation d'une cellule photovoltaïque à colorant conforme à l'invention (**CP1**). A titre comparatif, une cellule photovoltaïque ne faisant pas partie de l'invention a également été préparée en utilisant le DMII à titre de sel d'électrolyte (**CP2**).

Le protocole général de préparation des cellules **CP1** et **CP2** a été le suivant :

### Compositions d'électrolytes :

On a préparé la composition d'électrolyte **E1** conforme à l'invention suivante :
- Composé **3a** : 1 mol/L ;
- Diiode : 0,15 mol/L ;
- N-butylbenzimidazole : 0,5 mol/L ;
- Thiocyanate de guanidinium : 0,1 mol/L ;
- 3-méthoxypropionitrile : QS

A titre comparatif, on a préparé une composition d'électrolyte **E2** ne faisant pas partie de l'invention, de composition suivante :
- DMII : 1 mol/L ;
- Diiode : 0,15 mol/L ;
- N-butylbenzimidazole : 0,5 mol/L ;
- Thiocyanate de guanidinium : 0,1 mol/L ;
- 3-méthoxypropionitrile : QS

### Préparation de la photoanode :

Une couche de nanoparticules dioxyde de titane (diamètre 20 nm) de 8 µm a été appliquée par sérigraphie (« *Screen-Printing* ») sur une plaque de verre (20 x 14 mm) préalablement couverte d'une couche de FTO de 300 nm.

Une couche du complexe RuLL'(NCS)₂ ayant une épaisseur d'environ 2 nm a ensuite été déposée sur la couche de FTO par trempage (« *dip coating* »)*.*

### Contre-électrode

Une couche de nanoparticules de platine a été déposée sur la contre-électrode soit par pulvérisation cathodique à partir d'une cible de Pt ou alors par évaporation d'une solution d'un précurseur de platine « *drop casting* » en effectuant la décomposition thermique à 450°C sous air d'une ou de plusieurs gouttes d'une solution de 40 mmol/L de H₂PtCl₆ dans l'éthanol ou alternativement dans l'isopropanol déposée(s) sur la surface de l'électrode.

### Assemblage des cellules photovoltaïques

Des cellules photovoltaïques **CP1** conformes à l'invention et **CP2** ne faisant pas partie de l'invention ont ensuite été assemblées en utilisant respectivement les compositions d'électrolyte **E1** et **E2**, de la façon suivante :
Sur la contre-électrode a été effectué un trou conique de diamètre inférieur à 200 µm par micro-sablage pour permettre l'injection de l'électrolyte dans la cellule. La photo-électrode et la contre-électrode ont été assemblées en utilisant un joint polymère thermo-fusible du type Surlyn® ou Bynel® produit et vendu par la société Dupont. La cellule a été scellée à une température de 130°C pendant environ 5 secondes. L'électrolyte a été injecté par capillarité sous vide secondaire. La partie externe du microcone a enfin été obstruée par une lame de verre thermo scellée avec un polymère de type Surlyn® ou Bynel® à l'aide d'une panne de fer à souder. La soudure a été réalisée sous ultra-son à partir d'un eutectique d'indium et de gallium pour obtenir un excellent contact électrique.

Ces différentes cellules ont ensuite été testées en conversion photovoltaïque sous un éclairement AM 1,5 (« *Air Mass* » 1,5 Global) d'un simulateur de rayonnement solaire de classe 3A (Newport) équipé d'une lampe Xenon de 450 W. Les mesures ont été effectuées avec une station Oriel PVIV 3A couplée à un sourcemètre Keithley 2420.

La surface standard des électrodes était comprise entre 0,16 cm² et 0,36 cm² et la puissance surfacique incidente était de 100 mW/cm².

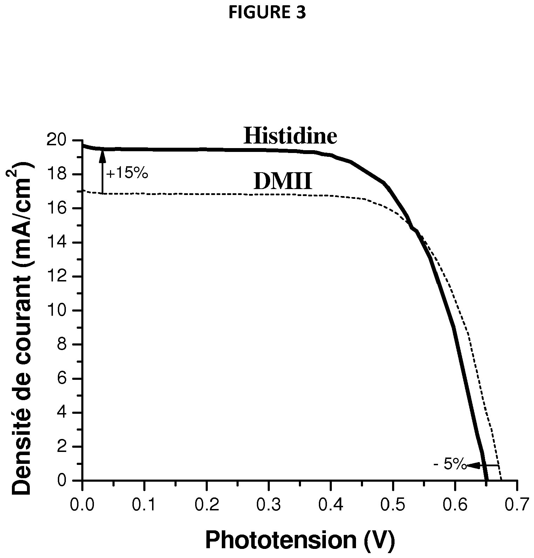

La figure 3 annexée compare la densité du courant (en mA/cm²) en fonction de la phototension (en V) pour chacune des cellules **CP1** et **CP2.** Sur cette figure la courbe en trait pointillé correspond à une cellule **CP2** ne faisant pas partie de l'invention et la courbe en trait continu correspond à une cellule **CP1** conforme à l'invention

Ces résultats montrent qu'en plus d'être *a priori* plus stable chimiquement et vis-à-vis de l'interface Pt, la substitution du DMII par le composé **3a** permet d'améliorer les rendements de conversion lumière - électricité, ceux-ci passant en effet de 8,0 % à 8,5 %. La principale amélioration grâce à l'emploi du composé **3a** provient de la génération de plus de photocourant, celui-ci passant de 16,9 mA/cm² à 19,5 mA/cm² (Fig. 3). Par contre, la photo-tension passe de 680 mV à 647 mV et le facteur de remplissage de 69,3 % à 67 %. Cette diminution est largement compensée par le gain en courant qui atteint plus de 15 %.

La stabilité des cellules **CP1** et **CP2** a également été étudiée en conditions de vieillissement accéléré à 70°C, avec une puissance surfacique incidente de lumière à 100 mW/cm² et en absence de filtres UV

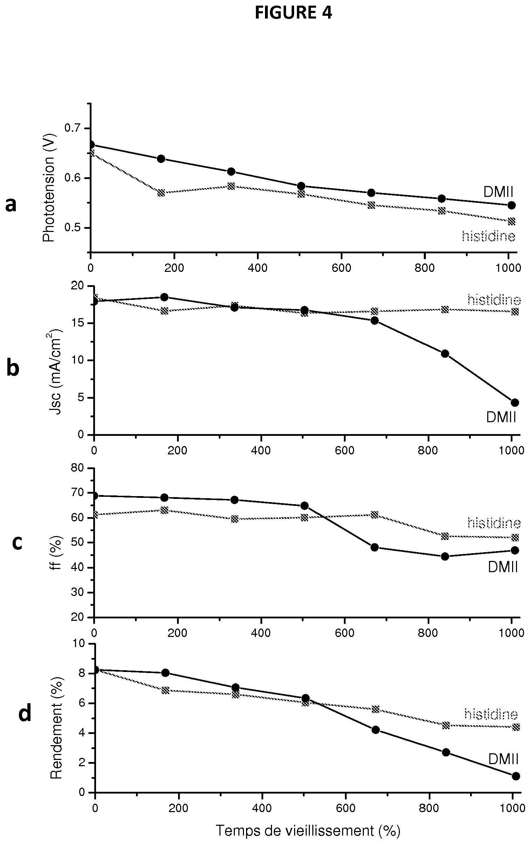

Les résultats obtenus sont reportés sur la figure 4 annexée. En particulier, la figure 4a donne les résultats de l'évolution de la phototension (en V) en fonction du temps de vieillissement (en heure), la figure 4b donne les résultats de l'évolution du photocourant (en mA/cm²) en fonction du temps de vieillissement (en heure), la figure 4c donne les résultats de l'évolution du facteur de remplissage (FF, en %) en fonction du temps de vieillissement (en heure) et la figure 4d donne les résultats de l'évolution du rendement de conversion lumière/électricité (en %) en fonction du temps de vieillissement (en heure). Sur ces figures, les courbes avec les cercles pleins correspondent à une cellule **CP2** ne faisant pas partie de l'invention et les courbes avec les carrés pleins correspondent à une cellule **CP1** conforme à l'invention.

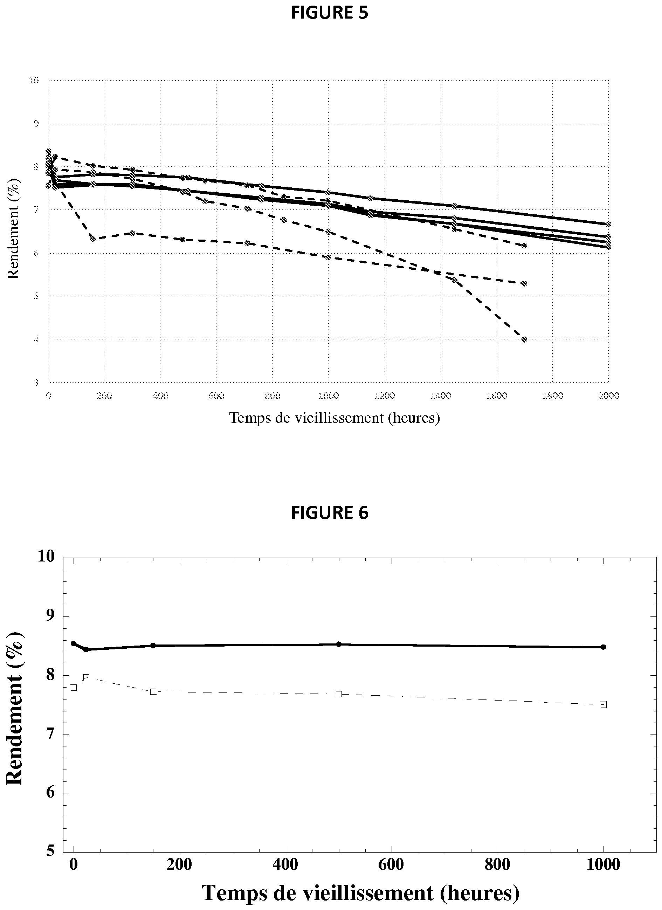

Ces résultats montrent que le rendement de conversion de la cellule **CP2** ne faisant pas partie de l'invention baisse assez sensiblement, notamment après 500 heures de vieillissement. Cette chute de rendement est principalement liée à une perte importante du photo-courant et du facteur de remplissage. Par contre, dans le cas de la cellule **CP1** conforme à l'invention, l'utilisation du composé **3a** à titre de sel d'électrolyte dans la composition d'électrolyte permet de maintenir le photo-courant à des valeurs élevées et très stables. Elle permet également de mieux maintenir le facteur de remplissage dans les conditions de vieillissement accéléré. Ainsi on observe que l'emploi du composé **3a** permet de maintenir un rendement de conversion satisfaisant avec une perte de rendement de seulement 25 % dans le cas du composé **3a** contre 85 % dans le cas du DMII après une durée de vieillissement de 1000 heures. La figure 5 donne les résultats de la comparaison entre une cellule CP1 et une cellule CP2 dans les conditions standards du protocole IEC 61646, c'est-à-dire à 60°C sous 100 mW.cm⁻² (filtre UV à 340 nm). Sur cette figure, le rendement de conversion lumière/électricité (en %) est fonction du temps de vieillissement (en heure) ; les courbes en trait plein correspondent aux résultats de cellules **CP1** conformes à l'invention et les courbes en trait discontinu correspondent aux résultats de cellules **CP2** ne faisant pas partie de l'invention. Les résultats donnés par la figure 5 montrent également la supériorité du composé **3a** tant au niveau des rendements que de la stabilité sur 2000 heures de vieillissement. Les quatre cellules assemblées avec **E1** (cellules **CP1**) possèdent une rétention de rendement de l'ordre de 82 à 85 %, soit supérieure aux 80 % requis par le protocole standard IEC 61646 et bien au-delà des 1000 heures de vieillissement accéléré préconisé par le protocole. Au contraire, les quatre cellules assemblées avec **E2** (cellules **CP2** non conformes à l'invention) montrent un déclin du rendement plus prononcé au cours du vieillissement. Une des quatre cellules avait un rendement inférieur à 2 % après 600 heures (non rapportée) et les trois autres montrent une rétention de 77 %, 66 % et 53 % après 1700 heures de vieillissement, soit des valeurs bien inférieures à celles observées avec les cellules **CP1**, et inférieures à la barre des 80 % du protocole IEC 61646.

Enfin, la figure 6 rapporte les résultats obtenus avec les cellules **CP1** et **CP2** lors d'un essai de vieillissement à très basse température (-40°C dans le noir). Sur cette figure, le rendement de conversion lumière/électricité (en %) est fonction du temps de vieillissement (en heure) ; la courbe en trait plein correspondent aux résultats de cellules **CP1** conformes à l'invention et les courbes en trait discontinu correspondent aux résultats de cellules **CP2** ne faisant pas partie de l'invention. Ces résultats montrent que **E1** est également très performant dans de telles conditions d'utilisation. L'utilisation de cet électrolyte montre une rétention après 1000 heures de 99,2 % contre 96,2 % pour **E2**. Les rendements après 1000 heures à -40°C sont de 8,48 % pour E1 et 7,51 % pour **E2**.

### EXEMPLE 10

### Préparation de cellules de conversion photovoltaïque à colorant et études de leurs propriétés avec des composés de formule (I) conformes à l'invention de la série 3 avec R₁ = CH₃, R₃ = H et R₂=R₄=CₙH₂ₙ₊₁ (1 ≤ n ≤ 6) (soit les composés 3a à 3f) et R₁ = C₂H₅, R₃ = H et R₂=R₄=CH₃ (composé 6)

Des cellules photovoltaïque à colorant ont été préparées en utilisant les composés de la série **3** (composés **3a** à **3f**) tels que préparés dans les exemples 1 à 6 ci-dessus, ainsi que le composé **6** tel que préparé à l'exemple 7 en utilisant par ailleurs, pour la composition de l'électrolyte, les mêmes ingrédients que dans l'électrolyte **E1** tel que préparé dans l'exemple 9. On a ensuite réalisé la préparation de la photo-anode, de la contre-électrode et l'assemblage de cellule de la même façon que dans l'exemple 9 ci-dessus.

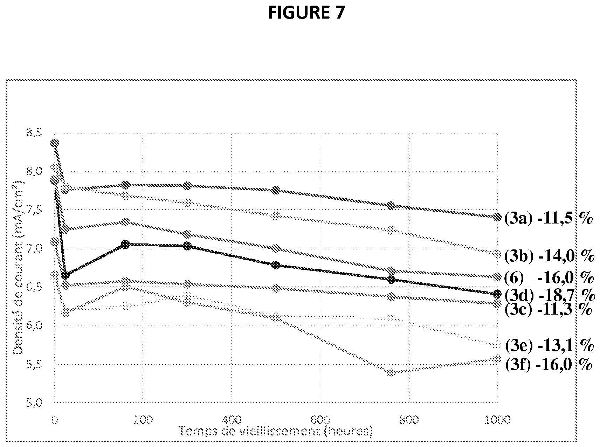

Les cellules ainsi préparées ont été testées en conditions de vieillissement accéléré à 60°C sous 100 mW.cm⁻² de lumière incidente filtrée contre les UV de longueur d'onde inférieure à 340 nm. Les résultats obtenus sont donnés par la figure 7 annexée sur laquelle la densité de courant (en mA/cm²) est fonction du temps de vieillissement (en heure). Ces résultats montrent qu'*a priori* les composés de la série **3** sont toujours très stables dans ces conditions de vieillissement puisque, après 1000 heures de vieillissement, la rétention du rendement est comprise entre 81 % pour la chaine hexyle (composé **3d**) et 89 % pour la chaine méthyle (composé **3a**) et propyle (composé **3c**). Les résultats semblent d'ailleurs indiquer que la chaine propyle est la plus stable. La série montre que plus chaine alkyle est longue, plus ce dernier est soluble dans le milieu considéré. Par contre, les résultats semblent indiquer que le rendement de conversion varie en fonction de la longueur de la chaine. L'allongement de la chaine ester diminue également un peu le rendement de conversion mais n'affecte pas la stabilité qui reste remarquable, bien meilleure que celle de l'état de l'art (**CP2**). Les composés de la série **3** montrent également une toute aussi bonne stabilité à basse température de -40°C, analogue aux résultats obtenus dans l'exemple précédent.

## Revendications

1. Utilisation d'au moins un halogénure de formule (I) suivante : 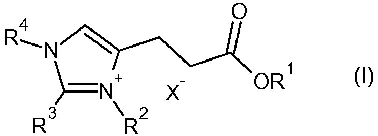 dans laquelle :
- R¹ représente un atome d'hydrogène ou un radical alkyle linéaire ayant de 1 à 10 atomes de carbone ;
- R² et R⁴, identiques ou différents, représentent chacun un radical alkyle linéaire ou ramifié ayant de 1 à 15 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote ;
- R³ représente un atome d'hydrogène ou un radical alkyle linéaire ou ramifié ayant de 1 à 15 atomes de carbone,
- X⁻ représente un anion iodure ou bromure,
à titre de sel d'électrolyte dans une composition d'électrolyte d'une cellule photoélectrochimique basée sur la sensibilisation à la lumière de molécules photo-actives.

2. Utilisation selon la revendication 1, **caractérisée en ce que** la cellule photoélectrochimique basée sur la sensibilisation à la lumière de molécules photo-actives est une cellule photovoltaïque à colorants.

3. Utilisation selon la revendication 1 ou 2, **caractérisée en ce que** les composés de formule (I) sont choisis parmi :
- le iodure de 4-(3-méthoxy-3-oxopropyl)-1,3-diméthyl-1*H*-imidazol-3-ium,
- le iodure de 4-(3-éthoxy-3-oxopropyl)-1,3-diméthyl-1*H*-imidazol-3-ium,
- le iodure de 4-(3-méthoxy-3-oxopropyl)-1,3-diéthyl-1*H*-imidazol-3-ium,
- le iodure de 4-(3-méthoxy-3-oxopropyl)-1,3-dipropyl-1*H*-imidazol-3-ium,
- le iodure de 4-(3-méthoxy-3-oxopropyl)-1,3-dibutyl-1*H*-imidazol-3-ium,
- le iodure de 4-(3-méthoxy-3-oxopropyl)-1,3-dipentyl-1*H*-imidazol-3-ium,
- le iodure de 4-(3-méthoxy-3-oxopropyl)-1,3-dihexyl-1*H*-imidazol-3-ium,
- le iodure de 4-(-3-méthoxy-3-oxopropyl)-1-méthyl-3-propyl-1*H*-imidazol-3-ium,
- le iodure de 3-éthyl-4-(3-méthoxy-3-oxopropyl)-1-méthyl-1*H*-imidazol-3-ium,
- le iodure de 4-(3-méthoxy-3-oxopropyl)-1-méthyl-3-propyl-1*H*-imidazol-3-ium,
- le iodure de 3-butyl-4-(3-méthoxy-3-oxopropyl)-1-méthyl-1*H*-imidazol-3-ium,
- le iodure de 4-(3-méthoxy-3-oxopropyl)-1-méthyl-3-pentyl-1*H*-imidazol-3-ium,
- le iodure de 3-hexyl-4-(3-méthoxy-3-oxopropyl)-1-méthyl-1*H*-imidazol-3-ium,
- le iodure de 1-éthyl-4-(3-méthoxy-3-oxopropyl)-3-méthyl-1*H*-imidazol-3-ium,
- le iodure de 1,3-diéthyl-4-(-3-méthoxy-3-oxopropyl)-1*H*-imidazol-3-ium,
- le iodure de 1-éthyl-4-(3-méthoxy-3-oxopropyl)-3-propyl-1*H*-imidazol-3-ium,
- le iodure de 3-butyl-1-éthyl-4-(3-méthoxy-3-oxopropyl)-1*H*-imidazol-3-ium,
- le iodure de 1-éthyl-4-(3-méthoxy-3-oxopropyl)-3-pentyl-1*H*-imidazol-3-ium,
- le iodure de 1-éthyl-3-hexyl-4-(3-méthoxy-3-oxopropyl)-1*H*-imidazol-3-ium,
- le iodure de 4-(3-méthoxy-3-oxopropyl)-3-méthyl-1-propyl-1*H*-imidazol-3-ium,
- le iodure de 3-éthyl-4-(3-méthoxy-3-oxopropyl)-1-propyl-1*H*-imidazol-3-ium,
- le iodure de 4-(3-méthoxy-3-oxopropyl)-1,3-dipropyl-1*H*-imidazol-3-ium,
- le iodure de 3-butyl-4-(-3-méthoxy-3-oxopropyl)-1-propyl-1*H*-imidazol-3-ium,
- le iodure de 4-(3-méthoxy-3-oxopropyl)-3-pentyl-1-propyl-1*H*-imidazol-3-ium,
- le iodure de 3-hexyl-4-(3-méthoxy-3-oxopropyl)-1-propyl-1*H*-imidazol-3-ium,
- le iodure de 1-butyl-4-(3-méthoxy-3-oxopropyl)-3-méthyl-1*H*-imidazol-3-ium,
- le iodure de 1-butyl-3-éthyl-4-(3-méthoxy-3-oxopropyl)-1*H*-imidazol-3-ium,
- le iodure de 1-butyl-4-(3-méthoxy-3-oxopropyl)-3-propyl-1*H*-imidazol-3-ium,
- le iodure de 1,3-dibutyl-4-(3-méthoxy-3-oxopropyl)-1*H*-imidazol-3-ium,
- le iodure de 1-butyl-4-(3-méthoxy-3-oxopropyl)-3-pentyl-1*H*-imidazol-3-ium,
- le iodure de 1-butyl-3-hexyl-4-(3-méthoxy-3-oxopropyl)-1*H*-imidazol-3-ium,
- le iodure de 4-(3-méthoxy-3-oxopropyl)-3-méthyl-1-pentyl-1*H*-imidazol-3-ium,
- le iodure de 3-éthyl-4-(3-méthoxy-3-oxopropyl)-1-pentyl-1*H*-imidazol-3-ium,
- le iodure de 4-(3-méthoxy-3-oxopropyl)-1-pentyl-3-propyl-1*H*-imidazol-3-ium,
- le iodure de 3-butyl-4-(3-méthoxy-3-oxopropyl)-1-pentyl-1*H*-imidazol-3-ium,
- le iodure de 4-(3-méthoxy-3-oxopropyl)-1,3-dipentyl-1*H*-imidazol-3-ium,
- le iodure de 3-hexyl-4-(3-méthoxy-3-oxopropyl)-1-pentyl-1*H*-imidazol-3-ium,
- le iodure de 1-hexyl-4-(3-méthoxy-3-oxopropyl)-3-méthyl-1*H*-imidazol-3-ium,
- le iodure de 3-éthyl-1-hexyl-4-(3-méthoxy-3-oxopropyl)-1*H*-imidazol-3-ium,
- le iodure de 1-hexyl-4-(3-méthoxy-3-oxopropyl)-3-propyl-1*H*-imidazol-3-ium,
- le iodure de 3-butyl-1-hexyl-4-(3-méthoxy-3-oxopropyl)-1*H*-imidazol-3-ium,
- le iodure de 1-hexyl-4-(3-méthoxy-3-oxopropyl)-3-pentyl-1*H*-imidazol-3-ium,
- le iodure de 1,3-dihexyl-4-(3-méthoxy-3-oxopropyl)-1*H*-imidazol-3-ium,
- le bromure de 3-éthyl-4-(3-méthoxy-3-oxopropyl)-1-méthyl-1*H*-imidazol-3-ium,
- le bromure de 4-(3-méthoxy-3-oxopropyl)-1-méthyl-3-propyl-1*H*-imidazol-3-ium,
- le bromure de 3-butyl-4-(3-méthoxy-3-oxopropyl)-1-méthyl-1*H*-imidazol-3-ium,
- le bromure de 4-(3-méthoxy-3-oxopropyl)-1-méthyl-3-pentyl-1*H*-imidazol-3-ium,
- le bromure de 3-hexyl-4-(3-méthoxy-3-oxopropyl)-1-méthyl-1*H*-imidazol-3-ium,
- le bromure de 1-éthyl-4-(3-méthoxy-3-oxopropyl)-3-méthyl-1*H*-imidazol-3-ium,
- le bromure de 1,3-diéthyl-4-(-3-méthoxy-3-oxopropyl)-1*H*-imidazol-3-ium,
- le bromure de 1-éthyl-4-(3-méthoxy-3-oxopropyl)-3-propyl-1*H*-imidazol-3-ium,
- le bromure de 3-butyl-1-éthyl-4-(3-méthoxy-3-oxopropyl)-1*H*-imidazol-3-ium,
- le bromure de 1-éthyl-4-(3-méthoxy-3-oxopropyl)-3-pentyl-1*H*-imidazol-3-ium,
- le bromure de 1-éthyl-3-hexyl-4-(3-méthoxy-3-oxopropyl)-1*H*-imidazol-3-ium,
- le bromure de 4-(3-méthoxy-3-oxopropyl)-3-méthyl-1-propyl-1*H*-imidazol-3-ium,
- le bromure de 3-éthyl-4-(3-méthoxy-3-oxopropyl)-1-propyl-1*H*-imidazol-3-ium,
- le bromure de 4-(3-méthoxy-3-oxopropyl)-1,3-dipropyl-1*H*-imidazol-3-ium,
- le bromure de 3-butyl-4-(-3-méthoxy-3-oxopropyl)-1-propyl-1*H*-imidazol-3-ium,
- le bromure de 4-(3-méthoxy-3-oxopropyl)-3-pentyl-1-propyl-1*H*-imidazol-3-ium,
- le bromure de 3-hexyl-4-(3-méthoxy-3-oxopropyl)-1-propyl-1*H*-imidazol-3-ium,
- le bromure de 1-butyl-4-(3-méthoxy-3-oxopropyl)-3-méthyl-1*H*-imidazol-3-ium,
- le bromure de 1-butyl-3-éthyl-4-(3-méthoxy-3-oxopropyl)-1*H*-imidazol-3-ium,
- le bromure de 1-butyl-4-(3-méthoxy-3-oxopropyl)-3-propyl-1*H*-imidazol-3-ium,
- le bromure de 1,3-dibutyl-4-(3-méthoxy-3-oxopropyl)-1*H*-imidazol-3-ium,
- le bromure de 1-butyl-4-(3-méthoxy-3-oxopropyl)-3-pentyl-1*H*-imidazol-3-ium,
- le bromure de 1-butyl-3-hexyl-4-(3-méthoxy-3-oxopropyl)-1*H*-imidazol-3-ium,
- le bromure de 4-(3-méthoxy-3-oxopropyl)-3-méthyl-1-pentyl-1*H*-imidazol-3-ium,
- le bromure de 3-éthyl-4-(3-méthoxy-3-oxopropyl)-1-pentyl-1*H*-imidazol-3-ium,
- le bromure de 4-(3-méthoxy-3-oxopropyl)-1-pentyl-3-propyl-1*H*-imidazol-3-ium,
- le bromure de 3-butyl-4-(3-méthoxy-3-oxopropyl)-1-pentyl-1*H*-imidazol-3-ium,
- le bromure de 4-(3-méthoxy-3-oxopropyl)-1,3-dipentyl-1*H*-imidazol-3-ium,
- le bromure de 3-hexyl-4-(3-méthoxy-3-oxopropyl)-1-pentyl-1*H*-imidazol-3-ium,
- le bromure de 1-hexyl-4-(3-méthoxy-3-oxopropyl)-3-méthyl-1*H*-imidazol-3-ium,
- le bromure de 3-éthyl-1-hexyl-4-(3-méthoxy-3-oxopropyl)-1*H*-imidazol-3-ium,
- le bromure de 1-hexyl-4-(3-méthoxy-3-oxopropyl)-3-propyl-1*H*-imidazol-3-ium,
- le bromure de 3-butyl-1-hexyl-4-(3-méthoxy-3-oxopropyl)-1*H*-imidazol-3-ium,
- le bromure de 1-hexyl-4-(3-méthoxy-3-oxopropyl)-3-pentyl-1*H*-imidazol-3-ium,
- le bromure de 1,3-dihexyl-4-(3-méthoxy-3-oxopropyl)-1*H*-imidazol-3-ium,
- le iodure de 4-(3-méthoxy-3-oxopropyl)-1,2,3-triméthyl-1*H*-imidazol-3-ium,
- le iodure de 3-éthyl-4-(3-méthoxy-3-oxopropyl)-1,2-diméthyl-1*H*-imidazol-3-ium,
- le iodure de 4-(3-méthoxy-3-oxopropyl)-1,2-diméthyl-3-propyl-1*H*-imidazol-3-ium,
- le iodure de 3-butyl-4-(3-méthoxy-3-oxopropyl)-1,2-diméthyl-1*H*-imidazol-3-ium,
- le iodure de 4-(3-méthoxy-3-oxopropyl)-1,2-diméthyl-3-pentyl-1*H*-imidazol-3-ium,
- le iodure de 3-hexyl-4-(3-méthoxy-3-oxopropyl)-1,2-diméthyl-1*H*-imidazol-3-ium,
- le iodure de 1,3-diéthyl-4-(3-méthoxy-3-oxopropyl)-2-méthyl-1*H*-imidazol-3-ium,
- le iodure de 4-(3-méthoxy-3-oxopropyl)-2-méthyl-1,3-dipropyl-1*H*-imidazol-3-ium,
- le iodure de 1,3-dibutyl-4-(3-méthoxy-3-oxopropyl)-2-méthyl-1*H*-imidazol-3-ium,
- le iodure de 4-(3-méthoxy-3-oxopropyl)-2-méthyl-1,3-dipentyl-1*H*-imidazol-3-ium,
- le iodure de 1,3-dihexyl-4-(3-méthoxy-3-oxopropyl)-2-méthyl-1*H*-imidazol-3-ium,
- le iodure de 1-éthyl-3-hexyl-4-(3-méthoxy-3-oxopropyl)-2-méthyl-1*H*-imidazol-3-ium,
- le bromure de 4-(3-méthoxy-3-oxopropyl)-1-méthyl-3-propyl-1*H*-imidazol-3-ium,
- le bromure de 4-(3-méthoxy-3-oxopropyl)-1,2,3-triméthyl-1*H*-imidazol-3-ium,
- le bromure de 3-éthyl-4-(3-méthoxy-3-oxopropyl)-1,2-diméthyl-1*H* imidazol-3-ium,
- le bromure de 4-(3-méthoxy-3-oxopropyl)-1,2-diméthyl-3-propyl-1*H*-imidazol-3-ium,
- le bromure de 3-butyl-4-(3-méthoxy-3-oxopropyl)-1,2-diméthyl-1*H*-imidazol-3-ium,
- le bromure de 4-(3-méthoxy-3-oxopropyl)-1,2-diméthyl-3-pentyl-1*H*-imidazol-3-ium,
- le bromure de 3-hexyl-4-(3-méthoxy-3-oxopropyl)-1,2-dimétliyl-1*H*-imidazol-3-ium,
- le bromure de 1,3-diéthyl-4-(3-méthoxy-3-oxopropyl)-2-méthyl-1*H*-imidazol-3-ium,
- le bromure de 4-(3-méthoxy-3-oxopropyl)-2-méthyl-1,3-dipropyl-1*H*-imidazol-3-ium,
- le bromure de 1,3-dibutyl-4-(3-méthoxy-3-oxopropyl)-2-méthyl-1*H*-imidazol-3-ium,
- le bromure de 4-(3-méthoxy-3-oxopropyl)-2-méthyl-1,3-dipentyl-1*H*-imidazol-3-ium, et
- le iodure de 1-éthyl-3-hexyl-4-(3-méthoxy-3-oxopropyl)-2-méthyl-1*H*-imidazol-3-ium.

4. Cellule photoélectrochimique basée sur la sensibilisation à la lumière de molécules photo-actives comprenant :
- un ensemble formant une photoélectrode comprenant :
i) un substrat transparent revêtu d'une couche d'un matériau d'électrode transparent et conducteur,
ii) une couche photoactive formée sur ledit matériau d'électrode, ladite couche photoactive comprenant un matériau porteur transparent et au moins une molécule photoactive ;
- un ensemble formant une contre-électrode ; et
- une composition d'électrolyte interposée entre ladite couche photoactive et ladite contre-électrode, ladite cellule étant **caractérisée en ce que** ladite composition d'électrolyte comprend, dans un solvant d'électrolyte, un couple redox réversible (I₃/I⁻) à base de diiode et d'au moins un composé de formule (I) tel que défini à l'une quelconque des revendications 1 ou 3.

5. Cellule selon la revendication 4, **caractérisée en ce qu'**elle est une cellule photovoltaïque à colorant.

6. Cellule selon la revendication 4 ou 5, **caractérisée en ce que** la concentration du ou des composés de formule (I) au sein de la composition d'électrolyte varie de 0,1 à 5 mol/L.

7. Cellule selon l'une quelconque des revendications 4 à 6, **caractérisée en ce que** la concentration en diiode au sein de la composition d'électrolyte varie de préférence de 0,001 à 0,5 mol/L.

8. Cellule selon l'une quelconque des revendications 4 à 7, **caractérisée en ce que** le ou les solvants d'électrolyte peuvent être choisis parmi les nitriles ; le sulfolane ; le diméthylsulfoxyde ; le dioxane ; le tétrahydrofurane ; le nitrométhane ; les amides ; la N-méthyl-2-pyrrolidinone ; les carbonates ; les éthylène glycols ; les alcools ; les cétones ; les anhydrides ; les éthers ; l'eau ; les phtalates ; les adipates ; les citrates ; les sébaçates ; les maléates ; les benzoates et les succinates.

9. Cellule selon l'une quelconque des revendications 4 à 7, **caractérisée en ce que** le ou les solvants est un liquide ionique choisi parmi le bis-(trifluorométhanesulfonyl)imide de 1-éthyl-3-méthylimidazolium, le bis-(trifluorométhanesulfonyl)imide de 1-butyl-3-méthylimidazolium, l'hexafluorophosphate de 1-butyl-3-méthylimidazolium, le bis(trifluorométhylsulfonyl)imide de 1-butyl-1-méthylpyrrolidinium et leurs mélanges.

10. Cellule selon l'une quelconque des revendications 4 à 9, **caractérisée en ce que** la composition d'électrolyte renferme en outre un ou plusieurs additifs choisis parmi les dérivés de benzimidazole ; les dérivés de la pyridine ; les dérivés de triazole ; les dérivés de pyrazole ; les dérivés d'imidazole ; le carbonate d'éthylène ; le thiocyanate de guanidine et l'acide chénodésoxycholique ; le iodure de magnésium ; et les sels alcalins de formules LiY ou NaY dans lesquels Y est un anion I⁻, Br⁻, trifluorosulfonate, ClO₄⁻, NO₃⁻ ou TFSI⁻.

11. Cellule selon l'une quelconque des revendications 4 à 10, **caractérisée en ce que** le substrat transparent de la photoélectrode est en un matériau souple ou rigide choisi parmi le verre ou une feuille en un matériau plastique.

12. Cellule selon l'une quelconque des revendications 4 à 11, **caractérisée en ce que** le matériau d'électrode de la photoélectrode est appliqué sur le substrat transparent et se présente sous la forme d'une couche d'un oxyde conducteur transparent choisi parmi l'oxyde d'étain dopé au fluor, l'oxyde d'indium et d'étain, l'oxyde d'indium dopé à l'antimoine, et l'oxyde de zinc dopé à l'aluminium, au gallium ou à l'indium.

13. Cellule selon l'une quelconque des revendications 4 à 12, **caractérisée en ce que** la molécule photoactive de la couche photoactive est un complexe de ruthénium ou d'osmium.

14. Cellule selon l'une quelconque des revendications 4 à 13, **caractérisée en ce que** la contre-électrode est constituée d'un contre-substrat transparent revêtu d'une couche de SnO₂ :F ou de ZnO dopé ou d'un empilement de couches conductrices transparentes et **en ce qu'**une couche de catalyseur est formée sur le matériau formant contre-électrode.

15. Cellule selon la revendication 14, **caractérisée en ce que** la couche de catalyseur est une couche de platine ou de carbone ou de chalcogénure inorganique.

16. Composés de formule (I') suivante : 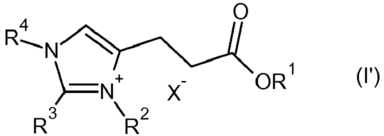 dans laquelle :
- R¹ représente un atome d'hydrogène ou un radical alkyle linéaire ayant de 1 à 10 atomes de carbone,
- R² et R⁴ identiques ou différents représentent chacun un radical alkyle linéaire ou ramifié ayant de 1 à 15 atomes de carbone, ledit radical alkyle étant éventuellement substitué par un ou plusieurs groupements aryle en C₆-C₂₀ pouvant éventuellement comporter un ou plusieurs hétéroatomes choisis parmi les atomes d'oxygène, de soufre et d'azote.
- R³ représente un atome d'hydrogène, un radical alkyle linéaire ou ramifié ayant de 1 à 15 atomes de carbone,
- X⁻ représente un anion iodure ou bromure,
étant entendu que :
- lorsque X⁻ représente un anion iodure, que R¹ représente un radical méthyle, et que R³ est un atome d'hydrogène, alors les radicaux R² et R⁴ ne peuvent représenter simultanément un radical méthyle ou un radical éthyle,
- lorsque X⁻ représente un anion bromure, que R¹ représente un radical méthyle, et que R³ est un atome d'hydrogène, alors les radicaux R² et R⁴ ne peuvent représenter simultanément un radical benzyle,
- lorsque X⁻ représente un anion bromure, que R¹ représente un radical méthyle, que R² est un radical butyle et que R³ est un atome d'hydrogène, alors le radical R⁴ est différent d'un radical méthyle, éthyle ou benzyle,
- lorsque X⁻ représente un anion iodure, que R¹ et R² représentent un radical méthyle, et que R³ est un atome d'hydrogène, alors le radical R⁴ est différent d'un radical trityle, et
- lorsque X⁻ représente un anion bromure, que R¹ représente un radical méthyle, que R² est un radical butyle et que R³ est un radical isopropyle, alors le radical R⁴ est différent d'un radical méthyle.

17. Composé de formule (I') selon la revendication 16, **caractérisé en ce qu'**il est choisi parmi :
- le iodure de 4-(3-éthoxy-3-oxopropyl)-1,3-diméthyl-1*H*-imidazol-3-ium,
- le iodure de 4-(3-méthoxy-3-oxopropyl)-1,3-dipropyl-1*H*-imidazol-3-ium,
- le iodure de 4-(3-méthoxy-3-oxopropyl)-1,3-dibutyl-1*H*-imidazol-3-ium,
- le iodure de 4-(3-méthoxy-3-oxopropyl)-1,3-dipentyl-1*H*-imidazol-3-ium,
- le iodure de 4-(3-méthoxy-3-oxopropyl)-1,3-dihexyl-1*H*-imidazol-3-ium,
- le iodure de 3-éthyl-4-(3-méthoxy-3-oxopropyl)-1-méthyl-1*H*-imidazol-3-ium,
- le iodure de 4-(3-méthoxy-3-oxopropyl)-1-méthyl-3-propyl-1*H*-imidazol-3-ium,
- le iodure de 3-butyl-4-(3-méthoxy-3-oxopropyl)-1-méthyl-1*H*-imidazol-3-ium,
- le iodure de 4-(3-méthoxy-3-oxopropyl)-1-méthyl-3-pentyl-1*H*-imidazol-3-ium,
- le iodure de 3-hexyl-4-(3-méthoxy-3-oxopropyl)-1-méthyl-1*H*-imidazol-3-ium,
- le iodure de 1-éthyl-4-(3-méthoxy-3-oxopropyl)-3-méthyl-1*H*-imidazol-3-ium,
- le iodure de 1-éthyl-4-(3-méthoxy-3-oxopropyl)-3-propyl-1*H*-imidazol-3-ium,
- le iodure de 3-butyl-1-éthyl-4-(3-méthoxy-3-oxopropyl)-1*H*-imidazol-3-ium,
- le iodure de 1-éthyl-4-(3-méthoxy-3-oxopropyl)-3-pentyl-1*H*-imidazol-3-ium,
- le iodure de 1-éthyl-3-hexyl-4-(3-méthoxy-3-oxopropyl)-1*H*-imidazol-3-ium,
- le iodure de 4-(3-méthoxy-3-oxopropyl)-3-méthyl-1-propyl-1*H*-imidazol-3-ium,
- le iodure de 3-éthyl-4-(3-méthoxy-3-oxopropyl)-1-propyl-1*H*-imidazol-3-ium,
- le iodure de 4-(3-méthoxy-3-oxopropyl)-1,3-dipropyl-1*H*-imidazol-3-ium,
- le iodure de 3-butyl-4-(-3-méthoxy-3-oxopropyl)-1-propyl-1*H*-imidazol-3-ium,
- le iodure de 4-(3-méthoxy-3-oxopropyl)-3-pentyl-1-propyl-1*H*-imidazol-3-ium,
- le iodure de 3-hexyl-4-(3-méthoxy-3-oxopropyl)-1-propyl-1*H*-imidazol-3-ium,
- le iodure de 1-butyl-4-(3-méthoxy-3-oxopropyl)-3-méthyl-1*H*-imidazol-3-ium,
- le iodure de 1-butyl-3-éthyl-4-(3-méthoxy-3-oxopropyl)-1*H*-imidazol-3-ium,
- le iodure de 1-butyl-4-(3-méthoxy-3-oxopropyl)-3-propyl-1*H*-imidazol-3-ium,
- le iodure de 1,3-dibutyl-4-(3-méthoxy-3-oxopropyl)-1*H*-imidazol-3-ium,
- le iodure de 1-butyl-4-(3-méthoxy-3-oxopropyl)-3-pentyl-1*H*-imidazol-3-ium,
- le iodure de 1-butyl-3-hexyl-4-(3-méthoxy-3-oxopropyl)-1*H*-imidazol-3-ium,
- le iodure de 4-(3-méthoxy-3-oxopropyl)-3-méthyl-1-pentyl-1*H*-imidazol-3-ium,
- le iodure de 3-éthyl-4-(3-méthoxy-3-oxopropyl)-1-pentyl-1*H*-imidazol-3-ium,
- le iodure de 4-(3-méthoxy-3-oxopropyl)-1-pentyl-3-propyl-1*H*-imidazol-3-ium,
- le iodure de 3-butyl-4-(3-méthoxy-3-oxopropyl)-1-pentyl-1*H*-imidazol-3-ium,
- le iodure de 4-(3-méthoxy-3-oxopropyl)-1,3-dipentyl-1*H*-imidazol-3-ium,
- le iodure de 3-hexyl-4-(3-méthoxy-3-oxopropyl)-1-pentyl-1*H*-imidazol-3-ium,
- le iodure de 1-hexyl-4-(3-méthoxy-3-oxopropyl)-3-méthyl-1*H*-imidazol-3-ium,
- le iodure de 3-éthyl-1-hexyl-4-(3-méthoxy-3-oxopropyl)-1*H*-imidazol-3-ium,
- le iodure de 1-hexyl-4-(3-méthoxy-3-oxopropyl)-3-propyl-1*H*-imidazol-3-ium,
- le iodure de 3-butyl-1-hexyl-4-(3-méthoxy-3-oxopropyl)-1*H*-imidazol-3-ium,
- le iodure de 1-hexyl-4-(3-méthoxy-3-oxopropyl)-3-pentyl-1*H*-imidazol-3-ium,
- le iodure de 1,3-dihexyl-4-(3-méthoxy-3-oxopropyl)-1*H*-imidazol-3-ium,
- le bromure de 3-éthyl-4-(3-méthoxy-3-oxopropyl)-1-méthyl-1*H*-imidazol-3-ium,
- le bromure de 4-(3-méthoxy-3-oxopropyl)-1-méthyl-3-propyl-1*H*-imidazol-3-ium,
- le bromure de 4-(3-méthoxy-3-oxopropyl)-1-méthyl-3-pentyl-1*H*-imidazol-3-ium,
- le bromure de 3-hexyl-4-(3-méthoxy-3-oxopropyl)-1-méthyl-1*H*-imidazol-3-ium,
- le bromure de 1-éthyl-4-(3-méthoxy-3-oxopropyl)-3-méthyl-1*H*-imidazol-3-ium,
- le bromure de 1,3-diéthyl-4-(-3-méthoxy-3-oxopropyl)-1*H*-imidazol-3-ium,
- le bromure de 1-éthyl-4-(3-méthoxy-3-oxopropyl)-3-propyl-1*H*-imidazol-3-ium,
- le bromure de 1-éthyl-4-(3-méthoxy-3-oxopropyl)-3-pentyl-1*H*-imidazol-3-ium,
- le bromure de 1-éthyl-3-hexyl-4-(3-méthoxy-3-oxopropyl)-1*H*-imidazol-3-ium,
- le bromure de 4-(3-méthoxy-3-oxopropyl)-3-méthyl-1-propyl-1*H*-imidazol-3-ium,
- le bromure de 3-éthyl-4-(3-méthoxy-3-oxopropyl)-1-propyl-1*H*-imidazol-3-ium,
- le bromure de 4-(3-méthoxy-3-oxopropyl)-1,3-dipropyl-1*H*-imidazol-3-ium,
- le bromure de 3-butyl-4-(-3-méthoxy-3-oxopropyl)-1-propyl-1*H*-imidazol-3-ium,
- le bromure de 4-(3-méthoxy-3-oxopropyl)-3-pentyl-1-propyl-1*H*-imidazol-3-ium,
- le bromure de 3-hexyl-4-(3-méthoxy-3-oxopropyl)-1-propyl-1*H*-imidazol-3-ium,
- le bromure de 1-butyl-4-(3-méthoxy-3-oxopropyl)-3-propyl-1*H*-imidazol-3-ium,
- le bromure de 1,3-dibutyl-4-(3-méthoxy-3-oxopropyl)-1*H*-imidazol-3-ium,
- le bromure de 1-butyl-4-(3-méthoxy-3-oxopropyl)-3-pentyl-1*H*-imidazol-3-ium,
- le bromure de 1-butyl-3-hexyl-4-(3-méthoxy-3-oxopropyl)-1*H*-imidazol-3-ium,
- le bromure de 4-(3-méthoxy-3-oxopropyl)-3-méthyl-1-pentyl-1*H*-imidazol-3-ium,
- le bromure de 3-éthyl-4-(3-méthoxy-3-oxopropyl)-1-pentyl-1*H*-imidazol-3-ium,
- le bromure de 4-(3-méthoxy-3-oxopropyl)-1-pentyl-3-propyl-1*H*-imidazol-3-ium,
- le bromure de 3-butyl-4-(3-méthoxy-3-oxopropyl)-1-pentyl-1*H*-imidazol-3-ium,
- le bromure de 4-(3-méthoxy-3-oxopropyl)-1,3-dipentyl-1*H*-imidazol-3-ium,
- le bromure de 3-hexyl-4-(3-méthoxy-3-oxopropyl)-1-pentyl-1*H*-imidazol-3-ium,
- le bromure de 1-hexyl-4-(3-méthoxy-3-oxopropyl)-3-méthyl-1*H*-imidazol-3-ium,
- le bromure de 3-éthyl-1-hexyl-4-(3-méthoxy-3-oxopropyl)-1*H*-imidazol-3-ium,
- le bromure de 1-hexyl-4-(3-méthoxy-3-oxopropyl)-3-propyl-1*H*-imidazol-3-ium,
- le bromure de 3-butyl-1-hexyl-4-(3-méthoxy-3-oxopropyl)-1*H*-imidazol-3-ium,
- le bromure de 1-hexyl-4-(3-méthoxy-3-oxopropyl)-3-pentyl-1*H*-imidazol-3-ium,
- le bromure de 1,3-dihexyl-4-(3-méthoxy-3-oxopropyl)-1*H*-imidazol-3-ium,
- le iodure de 4-(3-méthoxy-3-oxopropyl)-1,2,3-triméthyl-1*H*-imidazol-3-ium,
- le iodure de 3-éthyl-4-(3-méthoxy-3-oxopropyl)-1,2-diméthyl-1*H*-imidazol-3-ium,
- le iodure de 4-(3-méthoxy-3-oxopropyl)-1,2-diméthyl-3-propyl-1*H*-imidazol-3-ium,
- le iodure de 3-butyl-4-(3-méthoxy-3-oxopropyl)-1,2-diméthyl-1*H*-imidazol-3-ium,
- le iodure de 4-(3-méthoxy-3-oxopropyl)-1,2-diméthyl-3-pentyl-1*H*-imidazol-3-ium,
- le iodure de 3-hexyl-4-(3-méthoxy-3-oxopropyl)-1,2-diméthyl-1*H*-imidazol-3-ium,
- le iodure de 1,3-diéthyl-4-(3-méthoxy-3-oxopropyl)-2-méthyl-1*H*-imidazol-3-ium,
- le iodure de 4-(3-méthoxy-3-oxopropyl)-2-méthyl-1,3-dipropyl-1*H*-imidazol-3-ium,
- le iodure de 1,3-dibutyl-4-(3-méthoxy-3-oxopropyl)-2-méthyl-1*H*-imidazol-3-ium,
- le iodure de 4-(3-méthoxy-3-oxopropyl)-2-méthyl-1,3-dipentyl-1*H*-imidazol-3-ium,
- le iodure de 1,3-dihexyl-4-(3-méthoxy-3-oxopropyl)-2-méthyl-1*H*-imidazol-3-ium,
- le iodure de 1-éthyl-3-hexyl-4-(3-méthoxy-3-oxopropyl)-2-méthyl-1*H*-imidazol-3-ium,
- le bromure de 4-(3-méthoxy-3-oxopropyl)-1-méthyl-3-propyl-1*H*-imidazol-3-ium,
- le bromure de 4-(3-méthoxy-3-oxopropyl)-1,2,3-triméthyl-1*H*-imidazol-3-ium,
- le bromure de 3-éthyl-4-(3-méthoxy-3-oxopropyl)-1,2-diméthyl-1*H*-imidazol-3-ium,
- le bromure de 4-(3-méthoxy-3-oxopropyl)-1,2-diméthyl-3-propyl-1*H*-imidazol-3-ium,
- le bromure de 3-butyl-4-(3-méthoxy-3-oxopropyl)-1,2-diméthyl-1*H*-imidazol-3-ium,
- le bromure de 4-(3-méthoxy-3-oxopropyl)-1,2-diméthyl-3-pentyl-1*H*-imidazol-3-ium,
- le bromure de 3-hexyl-4-(3-méthoxy-3-oxopropyl)-1,2-diméthyl-1*H*-imidazol-3-ium,
- le bromure de 1,3-diéthyl-4-(3-méthoxy-3-oxopropyl)-2-méthyl-1*H*-imidazol-3-ium,
- le bromure de 4-(3-méthoxy-3-oxopropyl)-2-méthyl-1,3-dipropyl-1*H*-imidazol-3-ium,
- le bromure de 1,3-dibutyl-4-(3-méthoxy-3-oxopropyl)-2-méthyl-1*H*-imidazol-3-ium,
- le bromure de 4-(3-méthoxy-3-oxopropyl)-2-méthyl-1,3-dipentyl-1*H*-imidazol-3-ium, et
- le iodure de 1-éthyl-3-hexyl-4-(3-méthoxy-3-oxopropyl)-2-méthyl-1*H*-imidazol-3-ium.

## Patentansprüche

1. Verwendung von mindestens einem Halogenid der folgenden Formel (I): 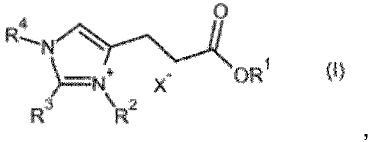 worin:
- R¹ ein Wasserstoffatom oder einen linearen Alkylrest mit 1 bis 10 Kohlenstoffatomen darstellt;
- R² und R⁴, die gleich oder verschieden sind, jeweils einen linearen oder verzweigten Alkylrest mit 1 bis 15 Kohlenstoffatomen darstellen, wobei der Alkylrest gegebenenfalls mit einer oder mehreren C₆-C₂₀-Arylgruppen substituiert ist, wobei die Arylgruppen gegebenenfalls ein oder mehrere Heteroatome aufweisen, ausgewählt aus Sauerstoff-, Schwefel- und Stickstoffatomen;
- R³ ein Wasserstoffatom oder einen linearen oder verzweigten Alkylrest mit 1 bis 15 Kohlenstoffatomen darstellt,
- X⁻ ein lodid- oder Bromidanion darstellt,
als Elektrolytsalz in einer Elektrolytzusammensetzung von einer photoelektrochemischen Zelle, wobei die photoelektrochemische Zelle auf Lichtsensibilisierung von photoaktiven Molekülen basiert.

2. Verwendung nach Anspruch 1, **dadurch gekennzeichnet, dass** die photoelektrochemische Zelle, die auf der Lichtsensibilisierung von photoaktiven Molekülen basiert, eine photovoltaische Farbstoffzelle ist.

3. Verwendung nach Anspruch 1 oder 2, **dadurch gekennzeichnet, dass** die Verbindungen der Formel (I) ausgewählt sind aus:
- 4-(3-Methoxy-3-oxopropyl)-1,3-dimethyl-1*H*-imidazol-3-iumiodid,
- 4-(3-Ethoxy-3-oxopropyl)-1,3-dimethyl-1*H*-imidazol-3-iumiodid,
- 4-(3-Methoxy-3-oxopropyl)-1,3-diethyl-1*H*-imidazol-3-iumiodid,
- 4-(3-Methoxy-3-oxopropyl)-1,3-dipropyl-1*H*-imidazol-3-iumiodid,
- 4-(3-Methoxy-3-oxopropyl)-1,3-dibutyl-1*H*-imidazol-3-iumiodid,
- 4-(3-Methoxy-3-oxopropyl)-1,3-dipentyl-1*H*-imidazol-3-iumiodid,
- 4-(3-Methoxy-3-oxopropyl)-1,3-dihexyl-1*H*-imidazol-3-iumiodid,
- 4-(3-Methoxy-3-oxopropyl)-1-methyl-3-propyl-1*H*-imidazol-3-iumiodid,
- 3-Ethyl-4-(3-methoxy-3-oxopropyl)-1-methyl-1*H*-imidazol-3-iumiodid,
- 4-(3-Methoxy-3-oxopropyl)-1-methyl-3-propyl-1*H*-imidazol-3-iumiodid,
- 3-Butyl-4-(3-methoxy-3-oxopropyl)-1-methyl-1*H*-imidazol-3-iumiodid,
- 4-(3-Methoxy-3-oxopropyl)-1-methyl-3-pentyl-1*H*-imidazol-3-iumiodid,
- 3-Hexyl-4-(3-methoxy-3-oxopropyl)-1-methyl-1*H*-imidazol-3-iumiodid,
- 1-Ethyl-4-(3-methoxy-3-oxopropyl)-3-methyl-1*H*-imidazol-3-iumiodid,
- 1,3-Diethyl-4-(3-methoxy-3-oxopropyl)-1*H*-imidazol-3-iumiodid,
- 1-Ethyl-4-(3-methoxy-3-oxopropy)-3-propyl-1*H*-imidazol-3-iumiodid,
- 3-Butyl-1-ethyl-4-(3-methoxy-3-oxopropyl)-1*H*-imidazol-3-iumiodid;
- 1-Ethyl-4-(3-methoxy-3-oxopropyl)-3-pentyl-1*H*-imidazol-3-iumiodid,
- 1-Ethyl-3-hexyl-4-(3-methoxy-3-oxopropyl)-1*H*-imidazol-3-iumiodid,
- 4-(3-Methoxy-3-oxopropyl)-3-methyl-1-propyl-1*H*-imidazol-3-iumiodid,
- 3-Ethyl-4-(3-methoxy-3-oxopropyl)-1-propyl-1*H*-imidazol-3-iumiodid,
- 4-(3-Methoxy-3-oxopropyl)-1,3-dipropyl-1*H*-imidazol-3-iumiodid,
- 3-Butyl-4-(3-methoxy-3-oxopropyl)-1-propyl-1*H*-imidazol-3-iumiodid,
- 4-(3-Methoxy-3-oxopropyl)-3-pentyl-1-propyl-1*H*-imidazol-3-iumiodid,
- 3-Hexyl-4-(3-methoxy-3-oxopropyl)-1-propyl-1*H*-imidazol-3-iumodid,
- 1-Butyl-4-(3-methoxy-3-oxopropyl)-3-methyl-1*H*-imidazol-3-iumiodid,
- 1-Butyl-3-ethyl-4-(3-methoxy-3-oxopropyl)-1*H*-imidazol-3-iumiodid,
- 1-Butyl-4-(3-methoxy-3-oxopropyl)-3-propyl-1*H*-imidazol-3-iumiodid,
- 1,3-Dibutyl-4-(3-methoxy-3-Oxopropyl)-1*H*-imidazol-3-iumiodid,
- 1-Butyl-4-(3-methoxy-3-oxopropyl)-3-pentyl-1*H*-imidazol-3-iumiodid;
- 1-Butyl-3-hexyl-4-(3-methoxy-3-oxopropyl)-1*H*-imidazol-3-iumiodid,
- 4-(3-Methoxy-3-oxopropyl)-3-methyl-1-pentyl-1*H*-imidazol-3-iumiodid,
- 3-Ethyl-4-(3-methoxy-3-oxopropyl)-1-pentyl-1*H*-imidazol-3-iumiodid,
- 4-(3-Methoxy-3-oxopropyl)-1-pentyl-3-propyl-1*H*-imidazol-3-iumiodid,
- 3-Butyl-4-(3-methoxy-3-oxopropyl)-1-pentyl-1*H*-imidazol-3-iumiodid,
- 4-(3-Methoxy-3-oxopropyl)-1,3-dipentyl-1*H*-imidazol-3-iumiodid,
- 3-Hexyl-4-(3-methoxy-3-oxopropyl)-1-pentyl-1*H*-imidazol-3-iumiodid,
- 1-Hexyl-4-(3-methoxy-3-oxopropyl)-3-methyl-1*H*-imidazol-3-iumiodid,
- 3-Ethyl-1-hexyl-4-(3-methoxy-3-oxopropyl)-1*H*-imidazol-3-iumiodid,
- 1-Hexyl-4-(3-methoxy-3-oxopropyl)-3-propyl-1*H*-imidazol-3-iumiodid;
- 3-Butyl-1-hexyl-4-(3-methoxy-3-oxopropyl)-1*H*-imidazol-3-iumiodid,
- 1-Hexyl-4-(3-methoxy-3-oxopropyl)-3-pentyl-1*H*-imidazol-3-iumiodid,
- 1,3-Dihexyl-4-(3-methoxy-3-oxopropyl)-1*H*-imidazol-3-iumiodid,
- 3-Ethyl-4-(3-methoxy-3-oxopropyl)-1-methyl-1*H*-imidazol-3-iumbromid,
- 4-(3-Methoxy-3-oxopropyl)-1-methyl-3-propyl-1*H*-imidazol-3-iumbromid,
- 3-butyl-4-(3-methoxy-3-oxopropyl)-1-methyl-1*H*-imidazol-3-iumbromid,
- 4-(3-Methoxy-3-oxopropyl)-1-methyl-3-pentyl-1*H*-imidazol-3-iumbromid,
- 3-Hexyl-4-(3-methoxy-3-oxopropyl)-1-methyl-1*H*-imidazol-3-iumbromid;
- 1-Ethyl-4-(3-methoxy-3-oxopropyl)-3-methyl-1*H*-imidazol-3-iumbromid;
- 1,3-Diethyl-4-(3-methoxy-3-oxopropyl)-1*H*-imidazol-3-iumbromid,
- 1-Ethyl-4-(3-methoxy-3-oxopropyl)-3-propyl-1*H*-imidazol-3-iumbromid,
- 3-Butyl-1-ethyl-4-(3-methoxy-3-oxopropyl)-1*H*-imidazol-3-iumbromid,
- 1-Ethyl-4-(3-methoxy-3-oxopropyl)-3-pentyl-1*H*-imidazol-3-iumbromid,
- 1-Ethyl-3-hexyl-4-(3-methoxy-3-oxopropyl)-1*H*-imidazol-3-iumbromid,
- 4-(3-Methoxy-3-oxopropyl)-3-methyl-1-propyl-1*H*-imidazol-3-iumbromid,
- 3-Ethyl-4-(3-methoxy-3-oxopropyl)-1-propyl-1*H*-imidazol-3-iumbromid,
- 4-(3-Methoxy-3-oxopropyl)-1,3-dipropyl-1*H*-imidazol-3-iumbromid,
- 3-Butyl-4-(3-methoxy-3-oxopropyl)-1-Propyl-1*H*-imidazol-3-iumbromid;
- 4-(3-Methoxy-3-oxopropyl)-3-pentyl-1-propyl-1*H*-imidazol-3-iumbromid;
- 3-Hexyl-4-(3-methoxy-3-oxopropyl)-1-propyl-1*H*-imidazol-3-iumbromid,
- 1-Butyl-4-(3-methoxy-3-oxopropyl)-3-methyl-1*H*-imidazol-3-iumbromid,
- 1-Butyl-3-ethyl-4-(3-methoxy-3-oxopropyl)-1*H*-imidazol-3-iumbromid,
- 1-Butyl-4-(3-methoxy-3-oxopropyl)-3-propyl-1*H*-imidazol-3-iumbromid,
- 1,3-Dibutyl-4-(3-methoxy-3-oxopropyl)-1*H*-imidazol-3-iumbromid,
- 1-Butyl-4-(3-methoxy-3-oxopropyl)-3-pentyl-1*H*-imidazol-3-iumbromid,
- 1-Butyl-3-hexyl-4-(3-methoxy-3-oxopropyl)-1*H*-imidazol-3-iumbromid,
- 4-(3-Methoxy-3-oxopropyl)-3-methyl-1-pentyl-1*H*-imidazol-3-iumbromid;
- 3-Ethyl-4-(3-methoxy-3-oxopropyl)-1-Pentyl-1*H*-imidazol-3-iumbromid,
- 4-(3-Methoxy-3-oxopropyl)-1-pentyl-3-propyl-1*H*-imidazol-3-iumbromid,
- 3-Butyl-4-(3-methoxy-3-oxopropyl)-1-pentyl-1*H*-imidazol-3-iumbromid,
- 4-(3-Methoxy-3-oxopropyl)-1,3-dipentyl-1*H*-imidazol-3-iumbromid,
- 3-Hexyl-4-(3-methoxy-3-oxopropyl)-1-pentyl-1*H*-imidazol-3-iumbromid,
- 1-Hexyl-4-(3-methoxy-3-oxopropyl)-3-methyl-1*H*-imidazol-3-iumbromid,
- 3-Ethyl-1-hexyl-4-(3-methoxy-3-oxopropyl)-1*H*-imidazol-3-iumbromid,
- 1-Hexyl-4-(3-methoxy-3-oxopropyl)-3-propyl-1*H*-imidazol-3-iumbromid,
- 3-Butyl-1-hexyl-4-(3-methoxy-3-oxopropyl)-1*H*-imidazol-3-iumbromid,
- 1-Hexyl-4-(3-methoxy-3-oxopropyl)-3-pentyl-1*H*-imidazol-3-iumbromid,
- 1,3-Dihexyl-4-(3-methoxy-3-oxopropyl)-1*H*-imidazol-3-iumbromid,
- 4-(3-Methoxy-3-oxopropyl)-1,2,3-trimethyl-1*H*-imidazol-3-iumiodid,
- 3-Ethyl-4-(3-methoxy-3-oxopropyl)-1,2-dimethyl-1*H*-imidazol-3-iumiodid,
- 4-(3-Methoxy-3-oxopropyl)-1,2-dimethyl-3-propyl-1*H*-imidazol-3-iumiodid;
- 3-Butyl-4-(3-methoxy-3-oxopropyl)-1,2-dimethyl-1*H*-imidazol-3-iumiodid,
- 4-(3-Methoxy-3-oxopropyl)-1,2-dimethyl-3-pentyl-1*H*-imidazol-3-iumiodid,
- 3-Hexyl-4-(3-methoxy-3-oxopropyl)-1,2-dimethyl-1*H*-imidazol-3-iumiodid,
- 1,3-Diethyl-4-(3-methoxy-3-oxopropyl)-2-methyl-1*H*-imidazol-3-iumiodid,
- 4-(3-Methoxy-3-oxopropyl)-2-methyl-1,3-dipropyl-1*H*-imidazol-3-iumiodid,
- 1,3-Dibutyl-4-(3-methoxy-3-oxopropyl)-2-methyl-1*H*-imidazol-3-iumiodid,
- 4-(3-Methoxy-3-oxopropyl)-2-methyl-1,3-dipentyl-1*H*-imidazol-3-iumiodid,
- 1,3-Dihexyl-4-(3-methoxy-3-oxopropyl)-2-methyl-1*H*-imidazol-3-iumiodid,
- 1-Ethyl-3-hexyl-4-(3-methoxy-3-oxopropyl)-2-methyl-1*H*-imidazol-3-iumiodid,
- 4-(3-Methoxy-3-oxopropyl)-1-methyl-3-propyl-1*H*-imidazol-3-iumbromid,
- 4-(3-Methoxy-3-oxopropyl)-1,2,3-trimethyl-1*H*-imidazol-3-iumbromid,
- 3-Ethyl-4-(3-methoxy-3-oxopropyl)-1,2-dimethyl-1*H*-imidazol-3-iumbromid,
- 4-(3-Methoxy-3-oxopropyl)-1,2-dimethyl-3-propyl-1*H*-imidazol-3-iumbromid,
- 3-Butyl-4-(3-methoxy-3-oxopropyl)-1,2-dimethyl-1*H*-Imidazol-3-iumbromid,
- 4-(3-Methoxy-3-oxopropyl)-1,2-dimethyl-3-pentyl-1*H*-imidazol-3-iumbromid,
- 3-Hexyl-4-(3-methoxy-3-oxopropyl)-1,2-dimethyl-1*H*-imidazol-3-iumbromid;
- 1,3-Diethyl-4-(3-methoxy-3-oxopropyl)-2-methyl-1*H*-imidazol-3-iumbromid,
- 4-(3-Methoxy-3-oxopropyl)-2-methyl-1,3-dipropyl-1*H*-imidazol-3-iumbromid,
- 1,3-Dibutyl-4-(3-methoxy-3-oxopropyl)-2-methyl-1*H*-imidazol-3-iumbromid;
- 4-(3-Methoxy-3-oxopropyl)-2-methyl-1,3-dipentyl-1*H*-imidazol-3-iumbromid, und
- 1-Ethyl-3-hexyl-4-(3-methoxy-3-oxopropyl)-2-methyl-1*H*-imidazol-3-iumiodid.

4. Photoelektrochemische Zelle, die auf Lichtsensibilisierung von photoaktiven Molekülen basiert, umfassend:
- eine Anordnung, die eine Photoelektrode bildet; umfassend:
- i) ein transparentes Substrat, welches beschichtet ist mit einer Schicht aus einem transparenten und leitenden Elektrodenmaterial ist,
- ii) eine photoaktive, auf dem Elektrodenmaterial gebildete Schicht, wobei die photoaktive Schicht ein transparentes Trägermaterial und mindestens ein photoaktives Molekül umfasst;
- eine Anordnung, die eine Gegenelektrode bildet, und
- eine Elektrolytzusammensetzung, die zwischen der photoaktiven Schicht und der Gegenelektrode angeordnet ist, wobei die Zelle **dadurch gekennzeichnet ist, dass** die Elektrolytzusammensetzung in einem Elektrolytlösungsmittel ein reversibles Redoxpaar (I₃/I⁻) umfasst, welches auf Basis von Diiode und von mindestens einer Verbindung der Formel (I) nach einem der Ansprüche 1 oder 3 ist.

5. Zelle nach Anspruch 4, **dadurch gekennzeichnet, dass** es sich um eine photovoltaische Farbstoffzelle handelt.

6. Zelle nach Anspruch 4 oder 5, **dadurch gekennzeichnet, dass** die Konzentration der Verbindung (en) der Formel (I) in der Elektrolytzusammensetzung von 0,1 bis 5 mol/l variiert.

7. Zelle nach einem der Ansprüche 4 bis 6, **dadurch gekennzeichnet, dass** die Diiode-Konzentration in der Elektrolytzusammensetzung vorzugsweise von 0,001 bis 0,5 mol / L variiert.

8. Zelle nach einem der Ansprüche 4 bis 7, **dadurch gekennzeichnet, dass** das oder die Elektrolytlösungsmittel ausgewählt sind aus Nitrilen; Sulfolan; Dimethylsulfoxid; Dioxan; Tetrahydrofuran; Nitromethan; Amiden; N-Methyl-2-pyrrolidinon; Karbonaten; Ethylenglykolen; Alkoholen; Ketonen; Anhydriden; Äthern; Wasser; Phthalaten; Adipaten; Zitraten; Sebacaten; Maleaten; Benzoaten und Succinaten.

9. Zelle nach einem der Ansprüche 4 bis 7, **dadurch gekennzeichnet, dass** das oder die Lösungsmittel eine ionische Flüssigkeit ist bzw. sind, die ausgewählt ist aus 1-Ethyl-3-methylimidazolium-bis-(trifluormethansulfonyl)imid, 1-Butyl-3-methylimidazolium-bis-(trifluormethansulfonyl)imid, 1-Butyl-3-methylimidazolium-hexafluorophosphat, 1-Butyl-1-methylpyrrolidinium-bis-(trifluormethylsulfonyl)imid, und Mischungen davon.

10. Zelle nach einem der Ansprüche 4 bis 9, **dadurch gekennzeichnet, dass** die Elektrolytzusammensetzung zusätzlich ein oder mehrere Additive umfasst, die ausgewählt sind aus Benzimidazol-; Pyridin-; Triazol-; Pyrazol-; Imidazolderivaten; Ethylenkarbonat; Guanidinthiocyanat und Chenodesoxycholsäure; Magnesiumiodid; und Alkalinsalzen der Formeln LiY oder NaY, worin Y ein I⁻-, Br⁻-, Trifluorsulfonat-, ClO₄⁻-, NO₃⁻- oder TFSI⁻-Anion ist.

11. Zelle nach einem der Ansprüche 4 bis 10, **dadurch gekennzeichnet, dass** das transparente Substrat der Photoelektrode aus einem flexiblen oder starren Material ist, das aus Glas oder einer Platte aus einem Kunststoffmaterial ausgewählt ist.

12. Zelle nach einem der Ansprüche 4 bis 11, **dadurch gekennzeichnet, dass** das Elektrodenmaterial der Photoelektrode auf das transparente Substrat aufgebracht ist, und in Form einer Schicht eines transparenten leitfähigen Oxids vorliegt, wobei das Oxid ausgewählt ist aus fluordotiertem Zinnoxid, Indium- und Zinnoxid, antimondotiertem Indiumoxid, und aluminium-, gallium oder indiumdotiertem Zinkoxid.

13. Zelle nach einem der Ansprüche 4 bis 12, **dadurch gekennzeichnet, dass** das
photoaktive Molekül der photoaktiven Schicht ein Ruthenium oder Osmiumkomplex ist.

14. Zelle nach einem der Ansprüche 4 bis 13, **dadurch gekennzeichnet, dass** die
Gegenelektrode aus einem transparenten Gegensubstrat besteht, wobei das Gegensubstrat mit einer Schicht aus SnO₂:F oder dotiertem ZnO oder einem Stapel aus leitenden transparenten Schichten beschichtet ist, und dadurch, dass eine Katalysatorschicht auf dem die Gegenelektrode bildenden Material gebildet ist.

15. Zelle nach Anspruch 14, **dadurch gekennzeichnet, dass** die Katalysatorschicht eine Schicht aus Platin oder Kohlenstoff oder anorganischem Chalkogenid ist.

16. Verbindungen der folgenden Formel (I'): 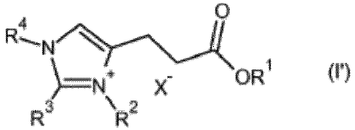 worin:
- R¹ ein Wasserstoffatom oder einen linearen Alkylrest mit 1 bis 10 Kohlenstoffatomen darstellt;
- R² und R⁴, die gleich oder verschieden sind, jeweils für einen linearen oder verzweigten Alkylrest mit 1 bis 15 Kohlenstoffatomen darstellen, wobei der Alkylrest gegebenenfalls mit einer oder mehreren C₆-C₂₀-Arylgruppen substituiert ist, wobei die Arylgruppen gegebenenfalls ein oder mehrere Heteroatome aufweisen, ausgewählt aus Sauerstoff-, Schwefel- und Stickstoffatomen;
- R³ ein Wasserstoffatom oder einen linearen oder verzweigten Alkylrest mit 1 bis 15 Kohlenstoffatomen darstellt,
- X⁻ ein lodid- oder Bromidanion darstellt,
wobei folgendes zu verstehen ist:
- wenn X⁻ ein lodidanion darstellt, R¹ einen Methylrest darstellt und R³ ein Wasserstoffatom ist, dann können die Reste R² und R⁴ nicht gleichzeitig einen Methylrest oder einen Ethylrest darstellen,
- wenn X⁻ ein Bromidanion darstellt, R¹ einen Methylrest darstellt; und R³ ein Wasserstoffatom ist, dann können die Reste R² und R⁴ nicht gleichzeitig einen Benzylrest darstellen,
- wenn X⁻ ein Bromidanion darstellt, R¹ einen Methylrest darstellt, R² ein Butylrest ist und R³ ein Wasserstoffatom ist, dann ist der Rest R⁴ verschieden von einem Methyl-, Ethyl- oder Benzylrest,
- wenn X⁻ ein lodidanion darstellt, R¹ und R² einen Methylrest darstellen und R³ ein Wasserstoffatom ist, dann ist der Rest R⁴ unterschiedlich als ein Tritylrest, und
- wenn X⁻ ein Bromidanion darstellt, R¹ einen Methylrest darstellt, R² ein Butylrest ist und R³ ein Isopropylrest ist, dann ist der Rest R⁴ unterschiedlich als ein Methylrest.

17. Verbindung der Formel (I') nach Anspruch 16, **dadurch gekennzeichnet, dass** sie ausgewählt ist aus:
- 4-(3-Ethoxy-3-oxopropyl)-1,3-dimethyl-1*H*-imidazol-3-iumiodid,
- 4-(3-Methoxy-3-oxopropyl)-1,3-dipropyl-1*H*-imidazol-3-iumiodid,
- 4-(3-Methoxy-3-oxopropyl)-1,3-dibutyl-1*H*-imidazol-3-iumiodid,
- 4-(3-Methoxy-3-oxopropyl)-1,3-dipentyl-1*H*-imidazol-3-iumiodid,
- 4-(3-Methoxy-3-oxopropyl)-1,3-dihexyl-1*H*-imidazol-3-iumiodid,
- 3-Ethyl-4-(3-methoxy-3-oxopropyl)-1-methyl-1*H*-imidazol-3-iumiodid,
- 4-(3-Methoxy-3-oxopropyl)-1-methyl-3-propyl-1*H*-imidazol-3-iumiodid,
- 3-Butyl-4-(3-methoxy-3-oxopropyl)-1-methyl-1*H*-imidazol-3-iumiodid,
- 4-(3-Methoxy-3-oxopropyl)-1-methyl-3-pentyl-1*H*-imidazol-3-iumiodid,
- 3-Hexyl-4-(3-Methoxy-3-oxopropyl)-1-methyl-1*H*-imidazol-3-iumiodid,
- 1-Ethyl-4-(3-methoxy-3-oxopropyl)-3-methyl-1*H*-imidazol-3-iumiodid,
- 1-Ethyl-4-(3-methoxy-3-oxopropy)-3-propyl-1*H*-imidazol-3-iumiodid,
- 3-Butyl-1-ethyl-4-(3-methoxy-3-oxopropyl)-1*H*-imidazol-3-iumiodid;
- 1-Ethyl-4-(3-methoxy-3-oxopropyl)-3-pentyl-1*H*-imidazol-3-iumiodid,
- 1-Ethyl-3-hexyl-4-(3-methoxy-3-oxopropyl)-1*H*-imidazol-3-iumiodid,
- 4-(3-Methoxy-3-oxopropyl)-3-methyl-1-propyl-1*H*-imidazol-3-iumiodid,
- 3-Ethyl-4-(3-methoxy-3-oxopropyl)-1-propyl-1*H*-imidazol-3-iumiodid,
- 4-(3-Methoxy-3-oxopropyl)-1,3-dipropyl-1*H*-imidazol-3-iumiodid,
- 3-Butyl-4-(3-Methoxy-3-oxopropyl)-1-propyl-1*H*-imidazol-3-iumiodid,
- 4-(3-Methoxy-3-oxopropyl)-3-pentyl-1-propyl-1*H*-imidazol-3-iumiodid,
- 3-Hexyl-4-(3-methoxy-3-oxopropyl)-1-propyl-1*H*-imidazol-3-iumodid,
- 1-Butyl-4-(3-methoxy-3-oxopropyl)-3-methyl-1*H*-imidazol-3-iumiodid,
- 1-Butyl-3-ethyl-4-(3-methoxy-3-oxopropyl)-1*H*-imidazol-3-iumiodid,
- 1-Butyl-4-(3-methoxy-3-oxopropyl)-3-propyl-1*H*-imidazol-3-iumiodid,
- 1,3-Dibutyl-4-(3-methoxy-3-Oxopropyl)-1*H*-imidazol-3-iumiodid,
- 1-Butyl-4-(3-methoxy-3-oxopropyl)-3-pentyl-1*H*-imidazol-3-iumiodid;
- 1-Butyl-3-hexyl-4-(3-methoxy-3-oxopropyl)-1*H*-imidazol-3-iumiodid,
- 4-(3-Methoxy-3-oxopropyl)-3-methyl-1-pentyl-1*H*-imidazol-3-iumiodid,
- 3-Ethyl-4-(3-methoxy-3-oxopropyl)-1-pentyl-1*H*-imidazol-3-iumiodid,
- 4-(3-Methoxy-3-oxopropyl)-1-pentyl-3-propyl-1*H*-imidazol-3-iumiodid,
- 3-Butyl-4-(3-methoxy-3-oxopropyl)-1-pentyl-1*H*-imidazol-3-iumiodid,
- 4-(3-Methoxy-3-oxopropyl)-1,3-dipentyl-1*H*-imidazol-3-iumiodid,
- 3-Hexyl-4-(3-methoxy-3-oxopropyl)-1-pentyl-1*H*-imidazol-3-iumiodid,
- 1-Hexyl-4-(3-methoxy-3-oxopropyl)-3-methyl-1*H*-imidazol-3-iumiodid,
- 3-Ethyl-1-hexyl-4-(3-methoxy-3-oxopropyl)-1*H*-imidazol-3-iumiodid,
- 1-Hexyl-4-(3-methoxy-3-oxopropyl)-3-propyl-1*H*-imidazol-3-iumiodid;
- 3-Butyl-1-hexyl-4-(3-methoxy-3-oxopropyl)-1*H*-imidazol-3-iumiodid,
- 1-Hexyl-4-(3-methoxy-3-oxopropyl)-3-pentyl-1*H*-imidazol-3-iumiodid,
- 1,3-Dihexyl-4-(3-methoxy-3-oxopropyl)-1*H*-imidazol-3-iumiodid,
- 3-Ethyl-4-(3-methoxy-3-oxopropyl)-1-methyl-1*H*-imidazol-3-iumbromid,
- 4-(3-Methoxy-3-oxopropyl)-1-methyl-3-propyl-1*H*-imidazol-3-iumbromid,
- 4-(3-Methoxy-3-oxopropyl)-1-methyl-3-pentyl-1*H*-imidazol-3-iumbromid,
- 3-Hexyl-4-(3-methoxy-3-oxopropyl)-1-methyl-1*H*-imidazol-3-iumbromid;
- 1-Ethyl-4-(3-methoxy-3-oxopropyl)-3-methyl-1*H*-imidazol-3-iumbromid;
- 1,3-Diethyl-4-(3-methoxy-3-oxopropyl)-1*H*-imidazol-3-iumbromid,
- 1-Ethyl-4-(3-methoxy-3-oxopropyl)-3-propyl-1*H*-imidazol-3-iumbromid,
- 1-Ethyl-4-(3-methoxy-3-oxopropyl)-3-pentyl-1*H*-imidazol-3-iumbromid,
- 1-Ethyl-3-hexyl-4-(3-methoxy-3-oxopropyl)-1*H*-imidazol-3-iumbromid,
- 4-(3-Methoxy-3-oxopropyl)-3-methyl-1-propyl-1*H*-imidazol-3-iumbromid,
- 3-Ethyl-4-(3-methoxy-3-oxopropyl)-1-propyl-1*H*-imidazol-3-iumbromid,
- 4-(3-Methoxy-3-oxopropyl)-1,3-dipropyl-1*H*-imidazol-3-iumbromid,
- 3-Butyl-4-(3-methoxy-3-oxopropyl)-1-propyl-1*H*-imidazol-3-iumbromid;
- 4-(3-Methoxy-3-oxopropyl)-3-pentyl-1-propyl-1*H*-imidazol-3-iumbromid;
- 3-Hexyl-4-(3-methoxy-3-oxopropyl)-1-propyl-1*H*-imidazol-3-iumbromid,
- 1-Butyl-4-(3-Methoxy-3-oxopropyl)-3-propyl-1*H*-imidazol-3-iumbromid,
- 1,3-Dibutyl-4-(3-methoxy-3-oxopropyl)-1*H*-imidazol-3-iumbromid,
- 1-Butyl-4-(3-methoxy-3-oxopropyl)-3-pentyl-1*H*-imidazol-3-iumbromid,
- 1-Butyl-3-hexyl-4-(3-methoxy-3-oxopropyl)-1*H*-imidazol-3-iumbromid,
- 4-(3-Methoxy-3-oxopropyl)-3-methyl-1-pentyl-1*H*-imidazol-3-iumbromid;
- 3-Ethyl-4-(3-methoxy-3-oxopropyl)-1-pentyl-1*H*-imidazol-3-iumbromid,
- 4-(3-Methoxy-3-oxopropyl)-1-pentyl-3-propyl-1*H*-imidazol-3-iumbromid,
- 3-Butyl-4-(3-methoxy-3-oxopropyl)-1-pentyl-1*H*-imidazol-3-iumbromid,
- 4-(3-Methoxy-3-oxopropyl)-1,3-dipentyl-1*H*-imidazol-3-iumbromid,
- 3-Hexyl-4-(3-methoxy-3-oxopropyl)-1-pentyl-1*H*-imidazol-3-iumbromid,
- 1-Hexyl-4-(3-Methoxy-3-oxopropyl)-3-methyl-1*H*-imidazol-3-iumbromid,
- 3-ethyl-1-hexyl-4-(3-methoxy-3-oxopropyl)-1*H*-imidazol-3-iumbromid,
- 1-Hexyl-4-(3-methoxy-3-oxopropyl)-3-propyl-1*H*-imidazol-3-iumbromid,
- 3-Butyl-1-hexyl-4-(3-methoxy-3-oxopropyl)-1*H*-imidazol-3-iumbromid,
- 1-Hexyl-4-(3-methoxy-3-oxopropyl)-3-pentyl-1*H*-imidazol-3-iumbromid,
- 1,3-Dihexyl-4-(3-methoxy-3-oxopropyl)-1*H*-imidazol-3-iumbromid,
- 4-(3-Methoxy-3-oxopropyl)-1,2,3-trimethyl-1*H*-imidazol-3-iumiodid,
- 3-Ethyl-4-(3-methoxy-3-oxopropyl)-1,2-dimethyl-1*H*-imidazol-3-iumiodid,
- 4-(3-Methoxy-3-oxopropyl)-1,2-dimethyl-3-propyl-1*H*-imidazol-3-iumiodid;
- 3-Butyl-4-(3-methoxy-3-oxopropyl)-1,2-dimethyl-1*H*-imidazol-3-iumiodid,
- 4-(3-Methoxy-3-oxopropyl)-1,2-dimethyl-3-pentyl-1*H*-imidazol-3-iumiodid,
- 3-Hexyl-4-(3-methoxy-3-oxopropyl)-1,2-dimethyl-1*H*-imidazol-3-iumiodid,
- 1,3-Diethyl-4-(3-Methoxy-3-oxopropyl)-2-methyl-1*H*-imidazol-3-iumiodid,
- 4-(3-Methoxy-3-oxopropyl)-2-methyl-1,3-dipropyl-1*H*-imidazol-3-iumiodid,
- 1,3-Dibutyl-4-(3-methoxy-3-oxopropyl)-2-methyl-1*H*-imidazol-3-iumiodid,
- 4-(3-Methoxy-3-oxopropyl)-2-methyl-1,3-dipentyl-1*H*-imidazol-3-iumiodid,
- 1,3-Dihexyl-4-(3-methoxy-3-oxopropyl)-2-methyl-1*H*-imidazol-3-iumiodid,
- 1-Ethyl-3-hexyl-4-(3-methoxy-3-oxopropyl)-2-methyl-1*H*-imidazol-3-iumiodid,
- 4-(3-Methoxy-3-oxopropyl)-1-methyl-3-propyl-1*H*-imidazol-3-iumbromid,
- 4-(3-Methoxy-3-oxopropyl)-1,2,3-trimethyl-1*H*-imidazol-3-iumbromid,
- 3-Ethyl-4-(3-methoxy-3-oxopropyl)-1,2-dimethyl-1*H*-imidazol-3-iumbromid,
- 4-(3-Methoxy-3-oxopropyl)-1,2-dimethyl-3-propyl-1*H*-imidazol-3-iumbromid,
- 3-Butyl-4-(3-methoxy-3-oxopropyl)-1,2-dimethyl-1*H*-lmidazol-3-iumbromid,
- 4-(3-Methoxy-3-oxopropyl)-1,2-dimethyl-3-pentyl-1*H*-imidazol-3-iumbromid,
- 3-Hexyl-4-(3-methoxy-3-oxopropyl)-1,2-dimethyl-1*H*-imidazol-3-iumbromid;
- 1,3-Diethyl-4-(3-methoxy-3-oxopropyl)-2-methyl-1*H*-imidazol-3-iumbromid,
- 4-(3-Methoxy-3-oxopropyl)-2-methyl-1,3-dipropyl-1*H*-imidazol-3-iumbromid,
- 1,3-Dibutyl-4-(3-methoxy-3-oxopropyl)-2-methyl-1*H*-imidazol-3-iumbromid;
- 4-(3-Methoxy-3-oxopropyl)-2-methyl-1,3-dipentyl-1*H*-imidazol-3-iumbromid, und
- 1-Ethyl-3-hexyl-4-(3-methoxy-3-oxopropyl)-2-methyl-1*H*-imidazol-3-iumiodid.

## Claims

1. Use of at least one halide of the following formula (I): 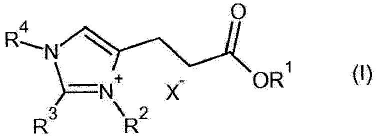 wherein:
- R¹ represents a hydrogen atom or a linear alkyl radical having from 1 to 10 carbon atoms;
- R² and R⁴, which may be identical or different, each represent a linear or branched alkyl radical having from 1 to 15 carbon atoms, said alkyl radical being optionally substituted by one or more C₆-C₂₀-aryl groups which may optionally contain one or more hetero atoms selected from oxygen, sulfur and nitrogen atoms;
- R³ represents a hydrogen atom or a linear or branched alkyl radical having from 1 to 15 carbon atoms;
- X⁻ represents an iodide or bromide anion,
as an electrolyte salt in an electrolyte composition of a photoelectrochemical cell based on the sensitisation to light of photoactive molecules.

2. Use according to claim 1, **characterised in that** the photoelectrochemical cell based on the sensitisation to light of photoactive molecules is a dye-sensitive photovoltaic cell.

3. Use according to claim 1 or 2, **characterised in that** the compounds of formula (I) are selected from:
- 4-(3-methoxy-3-oxopropyl)-1,3-dimethyl-1*H*-imidazol-3-ium iodide,
- 4-(3-ethoxy-3-oxopropyl)-1,3-dimethyl-1*H*-imidazol-3-ium iodide,
- 4-(3-methoxy-3-oxopropyl)-1,3-diethyl-1*H*-imidazol-3-ium iodide,
- 4-(3-methoxy-3-oxopropyl)-1,3-dipropyl-1*H*-imidazol-3-ium iodide,
- 4-(3-methoxy-3-oxopropyl)-1,3-dibutyl-1*H*-imidazol-3-ium iodide,
- 4-(3-methoxy-3-oxopropyl)-1,3-dipentyl-1*H*-imidazol-3-ium iodide,
- 4-(3-methoxy-3-oxopropyl)-1,3-dihexyl-1*H*-imidazol-3-ium iodide,
- 4-(3-methoxy-3-oxopropyl)-1-methyl-3-propyl-1*H*-imidazol-3-ium iodide,
- 3-ethyl-4-(3-methoxy-3-oxopropyl)-1-methyl-1*H*-imidazol-3-ium iodide,
- 4-(3-methoxy-3-oxopropyl)-1-methyl-3-propyl-1*H*-imidazol-3-ium iodide,
- 3-butyl-4-(3-methoxy-3-oxopropyl)-1-methyl-1*H*-imidazol-3-ium iodide,
- 4-(3-methoxy-3-oxopropyl)-1-methyl-3-pentyl-1*H*-imidazol-3-ium iodide,
- 3-hexyl-4-(3-methoxy-3-oxopropyl)-1-methyl-1*H*-imidazol-3-ium iodide,
- 1-ethyl-4-(3-methoxy-3-oxopropyl)-3-methyl-1*H*-imidazol-3-ium iodide,
- 1,3-diethyl-4-(3-methoxy-3-oxopropyl)-1*H*-imidazol-3-ium iodide,
- 1-ethyl-4-(3-methoxy-3-oxopropyl)-3-propyl-1*H*-imidazol-3-ium iodide,
- 3-butyl-1-ethyl-4-(3-methoxy-3-oxopropyl)-1*H*-imidazol-3-ium iodide,
- 1-ethyl-4-(3-methoxy-3-oxopropyl)-3-pentyl-1*H*-imidazol-3-ium iodide,
- 1-ethyl-3-hexyl-4-(3-methoxy-3-oxopropyl)-1*H*-imidazol-3-ium iodide,
- 4-(3-methoxy-3-oxopropyl)-3-methyl-1-propyl-1*H*-imidazol-3-ium iodide,
- 3-ethyl-4-(3-methoxy-3-oxopropyl)-1-propyl-1*H*-imidazol-3-ium iodide,
- 4-(3-methoxy-3-oxopropyl)-1,3-dipropyl-1*H*-imidazol-3-ium iodide,
- 3-butyl-4-(3-methoxy-3-oxopropyl)-1-propyl-1*H*-imidazol-3-ium iodide,
- 4-(3-methoxy-3-oxopropyl)-3-pentyl-1-propyl-1*H*-imidazol-3-ium iodide,
- 3-hexyl-4-(3-methoxy-3-oxopropyl)-1-propyl-1*H*-imidazol-3-ium iodide,
- 1-butyl-4-(3-methoxy-3-oxopropyl)-3-methyl-1*H*-imidazol-3-ium iodide,
- 1-butyl-3-ethyl-4-(3-methoxy-3-oxopropyl)-1*H*-imidazol-3-ium iodide,
- 1-butyl-4-(3-methoxy-3-oxopropyl)-3-propyl-1*H*-imidazol-3-ium iodide,
- 1,3-dibutyl-4-(3-methoxy-3-oxopropyl)-1*H*-imidazol-3-ium iodide,
- 1-butyl-4-(3-methoxy-3-oxopropyl)-3-pentyl-1*H*-imidazol-3-ium iodide,
- 1-butyl-3-hexyl-4-(3-methoxy-3-oxopropyl)-1*H*-imidazol-3-ium iodide,
- 4-(3-methoxy-3-oxopropyl)-3-methyl-1-pentyl-1*H*-imidazol-3-ium iodide,
- 3-ethyl-4-(3-methoxy-3-oxopropyl)-1-pentyl-1*H*-imidazol-3-ium iodide,
- 4-(3-methoxy-3-oxopropyl)-1-pentyl-3-propyl-1*H*-imidazol-3-ium iodide,
- 3-butyl-4-(3-methoxy-3-oxopropyl)-1-pentyl-1*H*-imidazol-3-ium iodide,
- 4-(3-methoxy-3-oxopropyl)-1,3-dipentyl-1*H*-imidazol-3-ium iodide,
- 3-hexyl-4-(3-methoxy-3-oxopropyl)-1-pentyl-1*H*-imidazol-3-ium iodide,
- 1-hexyl-4-(3-methoxy-3-oxopropyl)-3-methyl-1*H*-imidazol-3-ium iodide,
- 3-ethyl-1 -hexyl-4-(3-methoxy-3-oxopropyl)-1*H*-imidazol-3-ium iodide,
- 1-hexyl-4-(3-methoxy-3-oxopropyl)-3-propyl-1*H*-imidazol-3-ium iodide,
- 3-butyl-1-hexyl-4-(3-methoxy-3-oxopropyl)-1*H*-imidazol-3-ium iodide,
- 1-hexyl-4-(3-methoxy-3-oxopropyl)-3-pentyl-1*H*-imidazol-3-ium iodide,
- 1,3-dihexyl-4-(3-methoxy-3-oxopropyl)-1*H*-imidazol-3-ium iodide,
- 3-ethyl-4-(3-methoxy-3-oxopropyl)-1-methyl-1*H*-imidazol-3-ium bromide,
- 4-(3-methoxy-3-oxopropyl)-1-methyl-3-propyl-1*H*-imidazol-3-ium bromide,
- 3-butyl-4-(3-methoxy-3-oxopropyl)-1-methyl-1*H*-imidazol-3-ium bromide,
- 4-(3-methoxy-3-oxopropyl)-1-methyl-3-pentyl-1*H*-imidazol-3-ium bromide,
- 3-hexyl-4-(3-methoxy-3-oxopropyl)-1-methyl-1*H*-imidazol-3-ium bromide,
- 1-ethyl-4-(3-methoxy-3-oxopropyl)-3-methyl-1*H*-imidazol-3-ium bromide,
- 1,3-diethyl-4-(3-methoxy-3-oxopropyl)-1*H*-imidazol-3-ium bromide,
- 1-ethyl-4-(3-methoxy-3-oxopropyl)-3-propyl-1*H*-imidazol-3-ium bromide,
- 3-butyl-1-ethyl-4-(3-methoxy-3-oxopropyl)-1*H*-imidazol-3-ium bromide,
- 1-ethyl-4-(3-methoxy-3-oxopropyl)-3-pentyl-1*H*-imidazol-3-ium bromide,
- 1-ethyl-3-hexyl-4-(3-methoxy-3-oxopropyl)-1*H*-imidazol-3-ium bromide,
- 4-(3-methoxy-3-oxopropyl)-3-methyl-1-propyl-1*H*-imidazol-3-ium bromide,
- 3-ethyl-4-(3-methoxy-3-oxopropyl)-1-propyl-1*H*-imidazol-3-ium bromide,
- 4-(3-methoxy-3-oxopropyl)-1,3-dipropyl-1*H*-imidazol-3-ium bromide,
- 3-butyl-4-(3-methoxy-3-oxopropyl)-1-propyl-1*H*-imidazol-3-ium bromide,
- 4-(3-methoxy-3-oxopropyl)-3-pentyl-1-propyl-1*H*-imidazol-3-ium bromide,
- 3-hexyl-4-(3-methoxy-3-oxopropyl)-1-propyl-1*H*-imidazol-3-ium bromide,
- 1-butyl-4-(3-methoxy-3-oxopropyl)-3-methyl-1*H*-imidazol-3-ium bromide,
- 1-butyl-3-ethyl-4-(3-methoxy-3-oxopropyl)-1*H*-imidazol-3-ium bromide,
- 1-butyl-4-(3-methoxy-3-oxopropyl)-3-propyl-1*H*-imidazol-3-ium bromide,
- 1,3-dibutyl-4-(3-methoxy-3-oxopropyl)-1*H*-imidazol-3-ium bromide,
- 1-butyl-4-(3-methoxy-3-oxopropyl)-3-pentyl-1*H*-imidazol-3-ium bromide,
- 1-butyl-3-hexyl-4-(3-methoxy-3-oxopropyl)-1*H*-imidazol-3-ium bromide,
- 4-(3-methoxy-3-oxopropyl)-3-methyl-1-pentyl-1*H*-imidazol-3-ium bromide,
- 3-ethyl-4-(3-methoxy-3-oxopropyl)-1-pentyl-1*H*-imidazol-3-ium bromide,
- 4-(3-methoxy-3-oxopropyl)-1-pentyl-3-propyl-1*H*-imidazol-3-ium bromide,
- 3-butyl-4-(3-methoxy-3-oxopropyl)-1-pentyl-1*H*-imidazol-3-ium bromide,
- 4-(3-methoxy-3-oxopropyl)-1,3-dipentyl-1*H*-imidazol-3-ium bromide,
- 3-hexyl-4-(3-methoxy-3-oxopropyl)-1-pentyl-1*H*-imidazol-3-ium bromide,
- 1-hexyl-4-(3-methoxy-3-oxopropyl)-3-methyl-1*H*-imidazol-3-ium bromide,
- 3-ethyl-1-hexyl-4-(3-methoxy-3-oxopropyl)-1*H*-imidazol-3-ium bromide,
- 1-hexyl-4-(3-methoxy-3-oxopropyl)-3-propyl-1*H*-imidazol-3-ium bromide,
- 3-butyl-1-hexyl-4-(3-methoxy-3-oxopropyl)-1*H*-imidazol-3-ium bromide,
- 1-hexyl-4-(3-methoxy-3-oxopropyl)-3-pentyl-1*H*-imidazol-3-ium bromide,
- 1,3-dihexyl-4-(3-methoxy-3-oxopropyl)-1*H*-imidazol-3-ium bromide,
- 4-(3-methoxy-3-oxopropyl)-1,2,3-trimethyl-1*H*-imidazol-3-ium iodide,
- 3-ethyl-4-(3-methoxy-3-oxopropyl)-1,2-dimethyl-1*H*-imidazol-3-ium iodide,
- 4-(3-methoxy-3-oxopropyl)-1,2-dimethyl-3-propyl-1*H*-imidazol-3-ium iodide,
- 3-butyl-4-(3-methoxy-3-oxopropyl)-1,2-dimethyl-1*H*-imidazol-3-ium iodide,
- 4-(3-methoxy-3-oxopropyl)-1,2-dimethyl-3-pentyl-1*H*-imidazol-3-ium iodide,
- 3-hexyl-4-(3-methoxy-3-oxopropyl)-1,2-dimethyl-1*H*-imidazol-3-ium iodide,
- 1,3-diethyl-4-(3-methoxy-3-oxopropyl)-2-methyl-1*H*-imidazol-3-ium iodide,
- 4-(3-methoxy-3-oxopropyl)-2-methyl-1,3-dipropyl-1*H*-imidazol-3-ium iodide,
- 1,3-dibutyl-4-(3-methoxy-3-oxopropyl)-2-methyl-1*H*-imidazol-3-ium iodide,
- 4-(3-methoxy-3-oxopropyl)-2-methyl-1,3-dipentyl-1*H*-imidazol-3-ium iodide,
- 1,3-dihexyl-4-(3-methoxy-3-oxopropyl)-2-methyl-1*H*-imidazol-3-ium iodide,
- 1-ethyl-3-hexyl-4-(3-methoxy-3-oxopropyl)-2-methyl-1*H*-imidazol-3-ium iodide,
- 4-(3-methoxy-3-oxopropyl)-1-methyl-3-propyl-1*H*-imidazol-3-ium bromide,
- 4-(3-methoxy-3-oxopropyl)-1,2,3-trimethyl-1*H*-imidazol-3-ium bromide,
- 3-ethyl-4-(3-methoxy-3-oxopropyl)-1,2-dimethyl-1*H*-imidazol-3-ium bromide,
- 4-(3-methoxy-3-oxopropyl)-1,2-dimethyl-3-propyl-1*H*-imidazol-3-ium bromide,
- 3-butyl-4-(3-methoxy-3-oxopropyl)-1,2-dimethyl-1*H*-imidazol-3-ium bromide,
- 4-(3-methoxy-3-oxopropyl)-1,2-dimethyl-3-pentyl-1*H*-imidazol-3-ium bromide,
- 3-hexyl-4-(3-methoxy-3-oxopropyl)-1,2-dimethyl-1*H*-imidazol-3-ium bromide,
- 1,3-diethyl-4-(3-methoxy-3-oxopropyl)-2-methyl-1*H*-imidazol-3-ium bromide,
- 4-(3-methoxy-3-oxopropyl)-2-methyl-1,3-dipropyl-1*H*-imidazol-3-ium bromide,
- 1,3-dibutyl-4-(3-methoxy-3-oxopropyl)-2-methyl-1*H*-imidazol-3-ium bromide,
- 4-(3-methoxy-3-oxopropyl)-2-methyl-1,3-dipentyl-1*H*-imidazol-3-ium bromide, and
- 1-ethyl-3-hexyl-4-(3-methoxy-3-oxopropyl)-2-methyl-1*H*-imidazol-3-ium iodide.

4. Photoelectrochemical cell based on the sensitisation to light of photoactive molecules, comprising:
- an assembly forming a photoelectrode, comprising:
i) a transparent substrate coated with a layer of a transparent and conductive electrode material,
ii) a photoactive layer formed on said electrode material, said photoactive layer comprising a transparent carrier material and at least one photoactive molecule;
- an assembly forming a counter-electrode; and
- an electrolyte composition interposed between said photoactive layer and said counter-electrode, said cell being **characterised in that** said electrolyte composition comprises, in an electrolyte solvent, a reversible redox pair (I₃/I⁻) based on diiodine and at least one compound of formula (I) as defined in either claim 1 or claim 3.

5. Cell according to claim 4, **characterised in that** it is a dye-sensitive photovoltaic cell.

6. Cell according to claim 4 or 5, **characterised in that** the concentration of the compound or compounds of formula (I) in the electrolyte composition varies from 0.1 to 5 mol/L.

7. Cell according to any one of claims 4 to 6, **characterised in that** the concentration of diiodine in the electrolyte composition varies preferably from 0.001 to 0.5 mol/L.

8. Cell according to any one of claims 4 to 7, **characterised in that** the electrolyte solvent or solvents can be selected from nitriles; sulfolane; dimethyl sulfoxide; dioxane; tetrahydrofuran; nitromethane; amides; N-methyl-2-pyrrolidinone; carbonates; ethylene glycols; alcohols; ketones; anhydrides; ethers; water; phthalates; adipates; citrates; sebacates; maleates; benzoates; and succinates.

9. Cell according to any one of claims 4 to 7, **characterised in that** the solvent or solvents is/are an ionic liquid selected from 1-ethyl-3-methylimidazolium bis(trifluoromethanesulfonyl)imide, 1-butyl-3-methylimidazolium bis(trifluoromethanesulfonyl)imide, 1-butyl-3-methylimidazolium hexafluorophosphate, 1-butyl-1-methyl-pyrrolidinium bis(trifluoromethylsulfonyl)imide and mixtures thereof.

10. Cell according to any one of claims 4 to 9, **characterised in that** the electrolyte composition further contains one or more additives selected from benzimidazole derivatives; pyridine derivatives; triazole derivatives; pyrazole derivatives; imidazole derivatives; ethylene carbonate; guanidine thiocyanate and chenodeoxycholic acid; magnesium iodide; and the alkali salts of the formula LiY or NaY wherein Y is an anion I⁻, Br⁻, trifluorosulfonate, ClO₄⁻, NO₃⁻ or TFSI⁻.

11. Cell according to any one of claims 4 to 10, **characterised in that** the transparent substrate of the photoelectrode is made of a flexible or rigid material selected from glass or a sheet of a plastics material.

12. Cell according to any one of claims 4 to 11, **characterised in that** the electrode material of the photoelectrode is applied to the transparent substrate and is in the form of a layer of a transparent conductive oxide selected from fluorine-doped tin oxide, indium tin oxide, antimony-doped indium oxide, and aluminium-, gallium- or indium-doped zinc oxide.

13. Cell according to any one of claims 4 to 12, **characterised in that** the photoactive molecule of the photoactive layer is a ruthenium or osmium complex.

14. Cell according to any one of claims 4 to 13, **characterised in that** the counter-electrode is composed of a transparent counter-substrate coated with a layer of SnO₂:F or doped ZnO or of a stack of transparent conductive layers, and **in that** a catalyst layer is formed on the material forming the counter-electrode.

15. Cell according to claim 14, **characterised in that** the catalyst layer is a layer of platinum or of carbon or of inorganic chalcogenide.

16. Compounds of the following formula (I'): 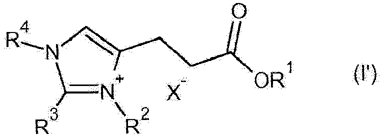 wherein:
- R¹ represents a hydrogen atom or a linear alkyl radical having from 1 to 10 carbon atoms;
- R² and R⁴, which may be identical or different, each represent a linear or branched alkyl radical having from 1 to 15 carbon atoms, said alkyl radical being optionally substituted by one or more C₆-C₂₀-aryl groups which may optionally contain one or more hetero atoms selected from oxygen, sulfur and nitrogen atoms;
- R³ represents a hydrogen atom or a linear or branched alkyl radical having from 1 to 15 carbon atoms;
- X⁻ represents an iodide or bromide anion,
it being understood that:
- when X⁻ represents an iodide anion, R¹ represents a methyl radical and R³ is a hydrogen atom, then the radicals R² and R⁴ may not simultaneously represent a methyl radical or an ethyl radical,
- when X⁻ represents a bromide anion, R¹ represents a methyl radical and R³ is a hydrogen atom, then the radicals R² and R⁴ may not simultaneously represent a benzyl radical,
- when X⁻ represents a bromide anion, R¹ represents a methyl radical, R² is a butyl radical and R³ is a hydrogen atom, then the radical R⁴ is other than a methyl, ethyl or benzyl radical,
- when X⁻ represents an iodide anion, R¹ and R² represent a methyl radical and R³ is a hydrogen atom, then the radical R⁴ is other than a trityl radical, and
- when X⁻ represents a bromide anion, R¹ represents a methyl radical, R² is a butyl radical and R³ is an isopropyl radical, then the radical R⁴ is other than a methyl radical.

17. Compound of formula (I') according to claim 16, **characterised in that** it is selected from:
- 4-(3-ethoxy-3-oxopropyl)-1,3-dimethyl-1*H*-imidazol-3-ium iodide,
- 4-(3-methoxy-3-oxopropyl)-1,3-dipropyl-1*H*-imidazol-3-ium iodide,
- 4-(3-methoxy-3-oxopropyl)-1,3-dibutyl-1*H*-imidazol-3-ium iodide,
- 4-(3-methoxy-3-oxopropyl)-1,3-dipentyl-1*H*-imidazol-3-ium iodide,
- 4-(3-methoxy-3-oxopropyl)-1,3-dihexyl-1*H*-imidazol-3-ium iodide,
- 3-ethyl-4-(3-methoxy-3-oxopropyl)-1-methyl-1*H*-imidazol-3-ium iodide,
- 4-(3-methoxy-3-oxopropyl)-1-methyl-3-propyl-1*H*-imidazol-3-ium iodide,
- 3-butyl-4-(3-methoxy-3-oxopropyl)-1-methyl-1*H*-imidazol-3-ium iodide,
- 4-(3-methoxy-3-oxopropyl)-1-methyl-3-pentyl-1*H*-imidazol-3-ium iodide,
- 3-hexyl-4-(3-methoxy-3-oxopropyl)-1-methyl-1*H*-imidazol-3-ium iodide,
- 1-ethyl-4-(3-methoxy-3-oxopropyl)-3-methyl-1*H*-imidazol-3-ium iodide,
- 1-ethyl-4-(3-methoxy-3-oxopropyl)-3-propyl-1*H*-imidazol-3-ium iodide,
- 3-butyl-1-ethyl-4-(3-methoxy-3-oxopropyl)-1*H*-imidazol-3-ium iodide,
- 1-ethyl-4-(3-methoxy-3-oxopropyl)-3-pentyl-1*H*-imidazol-3-ium iodide,
- 1-ethyl-3-hexyl-4-(3-methoxy-3-oxopropyl)-1*H*-imidazol-3-ium iodide,
- 4-(3-methoxy-3-oxopropyl)-3-methyl-1-propyl-1*H*-imidazol-3-ium iodide,
- 3-ethyl-4-(3-methoxy-3-oxopropyl)-1-propyl-1*H*-imidazol-3-ium iodide,
- 4-(3-methoxy-3-oxopropyl)-1,3-dipropyl-1*H*-imidazol-3-ium iodide,
- 3-butyl-4-(3-methoxy-3-oxopropyl)-1-propyl-1*H*-imidazol-3-ium iodide,
- 4-(3-methoxy-3-oxopropyl)-3-pentyl-1-propyl-1*H*-imidazol-3-ium iodide,
- 3-hexyl-4-(3-methoxy-3-oxopropyl)-1-propyl-1*H*-imidazol-3-ium iodide,
- 1-butyl-4-(3-methoxy-3-oxopropyl)-3-methyl-1*H*-imidazol-3-ium iodide,
- 1-butyl-3-ethyl-4-(3-methoxy-3-oxopropyl)-1*H*-imidazol-3-ium iodide,
- 1-butyl-4-(3-methoxy-3-oxopropyl)-3-propyl-1*H*-imidazol-3-ium iodide,
- 1,3-dibutyl-4-(3-methoxy-3-oxopropyl)-1*H*-imidazol-3-ium iodide,
- 1-butyl-4-(3-methoxy-3-oxopropyl)-3-pentyl-1*H*-imidazol-3-ium iodide,
- 1-butyl-3-hexyl-4-(3-methoxy-3-oxopropyl)-1*H*-imidazol-3-ium iodide,
- 4-(3-methoxy-3-oxopropyl)-3-methyl-1-pentyl-1*H*-imidazol-3-ium iodide,
- 3-ethyl-4-(3-methoxy-3-oxopropyl)-1-pentyl-1*H*-imidazol-3-ium iodide,
- 4-(3-methoxy-3-oxopropyl)-1-pentyl-3-propyl-1*H*-imidazol-3-ium iodide,
- 3-butyl-4-(3-methoxy-3-oxopropyl)-1-pentyl-1*H*-imidazol-3-ium iodide,
- 4-(3-methoxy-3-oxopropyl)-1,3-dipentyl-1*H*-imidazol-3-ium iodide,
- 3-hexyl-4-(3-methoxy-3-oxopropyl)-1-pentyl-1*H*-imidazol-3-ium iodide,
- 1-hexyl-4-(3-methoxy-3-oxopropyl)-3-methyl-1*H*-imidazol-3-ium iodide,
- 3-ethyl-1 -hexyl-4-(3-methoxy-3-oxopropyl)-1*H*-imidazol-3-ium iodide,
- 1-hexyl-4-(3-methoxy-3-oxopropyl)-3-propyl-1*H*-imidazol-3-ium iodide,
- 3-butyl-1-hexyl-4-(3-methoxy-3-oxopropyl)-1*H*-imidazol-3-ium iodide,
- 1-hexyl-4-(3-methoxy-3-oxopropyl)-3-pentyl-1*H*-imidazol-3-ium iodide,
- 1,3-dihexyl-4-(3-methoxy-3-oxopropyl)-1*H*-imidazol-3-ium iodide,
- 3-ethyl-4-(3-methoxy-3-oxopropyl)-1-methyl-1*H*-imidazol-3-ium bromide,
- 4-(3-methoxy-3-oxopropyl)-1-methyl-3-propyl-1*H*-imidazol-3-ium bromide,
- 4-(3-methoxy-3-oxopropyl)-1-methyl-3-pentyl-1*H*-imidazol-3-ium bromide,
- 3-hexyl-4-(3-methoxy-3-oxopropyl)-1-methyl-1*H*-imidazol-3-ium bromide,
- 1-ethyl-4-(3-methoxy-3-oxopropyl)-3-methyl-1*H*-imidazol-3-ium bromide,
- 1,3-diethyl-4-(3-methoxy-3-oxopropyl)-1*H*-imidazol-3-ium bromide,
- 1-ethyl-4-(3-methoxy-3-oxopropyl)-3-propyl-1*H*-imidazol-3-ium bromide,
- 1-ethyl-4-(3-methoxy-3-oxopropyl)-3-pentyl-1*H*-imidazol-3-ium bromide,
- 1-ethyl-3-hexyl-4-(3-methoxy-3-oxopropyl)-1*H*-imidazol-3-ium bromide,
- 4-(3-methoxy-3-oxopropyl)-3-methyl-1-propyl-1*H*-imidazol-3-ium bromide,
- 3-ethyl-4-(3-methoxy-3-oxopropyl)-1-propyl-1*H*-imidazol-3-ium bromide,
- 4-(3-methoxy-3-oxopropyl)-1,3-dipropyl-1*H*-imidazol-3-ium bromide,
- 3-butyl-4-(3-methoxy-3-oxopropyl)-1-propyl-1*H*-imidazol-3-ium bromide,
- 4-(3-methoxy-3-oxopropyl)-3-pentyl-1-propyl-1*H*-imidazol-3-ium bromide,
- 3-hexyl-4-(3-methoxy-3-oxopropyl)-1-propyl-1*H*-imidazol-3-ium bromide,
- 1-butyl-4-(3-methoxy-3-oxopropyl)-3-propyl-1*H*-imidazol-3-ium bromide,
- 1,3-dibutyl-4-(3-methoxy-3-oxopropyl)-1*H*-imidazol-3-ium bromide,
- 1-butyl-4-(3-methoxy-3-oxopropyl)-3-pentyl-1*H*-imidazol-3-ium bromide,
- 1-butyl-3-hexyl-4-(3-methoxy-3-oxopropyl)-1*H*-imidazol-3-ium bromide,
- 4-(3-methoxy-3-oxopropyl)-3-methyl-1-pentyl-1*H*-imidazol-3-ium bromide,
- 3-ethyl-4-(3-methoxy-3-oxopropyl)-1-pentyl-1*H*-imidazol-3-ium bromide,
- 4-(3-methoxy-3-oxopropyl)-1-pentyl-3-propyl-1*H*-imidazol-3-ium bromide,
- 3-butyl-4-(3-methoxy-3-oxopropyl)-1-pentyl-1*H*-imidazol-3-ium bromide,
- 4-(3-methoxy-3-oxopropyl)-1,3-dipentyl-1*H*-imidazol-3-ium bromide,
- 3-hexyl-4-(3-methoxy-3-oxopropyl)-1-pentyl-1*H*-imidazol-3-ium bromide,
- 1-hexyl-4-(3-methoxy-3-oxopropyl)-3-methyl-1*H*-imidazol-3-ium bromide,
- 3-ethyl-1-hexyl-4-(3-methoxy-3-oxopropyl)-1*H*-imidazol-3-ium bromide,
- 1-hexyl-4-(3-methoxy-3-oxopropyl)-3-propyl-1*H*-imidazol-3-ium bromide,
- 3-butyl-1-hexyl-4-(3-methoxy-3-oxopropyl)-1*H*-imidazol-3-ium bromide,
- 1-hexyl-4-(3-methoxy-3-oxopropyl)-3-pentyl-1*H*-imidazol-3-ium bromide,
- 1,3-dihexyl-4-(3-methoxy-3-oxopropyl)-1*H*-imidazol-3-ium bromide,
- 4-(3-methoxy-3-oxopropyl)-1,2,3-trimethyl-1*H*-imidazol-3-ium iodide,
- 3-ethyl-4-(3-methoxy-3-oxopropyl)-1,2-dimethyl-1*H*-imidazol-3-ium iodide,
- 4-(3-methoxy-3-oxopropyl)-1,2-dimethyl-3-propyl-1*H*-imidazol-3-ium iodide,
- 3-butyl-4-(3-methoxy-3-oxopropyl)-1,2-dimethyl-1*H*-imidazol-3-ium iodide,
- 4-(3-methoxy-3-oxopropyl)-1,2-dimethyl-3-pentyl-1*H*-imidazol-3-ium iodide,
- 3-hexyl-4-(3-methoxy-3-oxopropyl)-1,2-dimethyl-1*H*-imidazol-3-ium iodide,
- 1,3-diethyl-4-(3-methoxy-3-oxopropyl)-2-methyl-1*H*-imidazol-3-ium iodide,
- 4-(3-methoxy-3-oxopropyl)-2-methyl-1,3-dipropyl-1*H*-imidazol-3-ium iodide,
- 1,3-dibutyl-4-(3-methoxy-3-oxopropyl)-2-methyl-1*H*-imidazol-3-ium iodide,
- 4-(3-methoxy-3-oxopropyl)-2-methyl-1,3-dipentyl-1*H*-imidazol-3-ium iodide,
- 1,3-dihexyl-4-(3-methoxy-3-oxopropyl)-2-methyl-1*H*-imidazol-3-ium iodide,
- 1-ethyl-3-hexyl-4-(3-methoxy-3-oxopropyl)-2-methyl-1*H*-imidazol-3-ium iodide,
- 4-(3-methoxy-3-oxopropyl)-1-methyl-3-propyl-1*H*-imidazol-3-ium bromide,
- 4-(3-methoxy-3-oxopropyl)-1,2,3-trimethyl-1*H*-imidazol-3-ium bromide,
- 3-ethyl-4-(3-methoxy-3-oxopropyl)-1,2-dimethyl-1*H*-imidazol-3-ium bromide,
- 4-(3-methoxy-3-oxopropyl)-1,2-dimethyl-3-propyl-1*H*-imidazol-3-ium bromide,
- 3-butyl-4-(3-methoxy-3-oxopropyl)-1,2-dimethyl-1*H*-imidazol-3-ium bromide,
- 4-(3-methoxy-3-oxopropyl)-1,2-dimethyl-3-pentyl-1*H*-imidazol-3-ium bromide,
- 3-hexyl-4-(3-methoxy-3-oxopropyl)-1,2-dimethyl-1*H*-imidazol-3-ium bromide,
- 1,3-diethyl-4-(3-methoxy-3-oxopropyl)-2-methyl-1*H*-imidazol-3-ium bromide,
- 4-(3-methoxy-3-oxopropyl)-2-methyl-1,3-dipropyl-1*H*-imidazol-3-ium bromide,
- 1,3-dibutyl-4-(3-methoxy-3-oxopropyl)-2-methyl-1*H*-imidazol-3-ium bromide,
- 4-(3-methoxy-3-oxopropyl)-2-methyl-1,3-dipentyl-1*H*-imidazol-3-ium bromide, and
- 1-ethyl-3-hexyl-4-(3-methoxy-3-oxopropyl)-2-methyl-1*H*-imidazol-3-ium iodide.
